# FASCICULE DE BREVET EUROPEEN

(11) **EP 3 512 864 B1**
(45) Date de publication et mention de la délivrance du brevet: **20.01.2021**
(21) Numéro de dépôt: 17764847.4
(22) Date de dépôt: 15.09.2017
(51) Int. Cl.: C07J 41/00, C07J 9/00, A61K 31/575, A61P 31/04, A61P 31/10, A61P 31/12, A61P 33/00

(54) **DÉRIVÉS AMIDES DE SQUALAMINE POUR LE TRAITEMENT DES INFECTIONS**
AMIDDERIVATE VON SQUALAMIN ZUR BEHANDLUNG VON INFEKTIONEN
AMIDE DERIVATIVES OF SQUALAMINE FOR THE TREATMENT OF INFECTIONS

(30) Priorité: 15.09.2016 FR 1658650
(43) Date de publication de la demande: 24.07.2019
(73) Titulaire: Virbac, 06511 Carros Cedex (FR)
(72) Inventeur: BRUNEL, Jean-Michel, 13012 Marseille (FR); BLANCHET, Marine, 33820 Etauliers (FR); MARC, Jean-Pascal, 06570 Saint Paul de Vence (FR)
(74) Mandataire: Nony
(86) Numéro de dépôt international: PCT/EP2017/073267
(87) Numéro de publication internationale: WO 2018/050815

(56) Documents cités:
- US-A- 5 856 535
- WEN-HUA CHEN ET AL: "A Bioconjugate Approach toward Squalamine Mimics: Insight into the Mechanism of Biological Action", BIOCONJUGATE CHEMISTRY, vol. 17, no. 6, 1 novembre 2006 (2006-11-01), pages 1582-1591, XP055353519, ISSN: 1043-1802, DOI: 10.1021/bc060220n
- A. M. BELLINI ET AL: "Antimicrobial Activity of Basic Cholane Derivatives Part IX", ARCHIV DER PHARMAZIE, vol. 323, no. 4, 1 janvier 1990 (1990-01-01), pages 201-205, XP055024553, ISSN: 0365-6233, DOI: 10.1002/ardp.19903230404

## Description

La présente invention concerne des analogues de la squalamine pour leur utilisation dans le traitement des infections bactériennes, fongiques, virales ou parasitaires chez l'homme ou l'animal, ainsi que les compositions pharmaceutiques ou vétérinaires les comprenant.

En 1993, la squalamine, un stéroïde naturel, isolée majoritairement des tissus d'un petit requin *Squalus acanthias,* s'est révélée être une substance très active présentant essentiellement une activité antiangiogénique contre les cellules et une activité anti virale et antibactérienne.

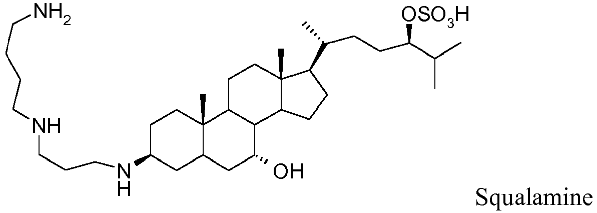

Chimiquement, la squalamine est une molécule originale présentant un caractère amphiphile. Elle comporte ainsi, une partie centrale apolaire (un squelette de type cholestane) et deux extrémités polaires (une chaîne polyamine et un groupement sulfate).

Initialement, ce polyaminostérol hydrosoluble avait suscité l'intérêt pour ses propriétés antiangiogéniques et antimicrobiennes sur une variété de bactéries à Gram positif (*Staphylococcus aureus, Enterococcus faecalis*) et à Gram négatif (*Escherichia coli, Pseudomonas aeruginosa*), des champignons *(Candia albicans, Candida tropicalis*) et des protozoaires.

La source naturelle de la squalamine étant limitée, on a recherché des dérivés synthétiques analogues aminostéroïdiens de la squalamine. On a décrit notamment des dérivés ou analogues comportant une chaîne polyaminée en position 3 ou 7 de cycles 10, 13 diméthyl, 17 octane cholestane ou cholestène, éventuellement hydroxylés en position 7 ou respectivement 3. En particulier, des dérivés de formule IIa - IIb - IIc - IId et II-1 ci-après ont été décrits comme présentant une activité antibactérienne similaire à la squalamine vis-à-vis de diverses bactéries Gram positif et Gram négatif multirésistantes.

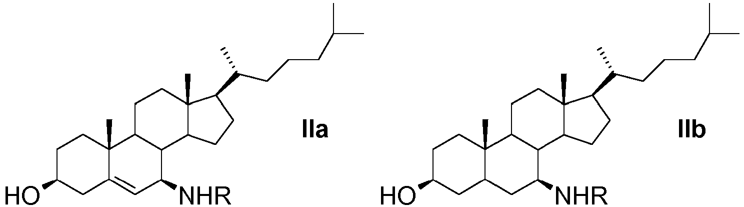

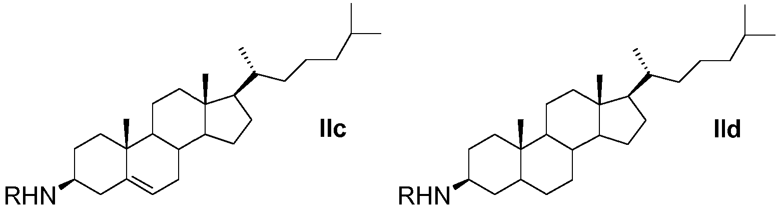

On a plus particulièrement suggéré une application de ces dérivés pour un traitement curatif des infections pulmonaires par voie aérosol. Cependant, la Demanderesse a observé que ces composés présentaient une cytotoxicité importante et que les composés de formule IIc et IId présentaient une faible activité contre certaines bactéries gram-négatives telles que *E. coli.*

On connaît en particulier de WO 2011/067501 des dérivés aminostéroïdiens antibactériens de type polyamino cholestane ou cholestène pour une application par voie topique locale, pour la décolonisation cutanéo-muqueuse rapide de *Staphylococcus aureus,* notamment sous la forme de pommade ou de crème.

Le brevet US 5,856,535 décrit par ailleurs des esters d'aminostérols, dont certains présentent entre autres des activités d'inhibition de l'angiogénèse, des activités antiprolifératives ou encore des activités antibactériennes. Toutefois aucun des composés décrits dans ce document ne sont en particulier des amides.

On connait également les documents Wen-Hua Chen et al « A bioconjugate approach toward squalamine mimics: insight into the mechanism of the biological action », Bioconjugate Chemistry, vol. 17, no. 6, 1582-1591 et AM Bellini et al « Antimicrobial activity of basic cholane derivatives part IX », Archiv des Pharmazie, vol. 323, no. 4, 201-205. Toutefois, ces documents décrivent non seulement des composés structurellement éloignés des composés de la présente invention mais encore ne présentant pas d'activités antimicrobiennes intéressantes.

Il a maintenant été découvert des composés analogues de la squalamine, présentant une bonne activité antibactérienne contre des bactéries gram-positives et gram-négatives, tout en étant avantageusement moins cytotoxiques que la squalamine. Cette nouvelle famille de molécules, grâce à sa structure chimique, présente une meilleure stabilité chimique que les composés décrits dans US 5,856,535.

Ces composés ont une activité intéressante pour prévenir et/ou inhiber et/ou traiter les infections bactériennes, fongiques, virales ou parasitaires chez l'homme ou l'animal. Ces composés sont également des composés de choix en tant que substituts aux antibiotiques. Selon un mode de réalisation particulier de l'invention, elle est destinée aux mammifères domestiques tels que ruminants, chevaux, porcins, chiens et chats et aux animaux sauvages. Selon un mode de réalisation encore plus particulier elle est destinée aux animaux de compagnie, encore plus précisément aux chiens et aux chats, ou encore aux rongeurs, et s'adresse plus particulièrement aux chiens et aux chats.

Les composés selon l'invention permettent une excellente activité contre les bactéries, tout en évitant l'apparition de résistances, qui est un atout majeur tant le problème de l'apparition de résistances aux antibiotiques classiques est devenu un problème de santé publique. De par leur mécanisme d'action, différent de celui des antibiotiques, les composés de l'invention se présentent donc comme d'excellents substituts aux antibiotiques.

Ainsi, selon un premier aspect, la présente invention est relative à un composé de formule (I) 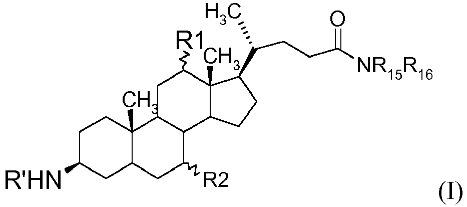 dans laquelle
R1 et R2 représentent indépendamment un atome d'hydrogène, un groupe SO₃H ou un groupe hydroxy,
R' représente un groupe -(CRₐR_{b})ₙ-X-(CR_{c}R_{d})ₘ-[Y-(CRₑR_{f})ₒ]ₜ-NR₉R₁₀,
Rₐ, R_{b}, R_{c}, R_{d}, Rₑ et R_{f} représentent indépendamment un atome d'hydrogène, un groupe (C₁-C₈)alkyle ou un groupe (C₆-C₁₀)aryle,
X et Y représentent indépendamment un groupe -NR11-, un groupe -O- ou un groupe hétérocyclique divalent comprenant au moins un atome d'azote, à 5 ou 6 chaînons,
R9 et R10 représentent indépendamment un atome d'hydrogène, un groupe (C₁-C₈)alkyle ou forment ensemble, avec l'atome d'azote qui les porte, un groupe hétérocyclique à 5 ou 6 chaînons, éventuellement substitué par un ou deux groupe(s) =O ou =S,
R11 représente un atome d'hydrogène, un groupe (C₁-C₈)alkyle ou un groupe -(CH₂)ₛ-NH₂,
R15 et R16 représentent indépendamment un atome d'hydrogène, un groupe (C₁-C₈)alkyle ou un groupe (C₆-C₁₀)aryle,
n, m, o et s représentent indépendamment un nombre entier compris entre 1 et 5,
t est égal à 0, 1, 2 ou 3,
ainsi que les stéréoisomères, mélanges de stéréoisomères, et/ou sels pharmaceutiquement acceptables de celui-ci.

Dans le contexte de la présente invention :
- Les radicaux « alkyles » représentent des radicaux hydrocarbonés saturés, en chaîne droite ou ramifiée, de 1 à 8 atomes de carbone, notamment de 1 à 6 atomes de carbone, de préférence de 1 à 4 atomes de carbone. On peut notamment citer, lorsqu'ils sont linéaires, les radicaux méthyle, éthyle, propyle, butyle, pentyle, hexyle. On peut notamment citer, lorsqu'ils sont ramifiés, les radicaux isopropyle, tert-butyl, 2-méthylbutyle, 2-méthylpentyle et 1-méthylpentyle.
- Par groupe « aryle », on entend, au sens de la présente demande, un système aromatique hydrocarboné, mono ou bicyclique de 6 à 10 atomes de carbone. Parmi les radicaux aryles, on peut notamment citer le radical phényle ou naphtyle, et encore plus particulièrement le radical phényle.
- Par groupe « hétérocyclyclique », on entend, au sens de la présente demande, un système hydrocarboné mono-, ou bicyclique, saturé, insaturé ou aromatique comprenant un ou plusieurs hétéroatomes tels que O, N ou S. Les groupes hétérocycliques incluent notamment les groupes hétéroaryle ou hétérocycloalkyle.

- Les groupes « hétéroaryles » désignent les systèmes aromatiques mono ou bicyclique, et ayant de 5 à 7 chaînons (atomes de cycle), notamment de 5 à 6 chaînons, comprenant un ou plusieurs hétéroatomes choisis parmi l'azote, l'oxygène ou le soufre. Parmi les radicaux hétéroaryles, on peut citer l'imidazolyle, le pyrazinyle, le thiényle, l'oxazolyle, le furazanyle et le pyrrolyle.
- Les radicaux « hétérocycloalkyles » désignent les systèmes mono ou bicycliques, saturés de 5 à 7 chaînons (atomes de cycle), notamment de 5 à 6 chaînons, comprenant un ou plusieurs hétéroatomes choisis parmi N, O ou S. Parmi les hétérocycloalkyles, on peut notamment citer la pyrazolidine, la pipéridine, la morpholine et la pipérazine.
- Tel qu'il est utilisé ici, le terme «pharmaceutiquement acceptable» se réfère à des composés, compositions et/ou formes de dosage qui sont, dans la portée d'un jugement médical valable, adapté pour une utilisation en contact avec les cellules des humains et des animaux inférieurs sans toxicité, irritation, réponse allergique indue et similaires, et sont proportionnés à un rapport avantage/risque raisonnable.
- L'expression « sels pharmaceutiquement acceptables » fait référence aux sels d'addition d'acides inorganiques et organiques, pharmaceutiquement acceptables, et les sels d'addition de bases pharmaceutiquement acceptables, des composés de la présente invention. Ces sels incluent des sels d'addition acides, c'est-à-dire des sels d'acide organique ou minéral d'un composé comportant une fonction basique telle qu'une amine, ou des sels d'addition basiques, c'est-à-dire des sels alcalins ou organiques d'un composé comportant une fonction acide telle qu'un acide carboxylique. Ces sels peuvent être préparés *in situ* pendant l'isolement final et/ou la purification des composés. En particulier, les sels d'addition acide peuvent être préparés en faisant réagir séparément le composé purifié avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, methanesulfonates, éthanesulfonates, benzenesulfonates, p-toluenesulfonates, cyclohexyl sulfamates et quinateslaurylsulfonate, et analogues. (Voir par exemple S.M. Berge et al. « Pharmaceutical Salts » J. Pharm. Sci, 66 :p.1-19 (1977)).
- Les sels d'addition basiques peuvent également être préparés en faisant réagir séparément le composé purifié sous sa forme acide avec une base organique ou inorganique et en isolant le sel ainsi formé. Des exemples de sels d'addition basiques comprennent les sels de sodium, potassium, calcium, baryum, zinc, magnésium et aluminium. Les sels de sodium et de potassium sont préférés. Les sels d'addition basiques peuvent notamment être préparés à partir d'hydrures ou d'hydroxydes de métal alcalin ou alcalino-terreux qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc.

Parmi les composés de formule générale (I), un premier sous-groupe de composés est constitué des composés pour lesquels R1 et R2 représentent indépendamment un atome d'hydrogène ou un groupe hydroxy,

Parmi les composés de formule générale (I), un deuxième sous-groupe de composés est constitué des composés pour lesquels R15 et R16 représentent indépendamment un atome d'hydrogène ou un groupe (C₁-C₄)alkyle,

Parmi les composés de formule générale (I), un troisième sous-groupe de composés est constitué des composés pour lesquels X est un groupe -NH- ou un groupe 1,4-piperydinyle,

Parmi les composés de formule générale (I), un quatrième sous-groupe de composés est constitué des composés pour lesquels R9 et R10 représentent un atome d'hydrogène.

Parmi les composés de formule générale (I), un cinquième sous-groupe de composés est constitué des composés pour lesquels Rₐ, R_{b}, R_{c}, R_{d}, Rₑ et R_{f} représente un atome d'hydrogène.

Parmi les composés de formule générale (I), un sixième sous-groupe de composés est constitué des composés pour lesquels Y est un groupe -NR11-, avec R11 représentant un atome d'hydrogène, un groupe (C₁-C₄)alkyle ou un groupe -(CH₂)ₛ-NH₂ où s est égal à 1, 2 ou 3.

Parmi les composés de formule générale (I), un septième sous-groupe de composés est constitué des composés pour lesquels m est égal à 2, 3, 4, ou 5, plus préférentiellement à 2 ou 3.

Parmi les composés de formule générale (I), un huitième sous-groupe de composés est constitué des composés pour lesquels n est égal à 2, 3, 4 ou 5, plus préférentiellement à 2, 3 ou 4, et encore plus préférentiellement à 2 ou 3.

Parmi les composés de formule générale (I), un neuvième sous-groupe de composés est constitué des composés pour lesquels m est différent de 4.

Parmi les composés de formule générale (I), un dixième sous-groupe de composés est constitué des composés pour lesquels o est égal à 2 ou 3.

Parmi les composés de formule générale (I), un onzième sous-groupe de composés est constitué des composés pour lesquels le groupe -NHR' est choisi parmi :

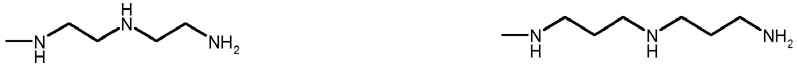

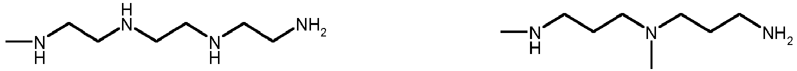

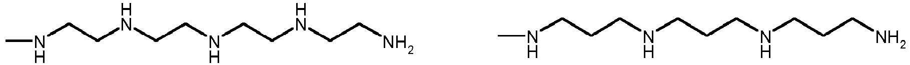

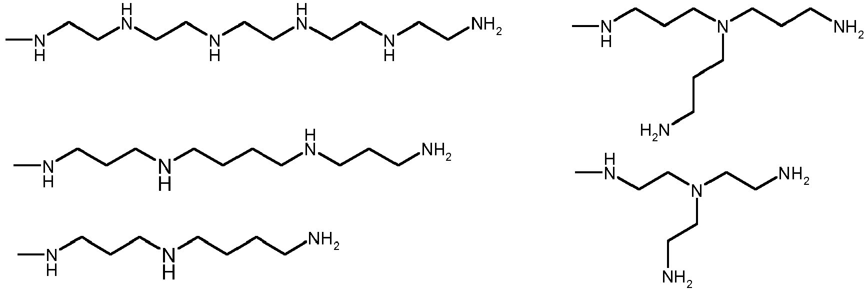

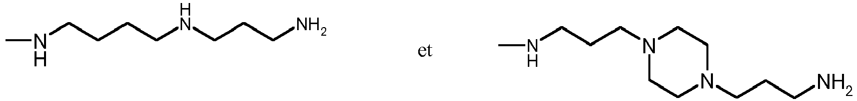

Selon une variante préférée, les composés de formule (I) sont synthétisés à partir des acides biliaires suivants :

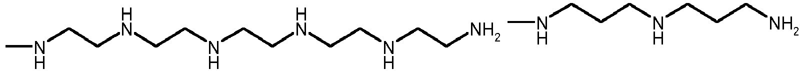

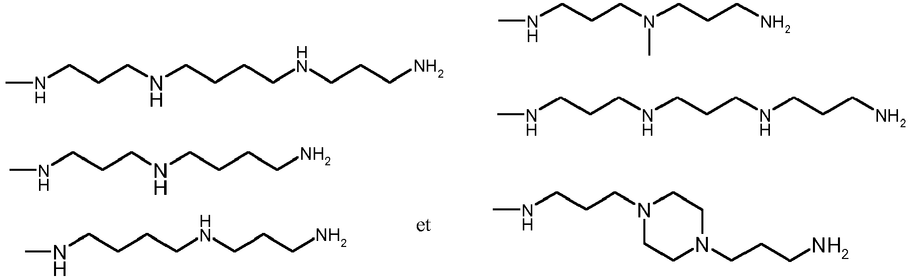

Les sous-groupes définis ci-dessus, pris isolément ou en combinaison, font également partie de l'invention.

Dès lors, la présente invention concerne un composé tel que défini précédemment, caractérisé en ce qu'il est défini par au moins l'un des sous-groupes suivants :
- premier sous-groupe de composés de formule (I) lesquels R1 et R2 représentent indépendamment un atome d'hydrogène ou un groupe hydroxy,
- deuxième sous-groupe de composés de formule (I) pour lesquels R15 et R16 représentent indépendamment un atome d'hydrogène ou un groupe (C₁-C₄)alkyle,
- troisième sous-groupe de composés de formule (I) pour lesquels X est un groupe -NH-, un groupe hétérocyclique à 6 chaînons comportant un ou deux atomes d'azote, de préférence un groupe 1,4-pipérazinylene ou un groupe 1,4-pipéridinylene,
- quatrième sous-groupe de composés de formule (I) pour lesquels R9 et R10 représentent un atome d'hydrogène,
- cinquième sous-groupe de composés de formule (I) pour lesquels Rₐ, R_{b}, R_{c}, R_{d}, Rₑ et R_{f} représentent un atome d'hydrogène,
- sixième sous-groupe de composés de formule (I) pour lesquels Y est un groupe -NR11-, avec R11 représentant un atome d'hydrogène, un groupe (C₁-C₄)alkyle ou un groupe -(CH₂)ₛ-NH₂ où s est égal à 1, 2 ou 3,
- septième sous-groupe de composés de formule (I) pour lesquels m est égal à 2, 3, 4, ou 5, plus préférentiellement à 2 ou 3,
- huitième sous-groupe de composés de formule (I) pour lesquels n est égal à 2, 3, 4 ou 5, plus préférentiellement à 2, 3 ou 4,
- neuvième sous-groupe de composés de formule (I) pour lesquels m est différente de 4,
- dixième sous-groupe de composés de formule (I) pour lesquels o est égal à 2 ou 3,
- onzième sous-groupe de composés de formule (I) pour lesquels le groupe - NHR' est choisi parmi : 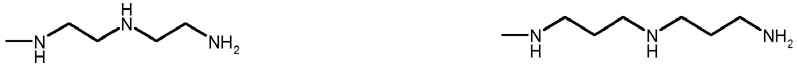 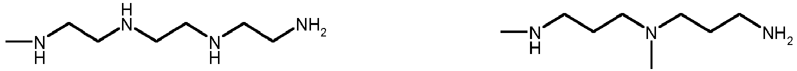 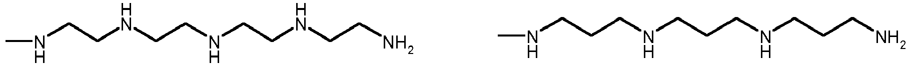 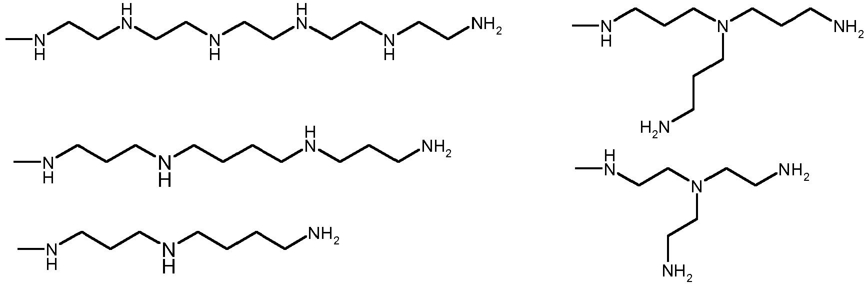 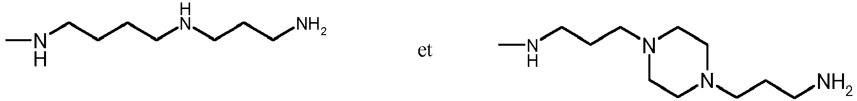 ou
- ou par la combinaison des sous-groupes tels que définis ci-dessus.

Selon un mode de réalisation particulier, la présente invention est relative à un composé tel que défini précédemment, caractérisé en ce qu'il représente la formule (I') 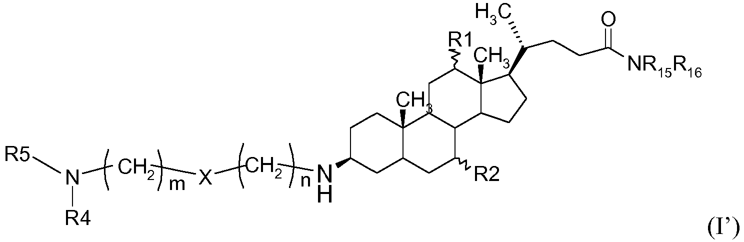 dans laquelle
R1 et R2 sont tels que définis en revendication 1 ou 2,
R15 et R16 représentent indépendamment un atome d'hydrogène ou un groupe (C₁-C₈)alkyle,
n représente le nombre entier 2, 3 ou 4,
m représente le nombre entier 2, 3 ou 4,
X représente un groupe -NR11- ou un groupe hétérocyclique divalent comprenant un ou deux atomes d'azote, à 5 ou 6 chaînons, tel qu'un groupe 1,4-pipérazinylene ou un groupe 1,4-pipéridinylene,
R4 et R11 représentent indépendamment un atome d'hydrogène, un groupe (C₁-C₈)alkyle ou un groupe -(CH₂)ₛ-NH₂,
R5 représente un atome d'hydrogène, un groupe -(CH₂)ₚ-NH₂, un groupe -(CH₂)ₚ-NH-(CH₂)_{q}-NH₂ ou un groupe -(CH₂)ₚ-NH-(CH₂)_{q}-NH-(CH₂)ᵣ-NH₂,
p, q, r et s représentent indépendamment un nombre entier pouvant varier entre 1 et 5,
ainsi que les stéréoisomères, mélanges de stéréoisomères, et/ou sels pharmaceutiquement acceptables de celui-ci.

Parmi les composés de formule (I) et (I') on préfère les composés pour lesquels n et m sont égaux à 3.

Selon un mode de réalisation particulier, la présente invention est relative à un composé tel que défini précédemment, caractérisé en ce que R15 et R16 représentent indépendamment un atome d'hydrogène ou un groupe (C₁-C₄)alkyle tel qu'un méthyle ou un isoproyle.

Selon un mode de réalisation particulier, la présente invention est relative à un composé tel que défini précédemment, caractérisé en ce que n est égal à 2 et m est égal à 3, n est égal à 2 et m est égal à 2, n est égale à 3 et m est égal à 4 ou bien n est égal à 3 et m est égal à 3.

Selon un mode de réalisation particulier, la présente invention est relative à un composé tel que défini précédemment, caractérisé en ce que X représente un groupe - NR11- ou un groupe 1,4-pipérazinylene et R4 et R11 représentant indépendamment un atome d'hydrogène, un groupe méthyle ou un groupe -(CH₂)ₛ-NH₂, dans lequel s est égal à 2 ou 3.

Selon un mode de réalisation particulier, la présente invention est relative à un composé tel que défini précédemment, caractérisé en ce que R5 représente un atome d'hydrogène, un groupe -(CH₂)ₚ-NH₂, un groupe -(CH₂)ₚ-NH-(CH₂)_{q}-NH₂ ou un groupe -(CH₂)ₚ-NH-(CH₂)_{q}-NH-(CH₂)ᵣ-NH₂, avec p est égal à 2 ou 3, q est égal à 2 et r est égal à 2.

Selon un mode de réalisation particulier n est égal à m.
La présente invention est également relative à un composé de formule (Ia) 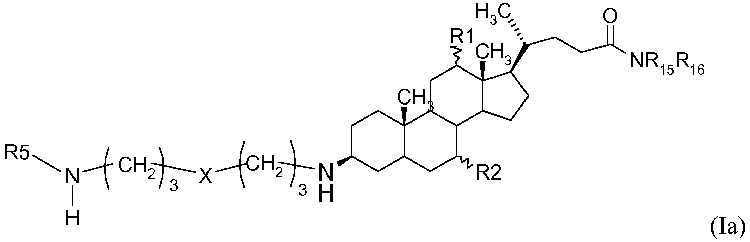 dans laquelle
R15, R16, R1 et R2 sont tels que définis précédemment,
X représente un groupe -NH- ou un groupe 1,4-pipérazinylene,
R5 représente un atome d'hydrogène ou un groupe -(CH₂)ₚ-NH₂, avec p est égal à 2 ou 3,
ainsi que les stéréoisomères, mélanges de stéréoisomères, et/ou sels pharmaceutiquement acceptables de celui-ci.

Selon un mode de réalisation préféré, la présente invention porte sur les composés de formule (Ia) pour lesquels R15 est un atome d'hydrogène et R16 est un groupe méthyle ou isopropyle ou encore R15 et R16 sont tous les deux un groupe éthyle.

La présente invention est également relative à un composé de formule (Ib) 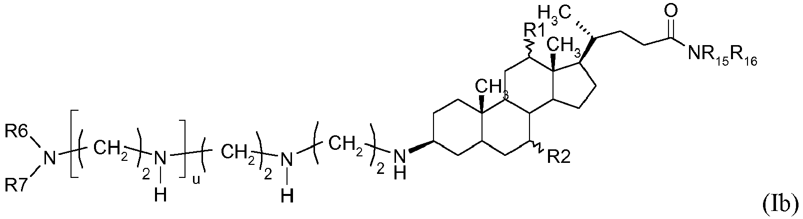 dans laquelle
R15, R16, R1 et R2 sont tels que définis précédemment,
u est égal à 0, 1, 2 ou 3, préférentiellement à 1, 2 ou 3,
R6 et R7 représentent indépendamment un atome d'hydrogène ou un groupe (C₁-C₈)alkyle, de préférence un atome d'hydrogène ou un groupe (C₁-C₄)alkyle,
ainsi que les stéréoisomères, mélanges de stéréoisomères, et/ou sels pharmaceutiquement acceptables de celui-ci.

La présente invention est également relative à un composé de formule (Ic) 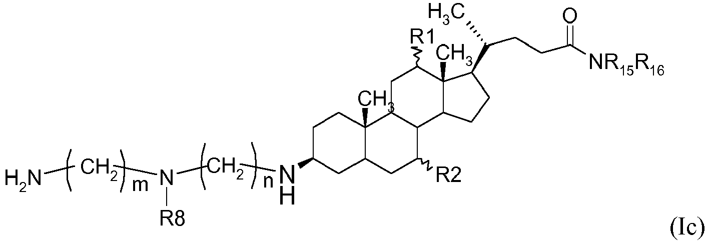 dans laquelle
R15, R16, R1, R2, n et m sont tels que définis précédemment, et
R8 représente un groupe (C₁-C₈)alkyle, de préférence un groupe méthyle, ou un groupe -(CH₂)ₛ-NH₂, avec s étant un nombre entier pouvant varier entre 1 et 5, de préférence égal à 2 ou 3,
ainsi que les stéréoisomères, mélanges de stéréoisomères, et/ou sels pharmaceutiquement acceptables de celui-ci.

Selon un mode de réalisation préféré, la présente invention porte sur les composés de formule (Ic) pour lesquels lorsque R8 représente un groupe -(CH₂)ₛNH₂, alors n =m=s.

Selon un mode de réalisation préféré, la présente invention porte sur les composés de formule (Ic) pour lesquels lorsque R8 représente un groupe méthyle, alors n est égal à m.

La présente invention est également relative à un composé de formule (Id) 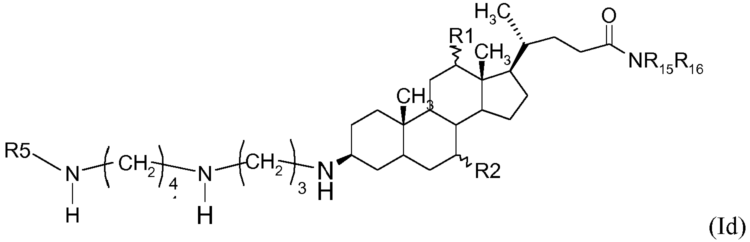 dans laquelle
R15, R16, R1 et R2 sont tels que définis précédemment,
R5 représente un groupe -(CH₂)ₚ-NH₂, avec p est égal à 2 ou 3,
ainsi que les stéréoisomères, mélanges de stéréoisomères, et/ou sels pharmaceutiquement acceptables de celui-ci.

La présente invention est également relative à un composé de formule (Ie) 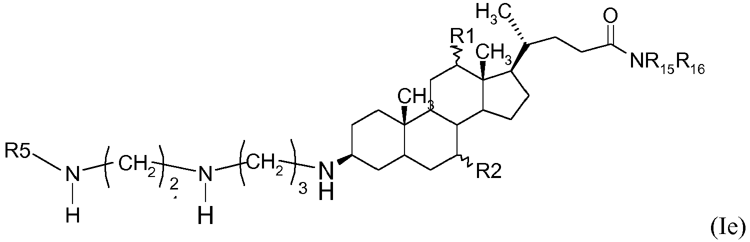 dans laquelle
R15, R16, R1 et R2 sont tels que définis précédemment,
R5 représente un groupe -(CH₂)ₚ-NH₂, avec p est égal à 2 ou 3,
ainsi que les stéréoisomères, mélanges de stéréoisomères, et/ou sels pharmaceutiquement acceptables de celui-ci.

Selon un mode de réalisation particulièrement préféré, la présente invention concerne un composé de formule (Ia) et de formule (Ic) tels que définies ci-dessus. On peut en particulier citer comme composés représentants de ce mode de réalisation particulier les composés (5) et (15) tels que définis ci-après.

Selon un mode de réalisation préféré de la présente invention, un composé de formule (I) est choisi parmi :
- **(1)** 3β-norspermino-N-isopropyl-désoxycholamide,
- **(2)** 3β-norspermidino-N-isopropyl-désoxycholamide,
- **(3)** 3β-(1,4-Bis(3-aminopropyl)pipérazine)-N-isopropyl-désoxycholamide,
- **(4)** 3β-norspermino-N-isopropyl-cholamide,
- **(5)** 3β-norspermidino-N-isopropyl-cholamide,
- **(6)** 3β-(1,4-Bis(3-aminopropyl)pipérazine)-N-isopropyl-désoxycholamide,
- **(7)** 3β-norspermino-N-isopropyl-chénodésoxycholamide,
- **(8)** 3β-norspermidino-N-isopropyl-chénodésoxycholamide,
- **(9)** 3β-(1,4-Bis(3-aminopropyl)pipérazine)-N-isopropyl-chénodésoxycho lamide,
- **(10)** 3β-norspermino-N-méthyl-chénodésoxycholamide,
- **(11)** 3β-norspermidino-N-méthyl-chénodésoxycholamide,
- **(12)** 3β-(1,4-Bis(3-aminopropyl)pipérazine)-N-méthyl-chénodésoxycho lamide,
- **(13)** 3β-norspermidino-N,N-diéthyl-chénodésoxycholamide,
- **(14)** 3β-norspermino-N-isopropyl-ursodésoxycholamide,
- **(15)** 3β-norspermidino-N-isopropyl-ursodésoxycholamide,
- **(16)** 3β-(1,4-Bis(3-aminopropyl)pipérazine)-N-isopropyl-ursodésoxycholamide,
- **(17)** 3β-norspermino-N-isopropyl-lithocholamide,
- **(18)** 3β-norspermidino-N-isopropyl-lithocholamide,
- **(19)** 3β-(1,4-Bis(3-aminopropyl)pipérazine)-N-isopropyl-lithocholamide,
- **(20)** 3 β-(pentaéthylènehexamine)-Nlamide,
- **(21)** 3β-(pentaéthylènehexamine)-N-isopropyl-cholamide,
- **(22)** 3 β-(pentaéthylènehexamine)-N-isopropyl-chénodésoxycholamide,
- **(23)** 3 β-(pentaéthylènehexamine)-N-isopropyl-ursodésoxycholamide,
- **(24)** N-isopropyl-3β-pentaéthylènehexamine-désoxycholamide,
- **(25)** 3β-(1,4-Bis(3-aminopropyl)pipérazine)-N-isopropyl-désoxycholamide,
- **(26)** 3 β-(Bis(3-aminopropyl)méthylamine)-N-isopropyl-cholamide,
- **(27)** 3β-(Bis(3-aminopropyl)méthylamine)-N-isopropyl-chénodésoxycho lamide,
- **(28)** 3β-(Bis(3-aminopropyl)méthylamine)-N-isopropyl-ursodésoxycholamide,
- **(29)** 3β-(Bis(3-aminopropyl)méthylamine)-N-isopropyl-lithocholamide,
- **(30)** 3β-spermino-N-isopropyl-désoxycholamide,
- **(31)** 3β-spermino-N-isopropyl-cholamide,
- **(32)** 3β-spermino-N-isopropyl-chénodésoxycholamide,
- **(33)** 3β-spermino-N-méthyl-désoxycholamide,
- **(34)** 3β-spermino-N,N-diéthyl-chénodésoxycholamide,
- **(35)** 3β-spermino-N-isopropyl-ursodésoxycholamide,
- **(36)** 3β-spermino-N-isopropyl-lithocholamide,
- **(37)** 3β-norspermidino-N-diisopropyl-chénodésoxycholamide,
- **(38)** 3β-norspermidino-N-cyclohexyl-chénodésoxycholamide,
- **(39)** 3β-norspermino-N,N-diéthyl-chénodésoxycholamide,
- **(40)** 3β-norspermino-N,N-diisopropyl-chénodésoxycholamide,
- **(42)** 3β-(Tris(3-aminopropyl)amine)-N-isopropyl-désoxycholamide,
- **(43)** 3β-(Tris(3-aminopropyl)amine)-N-isopropyl-cholamide,
- **(44)** 3 β-(Tris(3-aminopropyl)amine)-N,N-diéthyl-chénodésoxycholamide,
- **(45)** 3 β-(Tris(2-aminoéthyl)amine)-N-isopropyl-chénodésoxycholamide,
- **(46)** 3β-(Tris(3-aminopropyl)amine)-N-isopropyl-chénodésoxycholamide,
- **(47)** 3β-(Tris(3-aminopropyl)amine)-N-cyclohexyl-chénodésoxycholamide,
- **(48)** 3β-(Tris(3-aminopropyl)amine)-N-isopropyl-ursodésoxycholamide,
- **(49)** 3β-(Tris(3-aminopropyl)amine)-N-isopropyl-lithocholamide,
- **(50)** 3β-spermino-N,N-diisopropyl-chénodésoxycholamide,
- **(51)** 3β-spermino-N-cyclohexyl-chénodésoxycholamide,
- **(52)** 3β-spermidino-N-isopropyl-chénodésoxycholamide,
- **(53)** 3β-(Bis(3-aminopropyl)éthylènediamine)-N,N-diéthyl-chénodésoxy cholamide,
- **(54)** 3β-(Bis(3-aminopropyl)éthylènediamine)-N,N-diisopropyl-chénodésoxy cholamide,
- **(55)** Mélange 50/50 de 3β-spermidino-N-isopropyl-chénodésoxycholamide et de 3β-N-[4'N-(3'-aminopropyl)aminobutyl]amino-N-isopropyl-chénodésoxycholamide,
- **(56)** 3β-(Tris(3-aminopropyl)amine)-N,N-diisopropyl-chénodésoxy cholamide,
ou l'un de ses sels pharmaceutiquement acceptables.

Les composés de l'invention peuvent exister sous forme de bases libres ou de sels d'addition à des acides pharmaceutiquement acceptables.

Selon un mode de réalisation particulier de l'invention, de tels sels d'addition d'acides pharmaceutiquement acceptables comprennent le bromhydrate, le tartrate, le citrate, le trifluoroacétate, l'ascorbate, le chlorhydrate, le triflate, le maléate, le mesylate, le formate, l'acétate et le fumarate, et plus particulièrement le chlorhydrate.

Les composés de formule (I), (I'), (Ia), (Ib), (Ic), (Id) et (Ie) ainsi que les composés (1) à (56) peuvent se présenter sous forme de solvates tels que des hydrates. L'invention comprend ces solvates.

Un composé de formule (I), (I'), (la), (Ib), (Ic), (Id) ou (Ie) peut comprendre un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, sont inclus dans la portée de la présente invention. D'une manière générale, dans le cadre de la présente invention, lorsqu'une liaison est représentée par le symbole 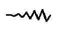 , cela signifie que le groupe porté par le carbone considéré peut-être en arrière ou en avant du plan de représentation de la molécule. Ainsi, la stéréochimie résultante du carbone portant ce groupe peut être S ou R.

Selon un autre aspect, la présente invention concerne un composé de formule (I), (I'), (la), (Ib), (Ic), (Id) ou (Ie) ou encore un composé (1) à (56) ou l'un de ses sels pharmaceutiquement acceptable, pour son utilisation à titre de médicament.

Selon un autre aspect, la présente invention concerne un composé de formule (I), (I'), (la), (Ib), (Ic), (Id) ou (Ie) pour son utilisation pour prévenir et/ou inhiber et/ou traiter les infections bactériennes, fongiques, virales ou parasitaires chez l'homme ou l'animal.

Selon la présente invention, le terme «prévenir» ou «prévention» signifie que pour réduire le risque d'apparition ou de ralentir l'apparition d'un phénomène donné, à savoir une infection bactérienne, fongique, virale ou parasitaire.

Les composés de la présente invention peuvent être préparés par des procédés classiques de synthèse organique pratiquée par l'homme du métier. Le schéma général de réaction décrit ci-dessous représente une méthode générale utile pour préparer les composés de la présente invention et n'est pas destiné à en limiter la portée ou l'utilité.

Ainsi, les composés de l'invention peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

Selon un mode de réalisation particulier, les composés de l'invention peuvent être préparés selon le schéma de synthèse 1 ci-dessous.

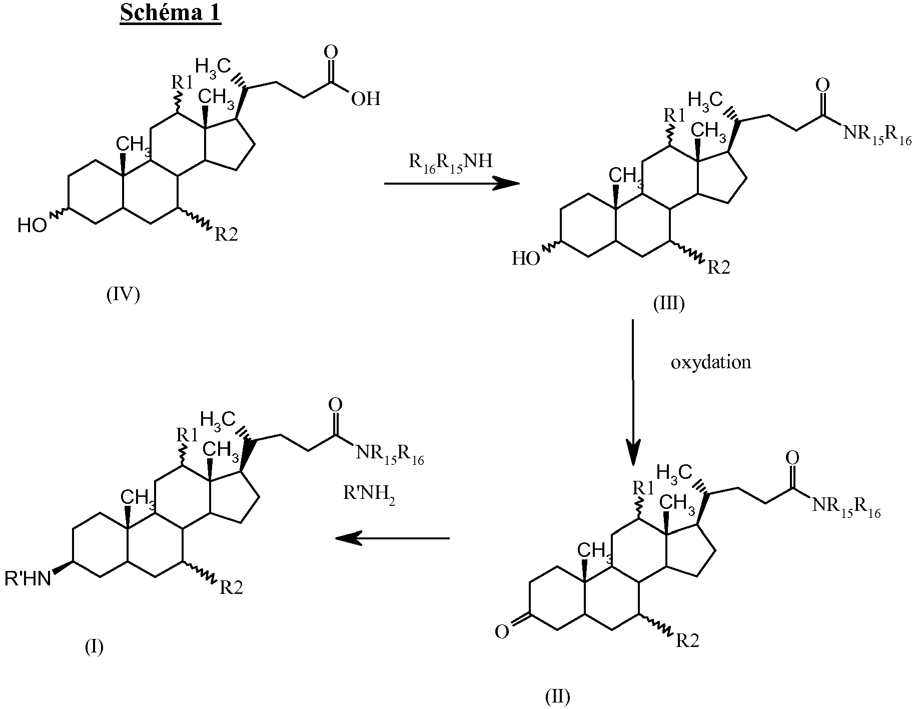

Selon ce schéma 1, un composé de formule (IV), dans laquelle R1 et R2 sont tels que définis précédemment, est mis à réagir, dans un solvant tel que CH₂Cl₂, THF, dioxane, par exemple en présence d'agent de couplage tel que HOBT/DCC, BOP, chloroformiate de méthyle avec un composé de formule R15R16NH, dans laquelle R15 et R16 sont tels que définis précédemment, par exemple à une température comprise entre - 20°C et 20°C, pour obtenir un composé de formule (III).

Toujours selon ce schéma 1, le composé de formule (III) ainsi obtenu est soumis à une oxydation en présence d'un ligand, par exemple du tri-*tert*-butylate d'aluminium, tri-isopropylate d'aluminium ou Ag₂CO₃, dans un solvant tel que par exemple le benzene, le toluène, le cyclohexane ou le trifluorotoluène, par exemple à une température comprise entre 20°C et 100°C, pour obtenir un composé de formule (II).

Toujours selon ce schéma 1, le composé de formule (II) ainsi obtenu est soumis à une amination réductrice par réaction avec un composé de formule R'NH₂, dans laquelle R' est tel que défini précédemment, en présence d'un agent réducteur tel que le tétraisopropylate de titane, le tétraisopropylate de zirconium, le NaBH₃CN, le NaBH₄, ou d'un mélange d'entre eux, préférentiellement le couple tétraisopropylate de titane/NaBH₄, par exemple à une température comprise entre -120°C et -10°C, préférentiellement -80°C et -10°C, pour obtenir le composé de formule (I).

Eventuellement ledit procédé peut également comprendre l'étape consistant à isoler le produit obtenu.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Le composé de départ de formule (IV) est disponible ou peut être préparé selon les méthodes connues de l'homme de l'art et/ou peuvent être préparés par l'application des procédés tels que décrits dans les Exemples ou leurs équivalents chimiques évidents.

Selon une variante préférée, les composés de formule (I) sont synthétisés à partir des composés de formule (IV), tels que définis ci-dessus ou acides biliaires suivants : 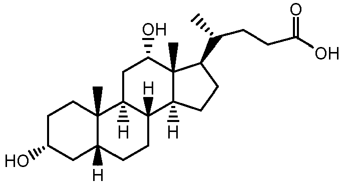 acide désoxycholique 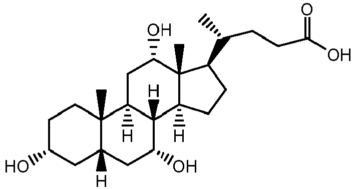 acide cholique 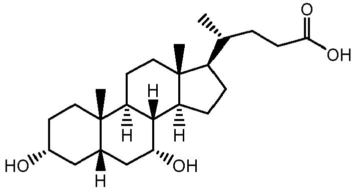 acide chénodésoxycholique 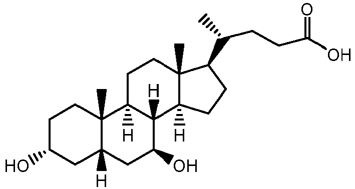 acide ursodésoxycholique 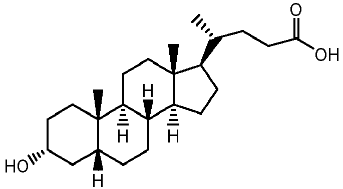 acide lithocholique

Ainsi, selon un autre objet, la présente invention concerne également le procédé de préparation des composés de formule (I) précédemment décrits, comprenant une étape d'amination réductrice du composé de formule (II) 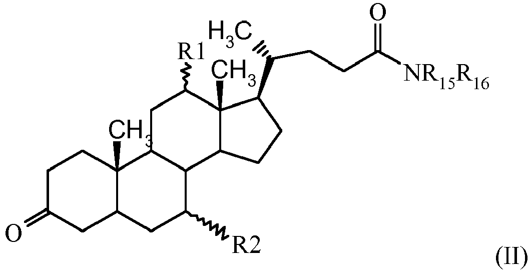 dans laquelle R15, R16, R1 et R2 sont tels que définis précédemment,
avec une amine de formule R'NH2, dans laquelle R' est telle que définie précédemment, en présence d'un agent réducteur pouvant être choisi parmi le tétraisopropylate de titane, le tétraisopropylate de zirconium, le NaBH₃CN, le NaBH₄, ou d'un mélange d'entre eux, préférentiellement le couple tétraisopropylate de titane/NaBH₄, pour obtenir ledit composé de formule (I).

Les structures chimiques et les données spectroscopiques de quelques composés de formule (I) de l'invention sont illustrées respectivement dans le tableau I et le tableau II suivant.

**Table I**

| | | | |
|---|---|---|---|
| 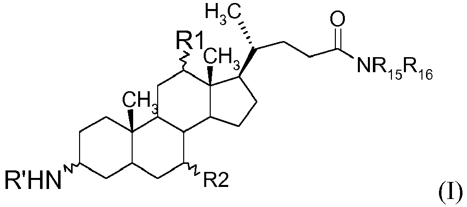 | | | |
| **Formule (Ia)** | | | |
| 1 | 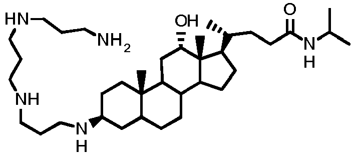 | 2 | 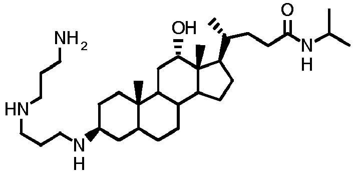 |
| 3 | 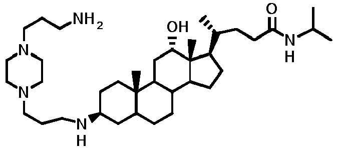 | 4 | 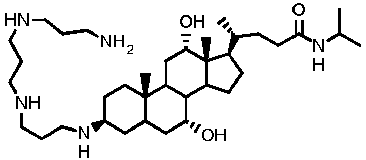 |
| 5 | 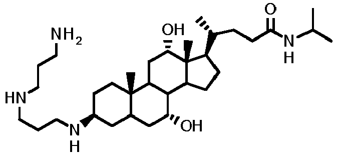 | 6 | 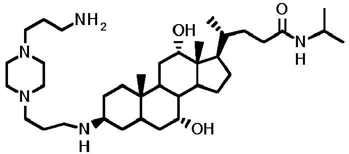 |
| 7 | 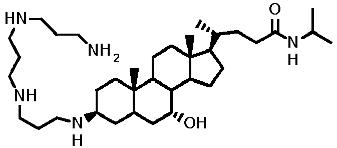 | 8 | 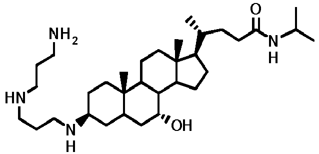 |
| 9 | 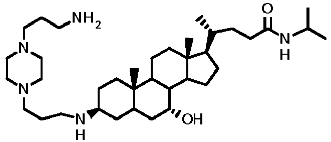 | 10 | 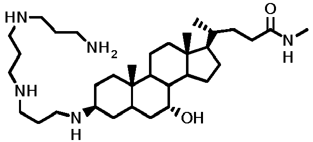 |
| 11 | 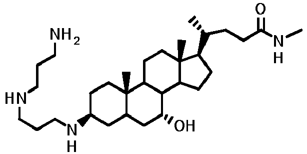 | 12 | 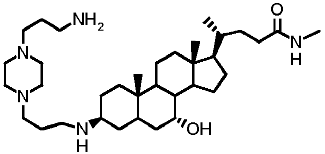 |
| 13 | 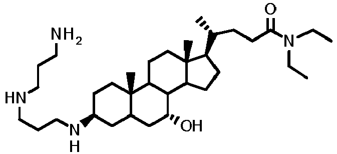 | 14 | 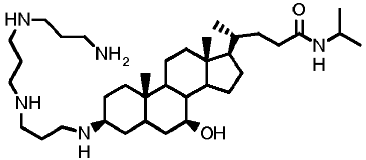 |
| 15 | 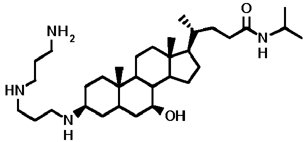 | 16 | 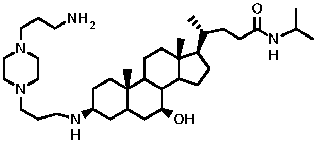 |
| 17 | 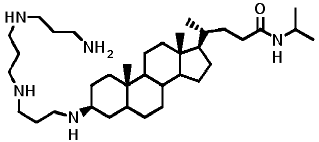 | 18 | 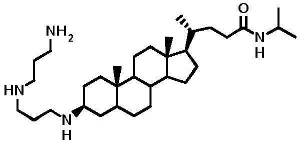 |
| 19 | 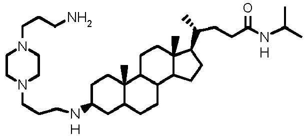 | 37 | 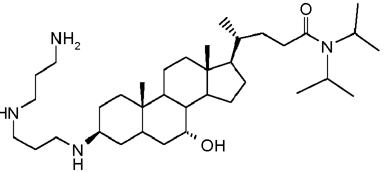 |
| 38 | 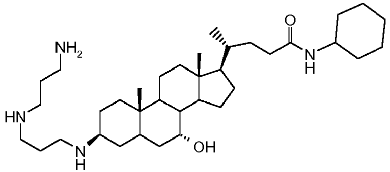 | 39 | 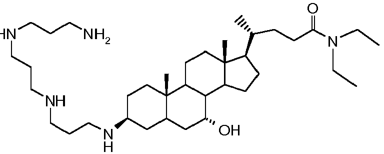 |
| 40 | 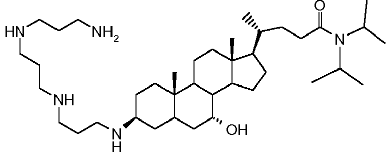 | | |

| **Formule (Ib)** | | | |
|---|---|---|---|
| 20 | 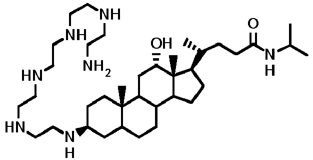 | 21 | 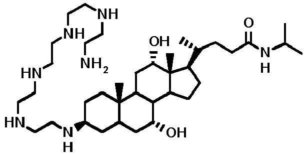 |
| 22 | 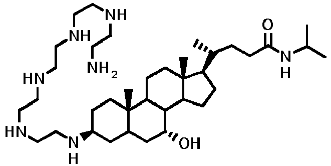 | 23 | 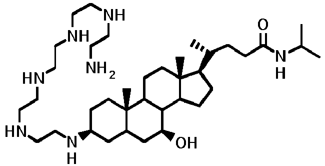 |
| 24 | 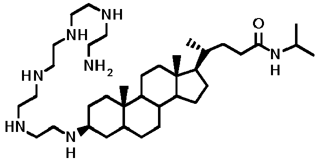 | | |

| **Formule (Ic)** | | | |
|---|---|---|---|
| 25 | 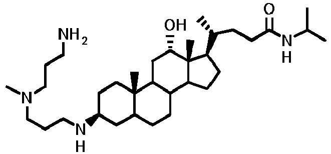 | 26 | 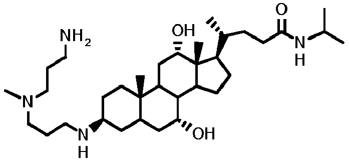 |
| 27 | 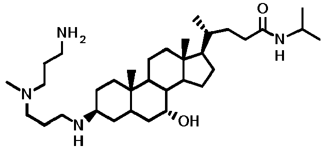 | 28 | 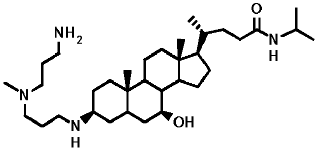 |
| 29 | 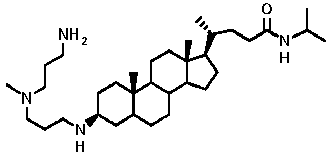 | 42 | 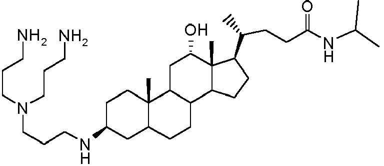 |
| 43 | 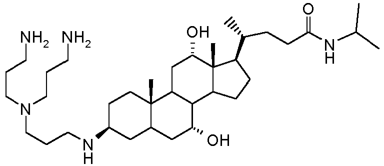 | 44 | 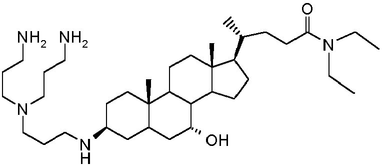 |
| 45 | 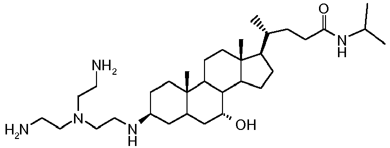 | 46 | 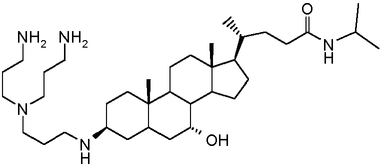 |
| 47 | 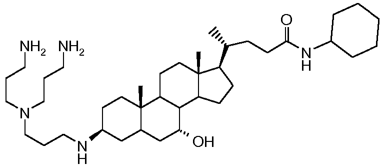 | 48 | 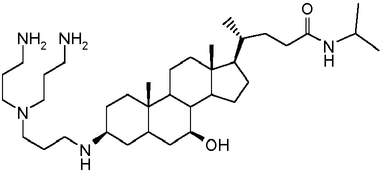 |
| 49 | 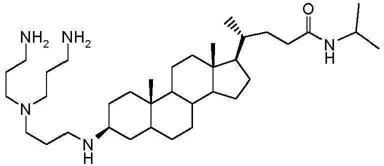 | 56 | 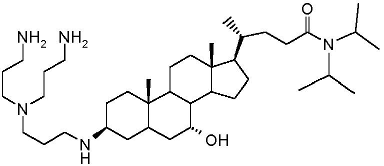 |

| **Formule (Id)** | | | |
|---|---|---|---|
| 30 | 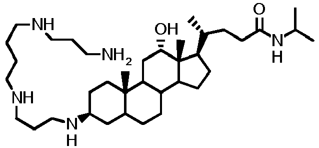 | 31 | 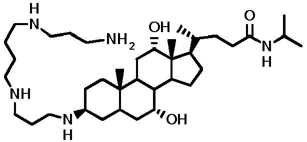 |
| 32 | 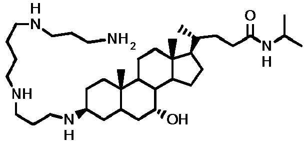 | 33 | 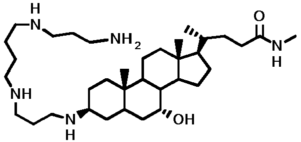 |
| 34 | 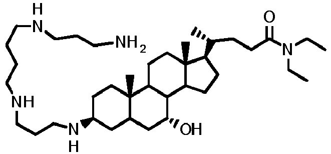 | 35 | 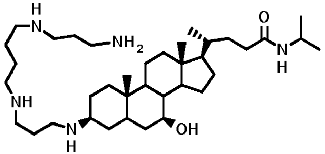 |
| 36 | 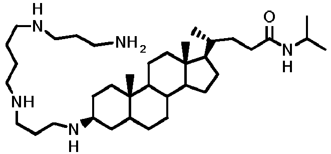 | 50 | 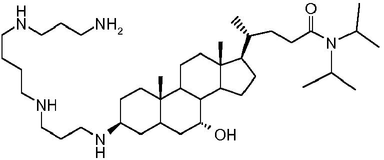 |
| 51 | 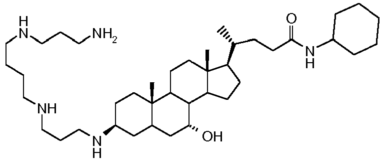 | 52 | 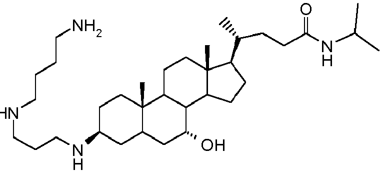 |

| **Formule (Ie)** | | | |
|---|---|---|---|
| 53 | 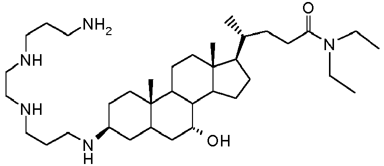 | 54 | 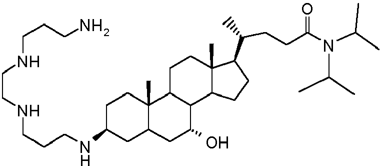 |

L'invention couvre également un mélange 50/50 de 3β-spermidino-N-isopropyl-chénodésoxycholamide et de 3β-N-[4'N-(3'-aminopropyl)aminobutyl]amino-N-isopropyl-chénodésoxycholamide nommé composé (55) dans la description qui va suivre

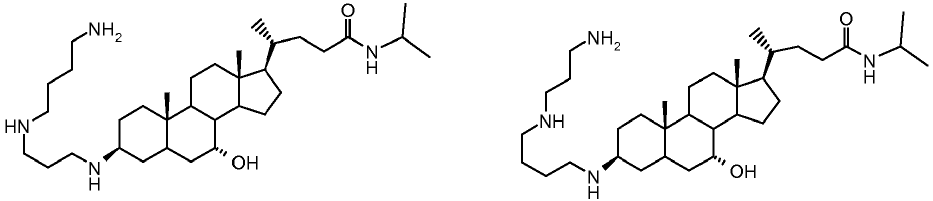

**Tableau II**

| | |
|---|---|
| Les structures chimiques synthétisées ont toutes été vérifiées par une analyse RMN du proton (¹H) et/ou du carbone (¹³C) dans du chloroforme deutéré CDCl₃ ou du méthanol deutéré CD₃OD sur un appareil de type Bruker AC 300. Les déplacements chimiques δ sont exprimés en ppm. Les fréquences d'enregistrements des noyaux ainsi que des références utilisées sont les suivantes : | |
| RMN du ¹H : 300 MHz, Si(CH₃)₄ | |
| RMN du ¹³C : 75 MHz, Si(CH₃)₄ | |
| Les abréviations utilisées pour l'écriture du spectre ¹H sont les suivantes : | |
| - s = singulet | |
| - d = doublet | |
| - t = triplet | |
| - q = quadruplet | |
| - m = massif | |

| **Ex** | **Caractérisation** |
|---|---|
| 1 | **RMN ¹H** (250 MHz, CD₃OD) : δ (ppm) = 3.94 (m, 2H), 2.71-2.59 (m, 13H), 2.21-0.70 (m, 54H). **RMN ¹³C** (63 MHz, CD₃OD) : δ (ppm) = 175.93, 74.19, 59.07, 49.31, 49.00, 48.24, 47.74, 45.85, 44.08, 42.41, 40.78, 37.59, 37.12, 37.01, 35.95, 35.92, 34.94, 34.58, 34.36, 33.56, 30.50, 30.30, 29.98, 28.84, 28.64, 28.27, 27.64, 25.04, 24.08, 22.82, 22.76, 17.85, 13.35 |
| 2 | **RMN ¹H** (250 MHz, CD₃OD) : δ (ppm) = 3.99-3.89 (m, 2H), 2.72-2.51 (m, 9H), 2.25-0.70 (m, 51H). **RMN ¹³C** (63 MHz, CD₃OD) : δ (ppm) = 175.55, 72.85, 59.06, 49.33, 49.06, 48.42, 48.25, 47.76, 45.84, 44.06, 42.41, 40.75, 37.58, 37.10, 37.01, 35.91, 34.95, 34.47, 34.37, 33.55, 33.34, 30.33, 29.98, 28.84, 28.62, 28.19, 27.64, 25.04, 24.07, 22.82, 22.76, 17.84, 13.35 |
| 3 | **RMN ¹H** (250 MHz, CD₃OD) : δ (ppm) = 3.99-3.89 (m, 2H), 2.69-2.38 (m, 17H), 2.27-0.70 (m, 50H). **RMN ¹³C** (63 MHz, CD₃OD) : δ (ppm) = 175.87, 74.13, 59.06, 58.03, 57.50, 54.10, 54.03, 49.30, 48.21, 47.75, 46.29, 44.04, 42.38, 41.16, 37.58, 37.01, 35.92, 34.92, 34.64, 34.35, 33.53, 30.55, 30.00, 28.85, 28.65, 28.34, 27.65, 27.42, 25.06, 24.12, 22.85, 22.79, 17.87, 13.38 |
| 4 | **RMN ¹H** (250 MHz, CD₃OD) : δ (ppm) = 3.96-3.95 (m, 2H), 3.80(m, 1H), 2.76-2.69 (m, 13H), 2.54-0.72 (m, 53H). **RMN ¹³C** (63 MHz, CD₃OD) : δ (ppm) = 175.88, 74.01, 69.00, 59.32, 57.39, 54.01, 48.21, 47.64, 45.53, 43.55, 43.13, 42.39, 41.16, 41.00, 40.54, 37.07, 36.92, 36.73, 36.38, 35.98, 34.43, 33.56, 32.12, 29.65, 28.89, 28.02, 27.45, 24.38, 23.44, 22.84, 22.78, 17.93, 13.16 |
| 5 | **RMN ¹H** (250 MHz, CD₃OD) : δ (ppm) = 3.97-3.94 (m, 2H), 3.80 (m, 1H), 2.90-2.59 (m, 9H), 2.29-0.71 (m, 50H). **RMN ¹³C** (63 MHz, CD₃OD) : δ (ppm) = 175.91, 74.00, 68.98, 59.23, 49.00, 48.22, 47.62, 45.64, 43.42, 43.19, 42.40, 41.15, 40.42, 37.07, 36.74, 36.30, 35.89, 34.43, 33.56, 31.66, 29.69, 28.87, 28.32, 28.05, 27.14, 24.34, 23.36, 22.82, 22.76, 17.90, 13.15 |
| 6 | **RMN ¹H** (250 MHz, CD₃OD) : δ (ppm) = 3.94 (m, 2H), 3.79(m, 1H), 2.76-2.39 (m, 23H), 2.28-0.71 (m, 43H). **RMN ¹³C** (63 MHz, CD₃OD) : δ (ppm) = 175.93, 74.03, 69.03, 59.34, 57.97, 57.44, 54.10, 54.02, 47.66, 46.24, 46.08, 43.65, 43.14, 42.41, 41.21, 41.09, 37.28, 37.07, 36.42, 36.02, 34.43, 33.56, 30.11, 29.74, 28.87, 28.06, 27.92, 26.67, 24.38, 23.48, 22.83, 22.76, 17.91, 13.16 |
| 7 | **RMN ¹H** (250 MHz, CD₃OD) : δ (ppm) = 3.94 (m, 1H), 3.80 (m, 1H), 2.92-2.74 (m, 13H), 2.20-0.69 (m, 54H). **RMN ¹³C** (63 MHz, CD₃OD) : δ (ppm) = 175.88, 68.97, 59.31, 57.60, 51.74, 48.54, 45.26, 43.83, 43.42, 42.43, 41.21, 40.91, 40.32, 37.06, 36.65, 36.06, 35.88, 34.40, 34.21, 33.56, 30.84, 29.44, 29.04, 27.99, 27.01, 24.74, 23.55, 22.81, 22.73, 21.91, 19.07, 12.33 |
| 8 | **RMN ¹H** (250 MHz, CD₃OD) : δ (ppm) = 3.95 (m, 1H), 3.80 (m, 1H), 2.75-2.63 (m, 9H), 2.27-0.70 (m, 51H). **RMN ¹³C** (63 MHz, CD₃OD) : δ (ppm) = 175.82, 69.03, 59.40, 57.51, 51.69, 48.94, 48.28, 45.64, 43.82, 43.61, 42.41, 41.20, 40.94, 40.63, 37.05, 36.76, 36.03, 34.36, 34.19, 33.55, 32.81, 29.57, 29.45, 28.01, 24.77, 23.72, 22.83, 22.76, 21.91, 19.11, 12.37 |
| 9 | **RMN ¹H** (250 MHz, CD₃OD) : δ (ppm) = 3.94 (m, 1H), 3.81 (m, 1H), 3.09-2.82 (m, 17H), 2.20-0.70 (m, 50H). **RMN ¹³C** (63 MHz, CD₃OD) : δ (ppm) = 175.88, 68.81, 59.37, 57.67, 55.76, 52.43, 52.27, 52.16, 52.09, 51.82, 51.99, 43.83, 43.09, 42.44, 41.24, 40.90, 40.75, 37.07, 36.67, 36.46, 34.43, 34.24, 34.16, 33.56, 30.91, 30.89, 29.46, 25.36, 24.74, 23.55, 22.80, 22.73, 19.08, 12.33 |
| 10 | **RMN ¹H** (250 MHz, CD₃OD) : δ (ppm) = 3.79 (m, 1H), 3.65 (s, 3H), 2.73-2.61 (m, 13H), 2.24-0.69 (m, 48H). **RMN ¹³C** (63 MHz, CD₃OD) : δ (ppm) = 176.40, 69.03, 59.45, 57.44, 52.18, 51.67, 48.95, 48.43, 45.73, 43.81, 43.69, 41.20, 40.96, 40.72, 37.57, 37.21, 36.80, 36.10, 34.18, 33.21, 32.39, 31.97, 30.13, 30.02, 29.41, 28.29, 24.79, 23.79, 21.95, 19.00, 12.42 |
| 11 | **RMN ¹H** (250 MHz, CD₃OD) : δ (ppm) = 3.79 (m, 1H), 3.65 (s, 3H), 2.72-2.63 (m, 9H), 2.44-0.69 (m, 45H). **RMN ¹³C** (63 MHz, CD₃OD) : δ (ppm) = 176.38, 68.95, 59.31, 57.34, 52.02, 51.57, 48.86, 48.19, 45.55, 43.70, 43.54, 41.10, 40.85, 40.55, 37.00, 36.79, 36.67, 35.93, 34.09, 32.77, 32.28, 31.87, 29.57, 29.29, 27.97, 24.67, 23.63, 21.83, 18.87, 12.28 |
| 12 | **RMN ¹H** (250 MHz, CD₃OD) : δ (ppm) = 3.79 (m, 1H), 3.65 (s, 3H), 2.71-0.42 (m, 17H), 2.32-0.70 (m, 44H). **RMN ¹³C** (63 MHz, CD₃OD) : δ (ppm) = 176.46, 69.03, 59.36, 58.05, 57.47, 54.13, 53.99, 52.18, 51.69, 46.18, 43.82, 43.66, 41.22, 41.12, 40.97, 37.58, 37.18, 37.18, 36.90, 36.79, 36.07, 34.21, 32.39, 31.97, 30.35, 29.40, 28.36, 26.93, 24.77, 23.75, 21.93, 18.98, 12.40 |
| 13 | **RMN ¹H** (250 MHz, CD₃OD) : δ (ppm) = 3.79 (m, 1H), 3.38 (m, 4H), 2.72-2.59 (m, 8H), 2.38-0.70 (m, 51H). **RMN ¹³C** (63 MHz, CD₃OD) : δ (ppm) = 175.42, 69.07, 59.48, 57.47, 51.66, 49.08, 48.42, 45.79, 43.83, 43.74, 41.62, 41.21, 40.98, 40.79, 37.86, 37.30, 37.18, 36.83, 36.11, 34.18, 33.58, 33.23, 31.15, 30.41, 29.48, 28.54, 24.80, 23.82, 21.94, 19.25, 14.84, 13.47, 12.42 |
| 14 | **RMN ¹H** (250 MHz, CD₃OD) : δ (ppm) = 3.94 (m, 1H), 3.46 (m, 1H), 2.89-2.65 (m, 13H), 2.54-0.71 (m, 54H). **RMN ¹³C** (63 MHz, CD₃OD) : δ (ppm) = 175.87, 72.04, 58.88, 57.64, 56.75, 48.31, 46.72, 45.74, 44.94, 44.66, 44.46, 42.42, 41.70, 40.80, 40.56, 38.82, 36.97, 36.68, 35.85, 35.75, 34.97, 34.40, 33.60, 32.19, 29.84, 29.71, 29.66, 28.10, 24.25, 22.81, 22.76, 22.54, 19.22, 12.80 |
| 15 | **RMN ¹H** (250 MHz, CD₃OD) : δ (ppm) = 3.94 (m, 1H), 3.47 (m, 1H), 2.85-2.49 (m, 9H), 2.24-0.71 (m, 51H). **RMN ¹³C** (63 MHz, CD₃OD) : δ (ppm) = 175.87, 72.06, 58.89, 57.65, 56.74, 48.94, 48.39, 45.85, 44.95, 44.67, 44.50, 42.42, 41.72, 40.81, 40.70, 38.87, 36.97, 36.75, 35.86, 35.78, 35.25, 34.39, 33.60, 33.06, 30.26, 29.84, 28.10, 24.28, 22.81, 22.76, 22.54, 19.23, 12.80 |
| 16 | **RMN ¹H** (250 MHz, CD₃OD) : δ (ppm) = 3.94 (m, 1H), 3.47 (m, 1H), 2.70-2.38 (m, 17H), 2.20-0.71 (m, 50H). **RMN ¹³C** (63 MHz, CD₃OD) : δ (ppm) = 175.74, 72.01, 58.86, 58.05, 57.67, 56.74, 54.13, 54.03, 46.41, 44.94, 44.65, 44.48, 44.39, 42.38, 41.73, 41.16, 40.81, 38.93, 36.97, 36.80, 35.89, 35.78, 35.49, 34.39, 33.59, 30.51, 29.87, 28.33, 28.11, 27.37, 24.37, 22.87, 22.81, 22.56, 19.29, 12.87 |
| 17 | **RMN ¹H** (250 MHz, CD₃OD) : δ (ppm) = 3.94 (m, 1H), 2.96-2.77 (m, 13H), 2.52-0.69 (m, 55H). **RMN ¹³C** (63 MHz, CD₃OD) : δ (ppm) = 175.89, 59.07, 58.00, 57.69, 47.93, 44.06, 42.44, 41.91, 41.59, 39.96, 37.29, 36.99, 36.03, 34.40, 33.53, 29.42, 29.01, 28.75, 28.24, 26.01, 25.37, 23.96, 23.93, 22.79, 22.73, 22.07, 19.03, 12.61 |
| 18 | **RMN ¹H** (250 MHz, CD₃OD) : δ (ppm) = 3.94 (m, 1H), 2.74-2.62 (m, 9H), 2.25-0.69 (m, 52H). **RMN ¹³C** (63 MHz, CD₃OD) : δ (ppm) = 175.86, 59.04, 58.06, 57.63, 48.92, 48.37, 45.83, 44.07, 43.92, 42.43, 41.97, 41.67, 40.67, 37.37, 37.05, 36.98, 36.26, 34.35, 33.52, 32.90, 30.02, 29.44, 28.55, 28.11, 27.80, 25.42, 24.28, 22.81, 22.75, 22.11, 19.06, 12.65 |
| 19 | **RMN ¹H** (250 MHz, CD₃OD) : δ (ppm) = 3.94 (m, 1H), 2.72-2.39 (m, 15H), 2.23-0.96 (m, 53H). **RMN ¹³C** (63 MHz, CD₃OD) : δ (ppm) = 175.88, 59.01, 58.12, 58.02, 57.66, 57.50, 54.14, 54.02, 46.36, 44.09, 43.90, 42.43, 42.00, 41.69, 41.09, 37.37, 37.06, 36.99, 36.27, 34.40, 34.35, 33.53, 30.17, 29.44, 28.55, 28.28, 27.83, 27.12, 25.42, 24.28, 22.81, 22.75, 22.11, 19.05, 12.65 |
| 20 | **RMN ¹H** (250 MHz, CD₃OD) : δ (ppm) = 3.99-3.89 (m, 2H), 2.85-2.54 (m, 21H), 2.27-0.70 (m, 50H). **RMN ¹³C** (63 MHz, CD₃OD) : δ (ppm) = 175.87, 74.06, 59.10, 57.27, 55.25, 55.05, 54.99, 54.27, 54.18, 49.31, 48.23, 47.74, 46.97, 46.32, 46.18, 43.95, 42.38, 37.55, 36.99, 35.84, 34.94, 34.39, 33.52, 29.98, 28.84, 28.55, 27.60, 25.02, 24.02, 22.84, 22.78, 22.27, 17.86, 13.37 |
| 21 | **RMN ¹H** (250 MHz, CD₃OD) : δ (ppm) = 3.96-3.91 (m, 2H), 3.79 (m, 1H), 2.85-2.46 (m, 24H), 2.24-0.93 (m, 43H), 0.71 (s, 3H). **RMN ¹³C** (63 MHz, CD₃OD) : δ (ppm) = 175.95, 74.10, 69.11, 59.54, 55.35, 54.48, 54.27, 54.07, 49.68, 48.19, 47.66, 47.07, 46.21, 43.73, 43.11, 42.41, 41.89, 41.21, 39.08, 37.77, 37.14, 37.06, 36.48, 36.06, 34.40, 33.55, 29.70, 28.87, 28.34, 28.04, 24.38, 23.52, 22.82, 22.76, 17.91, 13.15 |
| 22 | **RMN ¹H** (250 MHz, CD₃OD) : δ (ppm) = 3.94 (m, 1H), 3.79 (m, 1H), 2.85-2.43 (m, 23H), 2.37-0.69 (m, 48H). **RMN ¹³C** (63 MHz, CD₃OD) : δ (ppm) = 175.87, 69.10, 59.59, 58.60, 57.52, 55.37, 54.48, 54.28, 54.08, 51.70, 43.84, 42.42, 41.92, 41.23, 40.97, 39.11, 37.23, 37.05, 36.82, 36.10, 34.37, 34.21, 33.56, 29.44, 28.64, 28.59, 24.78, 23.76, 22.83, 22.76, 21.94, 19.10, 12.36 |
| 23 | **RMN ¹H** (250 MHz, CD₃OD) : δ (ppm) = 3.94 (m, 1H), 3.47 (m, 1H), 2.84-2.46 (m, 22H), 2.26-0.71 (m, 49H). **RMN ¹³C** (63 MHz, CD₃OD) : δ (ppm) = 175.81, 72.04, 57.68, 56.74, 54.29, 54.26, 49.93, 47.10, 46.93, 46.37, 46.23, 44.94, 44.66, 44.50, 42.41, 41.93, 41.73, 40.83, 39.12, 38.90, 36.97, 36.77, 35.78, 34.39, 33.59, 29.85, 28.41, 28.10, 24.33, 22.84, 22.78, 22.54, 19.25, 12.83 |
| 24 | **RMN ¹H** (250 MHz, CD₃OD) : δ (ppm) = 3.95 (m, 1H), 2.84-2.53 (m, 22H), 2.27-0.69 (m, 50H). **RMN ¹³C** (63 MHz, CD₃OD) : δ (ppm) = 175.80, 58.07, 57.63, 54.28, 52.23, 44.06, 43.91, 42.41, 41.99, 41.81, 41.67, 37.37, 36.97, 36.27, 34.39, 34.34, 33.51, 29.43, 28.58, 28.42, 28.32, 27.83, 25.44, 24.33, 22.84, 22.78, 22.11, 19.09, 12.69 |
| 25 | **RMN ¹H** (250 MHz, CD₃OD) : δ (ppm) = 3.99-3.91 (m, 2H), 2.68-0.70 (m, 62H). **RMN ¹³C** (63 MHz, CD₃OD) : δ (ppm) = 175.84, 74.13, 59.10, 57.14, 56.62, 56.57, 49.29, 47.69, 46.19, 44.06, 42.47, 42.37, 41.13, 37.58, 36.99, 35.91, 34.92, 34.66, 34.33, 33.53, 31.06, 30.98, 29.98, 28.84, 28.65, 28.33, 27.92, 27.65, 25.06, 24.14, 22.86, 22.80, 17.87, 13.39 |
| 26 | **RMN ¹H** (250 MHz, CD₃OD) : δ (ppm) = 3.94-3.89 (m, 2H), 2.75-2.40 (m, 14H), 2.24-0.85 (m, 45H), 0.71 (s, 3H). **RMN ¹³C** (63 MHz, CD₃OD) : δ (ppm) = 175.98, 74.08, 69.06, 59.99, 57.30, 56.64, 56.60, 50.72, 48.22, 47.67, 42.43, 41.05, 37.09, 37.03, 36.43, 36.00, 34.43, 34.37, 33.56, 30.61, 30.52, 28.89, 27.61, 24.36, 23.46, 22.81, 22.74, 17.89, 13.15 |
| 27 | **RMN ¹H** (250 MHz, CD₃OD) : δ (ppm) = 3.94 (m, 1H), 3.79 (m, 1H), 2.70-0.69 (m, 51H). **RMN ¹³C** (62 MHz, CD₃OD) : δ (ppm) = 175.80, 69.04, 59.50, 57.50, 57.13, 56.55, 51.69, 46.04, 43.82, 43.69, 42.43, 42.39, 41.21, 41.05, 40.96, 37.60, 37.21, 37.04, 36.80, 36.08, 34.35, 34.19, 33.54, 30.69, 29.45, 28.35, 27.52, 24.78, 23.77, 22.84, 22.77, 21.93, 19.12, 12.39 |
| 28 | **RMN ¹H** (250 MHz, CD₃OD) : δ (ppm) = 3.94 (m, 1H), 3.47 (m, 1H), 2.75-2.32 (m, 12H), 2.24-0.71 (m, 50H). **RMN ¹³C** (62 MHz, CD₃OD) : δ (ppm) = 175.84, 72.05, 58.94, 57.64, 57.01, 56.73, 56.57, 46.17, 44.93, 44.82, 44.66, 44.48, 42.41, 41.70, 41.05, 40.80, 36.95, 35.77, 34.41, 34.39, 33.59, 30.54, 30.42, 29.84, 28.09, 27.74, 24.30, 22.83, 22.76, 19.24, 12.82 |
| 29 | **RMN ¹H** (250 MHz, CD₃OD) : δ (ppm) =3.94 (m, 1H), 2.75-2.37 (m, 10H), 2.24-0.69 (m, 53H). **RMN ¹³C** (62 MHz, CD₃OD) : δ (ppm) = 175.81, 58.05, 57.59, 56.57, 46.26, 44.06, 43.98, 42.49, 42.41, 41.97, 41.66, 41.14, 37.39, 37.22, 36.97, 36.31, 34.37, 33.51, 31.09, 31.04, 29.43, 27.82, 25.44, 24.36, 22.83, 22.77, 22.11, 19.08, 12.69 |
| 30 | **RMN ¹H** (250 MHz, CD₃OD) : δ (ppm) = 3.94-3.91 (m, 2H), 2.83-2.53 (m, 13H), 2.26-0.70 (m, 56H). **RMN ¹³C** (63 MHz, CD₃OD) : δ (ppm) = 175.93, 74.12, 59.03, 49.98, 49.30, 48.27, 47.76, 45.42, 43.93, 42.41, 40.37, 37.56, 37.01, 35.81, 34.94, 34.41, 33.55, 31.13, 29.98, 28.84, 28.74, 28.51, 27.61, 27.43, 25.03, 23.91, 22.82, 22.76, 17.86, 13.34 |
| 31 | **RMN ¹H** (250 MHz, CD₃OD) : δ (ppm) = 3.96-3.94 (m, 2H), 3.79 (m, 1H), 2.85-2.68 (m, 13H), 2.54-0.71 (m, 55H). **RMN ¹³C** (63 MHz, CD₃OD) : δ (ppm) = 175.91, 74.02, 69.02, 59.34, 50.08, 48.66, 48.21, 47.64, 45.47, 43.52, 43.12, 42.40, 41.16, 40.48, 37.08, 36.87, 36.65, 36.49, 36.35, 35.95, 34.44, 33.56, 31.65, 29.68, 28.87, 28.64, 28.02, 27.84, 27.78, 27.36, 24.36, 23.41, 22.82, 22.76, 17.91, 13.14 |
| 32 | **RMN ¹H** (250 MHz, CD₃OD) : δ (ppm) = 3.89 (m, 1H), 3.74 (m, 1H), 2.70-2.41 (m, 13H), 2.17-0.64 (m, 56H). **RMN ¹³C** (63 MHz, CD₃OD) : δ (ppm) = 175.82, 69.03, 59.40, 57.55, 51.70, 50.44, 50.33, 48.27, 45.61, 43.83, 43.63, 42.41, 41.22, 40.95, 40.63, 37.06, 36.76, 36.04, 34.37, 34.21, 33.56, 32.53, 29.46, 29.37, 28.10, 27.98, 24.78, 23.71, 22.83, 22.76, 21.94, 19.11, 12.37 |
| 33 | **RMN ¹H** (250 MHz, CD₃OD) : δ (ppm) = 3.79 (m, 1H), 3.65 (s, 3H), 2.75-2.64 (m, 13H), 2.27-0.69 (m, 50H). **RMN ¹³C** (63 MHz, CD₃OD) : δ (ppm) = 176.47, 69.04, 59.40, 57.46, 52.17, 51.69, 50.46, 50.36, 48.27, 45.62, 43.81, 43.63, 41.20, 40.95, 40.63, 37.27, 37.11, 36.90, 36.76, 36.03, 34.19, 32.61, 32.39, 31.97, 29.46, 29.40, 28.11, 28.04, 24.77, 23.72, 21.93, 18.96, 12.37 |
| 34 | **RMN ¹H** (250 MHz, CD₃OD) : δ (ppm) = 3.79 (m, 1H), 3.38 (m, 4H), 2.73-2.62 (m, 13H), 2.40-0.70 (m, 55H). **RMN ¹³C** (63 MHz, CD₃OD) : δ (ppm) = 175.43, 69.05, 59.44, 57.50, 51.67, 50.57, 50.49, 48.89, 48.34, 45.69, 43.83, 43.75, 43.68, 41.62, 41.20, 40.97, 40.70, 37.54, 37.18, 36.79, 36.07, 34.18, 33.23, 33.07, 31.17, 29.89, 29.48, 28.25, 24.79, 23.76, 21.94, 19.23, 14.83, 13.45, 12.40 |
| 35 | **RMN ¹H** (250 MHz, CD₃OD) : δ (ppm) = 3.94 (m, 1H), 3.47 (m, 1H), 2.98-2.52 (m, 15H), 2.33-0.71 (m, 54H). **RMN ¹³C** (63 MHz, CD₃OD) : δ (ppm) = 175.86, 72.04, 58.87, 57.65, 56.76, 55.13, 50.29, 45.73, 45.66, 44.94, 44.66, 44.44, 42.42, 41.69, 40.80, 40.56, 38.81, 36.97, 36.65, 35.74, 34.42, 33.60, 32.03, 29.83, 29.56, 28.02, 24.32, 22.82, 22.76, 22.54, 19.23, 12.80 |
| 36 | **RMN ¹H** (250 MHz, CD₃OD) : δ (ppm) = 3.94 (m, 1H), 2.98-2.69 (m, 13H), 2.20-0.69 (m, 57H). **RMN ¹³C** (63 MHz, CD₃OD) : δ (ppm) = 175.85, 59.05, 57.98, 57.65, 49.71, 49.65, 47.94, 45.00, 44.05, 43.58, 42.43, 41.88, 41.59, 39.91, 37.28, 36.98, 36.29, 36.03, 34.38, 33.53, 29.43, 28.88, 27.39, 27.33, 25.38, 24.51, 24.00, 22.81, 22.74, 22.06, 19.04, 12.63 |
| 37 | **RMN ¹H** (250 MHz, CD₃OD) : δ (ppm) = 4.05 (m, 1H), 3.80 (m, 1H), 3.53 (m, 1H), 2.79-2.63 (m, 8H), 2.52-0.95 (m, 54H), 0.71 (s, 3H). **RMN ¹³C** (63 MHz, CD₃OD) : δ (ppm) = 175.27, 69.02, 59.41, 57.49, 51.69, 50.64, 48.95, 48.27, 47.11, 45.69, 43.84, 43.59, 41.19, 40.95, 40.65, 37.25, 37.10, 37.04, 36.74, 36.00, 34.21, 33.54, 33.38, 32.80, 29.54, 29.37, 27.89, 24.78, 23.70, 21.93, 21.30, 21.12, 19.23, 12.39. |
| 38 | **RMN ¹H** (250 MHz, CD₃OD) : δ (ppm) = 3.79 (m, 1H), 3.61 (m, 1H), 2.87-2.61 (m, 9H), 2.47-0.95 (m, 52H), 0.69 (s, 3H). **RMN ¹³C** (63 MHz, CD₃OD) : δ (ppm) = 175.86, 69.08, 59.44, 57.54, 51.70, 49.75, 48.34, 45.71, 43.84, 43.66, 41.21, 40.95, 40.70, 37.41, 37.14, 37.06, 36.79, 36.05, 34.37, 34.21, 34.02, 33.94, 33.61, 33.11, 29.80, 29.47, 28.15, 26.81, 26.35, 24.78, 23.73, 21.93, 19.11, 12.37 |
| 39 | **RMN ¹H** (250 MHz, CD₃OD) : δ (ppm) = 3.79 (m, 1H), 3.38 (m, 4H), 2.83-2.60 (m, 13H), 2.46-0.95 (m, 50H), 0.70 (s, 3H). **RMN ¹³C** (63 MHz, CD₃OD) : δ (ppm) = 175.42, 69.06, 59.47, 57.48, 51.66, 48.99, 48.93, 45.76, 43.83, 43.74, 41.62, 41.20, 40.97, 40.76, 37.74, 37.19, 36.82, 36.10, 34.18, 33.48, 33.23, 31.14, 30.25, 29.89, 29.49, 28.43, 24.80, 23.79, 21.94, 19.23, 14.83, 13.46, 12.40 |
| 40 | **RMN ¹H** (250 MHz, CD₃OD) : δ (ppm) = 4.06 (m, 1H), 3.79 (m, 1H), 3.53 (m, 1H), 2.88-2.61 (m, 12H), 2.49-0.95 (m, 57H), 0.70 (s, 3H). **RMN ¹³C** (63 MHz, CD₃OD) : δ (ppm) = 175.31, 69.06, 59.45, 57.51, 51.71, 50.68, 48.95, 48.43, 47.13, 45.71, 43.85, 43.67, 41.21, 40.97, 40.72, 37.39, 37.28, 37.13, 36.79, 36.04, 34.21, 33.56, 33.40, 33.16, 31.12, 30.12, 29.78, 29.57, 28.13, 24.79, 23.71, 21.93, 21.28, 21.10, 19.21, 12.36 |
| 42 | **RMN ¹H** (250 MHz, CD₃OD) : δ (ppm) = 3.97-3.91 (m, 2H), 2.78-2.49 (m, 13H), 2.25-0.95 (m, 51H), 0.71 (s, 3H). **RMN ¹³C** (63 MHz, CD₃OD) : δ (ppm) = 175.93, 74.13, 59.15, 53.41, 52.83, 49.35, 48.25, 47.75, 46.14, 43.96, 42.41, 41.01, 37.55, 37.01, 35.85, 34.95, 34.38, 34.14, 33.53, 30.11, 30.05, 29.98, 28.84, 28.55, 27.91, 27.61, 27.24, 25.02, 24.02, 22.82, 22.76, 17.85, 13.35. |
| 43 | **RMN ¹H** (250 MHz, CD₃OD) : δ (ppm) = 3.97-3.91 (m, 2H), 3.79 (m, 1H), 2.83-2.39 (m, 15H), 2.27-0.94 (m, 48H), 0.71 (s, 3H). **RMN ¹³C** (63 MHz, CD₃OD) : δ (ppm) = 175.93, 74.05, 69.04, 59.40, 53.67, 52.87, 52.77, 48.19, 47.64, 46.02, 43.61, 43.15, 42.41, 41.18, 41.01, 37.08, 36.41, 36.00, 34.41, 33.57, 30.20, 29.71, 28.89, 28.05, 27.83, 26.91, 24.36, 23.46, 22.82, 22.76, 17.90, 13.15. |
| 44 | **RMN ¹H** (250 MHz, CD₃OD) : δ (ppm) = 3.80 (m, 1H), 3.41-3.35 (m, 4H), 2.80-2.53 (m, 13H), 2.43-0.96 (m, 50H), 0.70 (s, 3H). **RMN ¹³C** (63 MHz, CD₃OD) : δ (ppm) = 175.41, 68.97, 59.41, 57.50, 53.29, 52.77, 51.69, 45.66, 43.82, 43.75, 43.46, 41.62, 41.18, 40.91, 40.74, 37.17, 36.68, 35.93, 34.19, 33.23, 31.19, 29.46, 29.21, 27.40, 26.48, 24.77, 23.66, 21.93, 19.24, 14.84, 13.47, 12.41. |
| 45 | **RMN ¹H** (250 MHz, CD₃OD) : δ (ppm) = 3.94 (m, 1H), 3.80 (m, 1H), 2.83-2.57 (m, 13H), 2.27-0.93 (m, 45H), 0.69 (s, 3H). **RMN ¹³C** (63 MHz, CD₃OD) : δ (ppm) = 175.89, 69.06, 59.41, 57.64, 57.53, 57.49, 56.98, 54.03, 51.77, 44.82, 43.85, 43.53, 42.43, 41.24, 41.20, 40.95, 39.99, 37.05, 36.71, 35.95, 34.41, 34.27, 33.56, 29.45, 27.67, 24.74, 23.60, 22.80, 22.73, 21.90, 19.07, 12.34. |
| 46 | **RMN ¹H** (250 MHz, CD₃OD) : δ (ppm) = 3.94 (m, 1H), 3.79 (m, 1H), 2.83-2.37 (m, 15H), 2.24-0.95 (m, 49H), 0.69 (s, 3H). **RMN ¹³C** (63 MHz, CD₃OD) : δ (ppm) = 175.77, 69.01, 59.56, 57.47, 53.55, 52.83, 51.68, 46.23, 43.81, 43.68, 42.38, 41.13, 40.94, 37.67, 37.22, 37.03, 36.81, 36.11, 34.33, 34.19, 33.54, 30.61, 29.46, 28.40, 27.38, 24.78, 23.80, 22.86, 22.79, 21.94, 19.13. |
| 47 | **RMN ¹H** (250 MHz, CD₃OD) : δ (ppm) = 3.79 (m, 1H), 3.61 (m, 1H), 2.80-2.47 (m, 13H), 2.21-0.95 (m, 57H), 0.69 (s, 3H). **RMN ¹³C** (63 MHz, CD₃OD) : δ (ppm) = 175.88, 69.09, 59.57, 57.54, 53.56, 52.85, 51.71, 49.76, 46.22, 43.84, 43.71, 41.13, 40.97, 37.19, 37.05, 36.82, 36.07, 34.37, 34.22, 34.02, 33.94, 33.60, 30.60, 29.47, 28.36, 27.34, 26.81, 26.34, 24.77, 23.73, 21.94, 19.10, 12.36 |
| 48 | **RMN ¹H** (250 MHz, CD₃OD) : δ (ppm) = 3.94 (m, 1H), 3.47 (m, 1H), 2.89-2.52 (m, 13H), 2.37-0.96 (m, 50H), 0.71 (s, 3H). **RMN ¹³C** (63 MHz, CD₃OD) : δ (ppm) = 175.88, 72.05, 59.99, 57.67, 56.71, 53.38, 52.84, 46.23, 44.94, 44.66, 44.42, 42.43, 40.96, 40.82, 38.80, 36.98, 35.75, 34.40, 33.60, 29.84, 28.10, 24.25, 22.81, 22.75, 19.21, 12.79 |
| 49 | **RMN ¹H** (250 MHz, CD₃OD) : δ (ppm) = 3.94 (m, 1H), 2.97-2.50 (m, 12H), 2.25-0.86 (m, 53H), 0.69 (s, 3H). **RMN ¹³C** (63 MHz, CD₃OD) : δ (ppm) = 175.87, 59.16, 58.08, 57.63, 53.52, 53.31, 52.85, 46.12, 44.07, 43.92, 43.84, 42.43, 41.99, 41.66, 40.89, 37.36, 37.20, 36.98, 36.33, 36.23, 34.37, 33.90, 33.52, 29.62, 29.43, 27.79, 27.10, 25.41, 24.22, 22.81, 22.75, 22.10, 19.05, 12.66 |
| 50 | **RMN ¹H** (250 MHz, CD₃OD) : δ (ppm) = 4.05 (m, 1H), 3.80 (m, 1H), 3.53 (m, 1H), 2.80-2.61 (m, 13H), 2.45-0.95 (m, 58H), 0.70 (s, 3H). **RMN ¹³C** (63 MHz, CD₃OD) : δ (ppm) = 175.28, 69.07, 59.45, 57.47, 51.67, 50.62, 50.52, 48.89, 48.35, 47.11, 45.71, 43.84, 43.68, 41.19, 40.96, 40.72, 37.56, 37.25, 36.85, 36.79, 36.09, 35.83, 34.19, 33.54, 33.38, 33.21, 29.96, 29.54, 28.30, 24.79, 23.75, 21.93, 21.30, 21.12, 19.23, 12.39 |
| 51 | **RMN ¹H** (250 MHz, CD₃OD) : δ (ppm) = 3.79 (m, 1H), 3.61 (m, 1H), 2.76-2.62 (m, 13H), 2.49-0.95 (m, 57H), 0.69 (s, 3H). **RMN ¹³C** (63 MHz, CD₃OD) : δ (ppm) = 175.83, 69.04, 59.43, 57.56, 51.70, 50.55, 50.44, 49.74, 48.32, 45.69, 43.83, 43.63, 41.21, 40.95, 40.69, 37.30, 37.05, 36.76, 36.05, 34.39, 34.21, 34.00, 33.92, 33.60, 32.93, 29.56, 29.46, 28.26, 28.06, 26.80, 26.34, 24.77, 23.71, 21.92, 19.12, 12.38. |
| 52 | **RMN ¹H** (250 MHz, CD₃OD) : δ (ppm) = 3.94 (m, 1H), 3.80 (m, 1H), 2.82-2.60 (m, 9H), 2.42-0.96 (m, 50H), 0.69 (s, 3H). **RMN ¹³C** (63 MHz, CD₃OD) : δ (ppm) = 175.90, 69.00, 59.37, 57.63, 51.76, 50.06, 48.65, 45.53, 43.84, 43.45, 42.44, 41.84, 41.73, 41.23, 40.91, 40.60, 37.09, 36.67, 36.35, 34.42, 34.24, 33.57, 30.03, 29.46, 28.25, 27.70, 27.21, 24.74, 23.55, 22.80, 22.73, 21.89, 19.07, 12.33 |
| 53 | **RMN ¹H** (250 MHz, CD₃OD) : δ (ppm) = 3.79 (m, 1H), 3.38 (m, 4H), 2.71-2.60 (m, 13H), 2.46-0.95 (m, 48H), 0.70 (s, 3H). **RMN ¹³C** (63 MHz, CD₃OD) : δ (ppm) = 175.50, 69.12, 59.49, 57.50, 51.67, 49.87, 45.69, 43.84, 43.76, 41.64, 41.21, 40.97, 40.73, 37.79, 37.25, 37.20, 36.83, 36.08, 34.19, 33.66, 33.24, 31.16, 30.49, 29.49, 28.48, 24.79, 23.76, 21.93, 19.20, 14.80, 13.42, 12.36. |
| 54 | **RMN ¹H** (250 MHz, CD₃OD) : δ (ppm) = 4.05 (m, 1H), 3.79 (m, 1H), 3.53 (m, 1H), 2.82-2.61 (m, 13H), 2.47-0.95 (m, 54H), 0.70 (s, 3H). **RMN ¹³C** (63 MHz, CD₃OD) : δ (ppm) = 175.15, 69.00, 59.49, 57.41, 51.63, 50.63, 50.60, 50.17, 48.44, 47.07, 45.72, 43.82, 43.73, 41.18, 40.97, 40.73, 37.79, 37.25, 36.82, 36.12, 34.16, 33.62, 33.50, 33.35, 30.52, 29.57, 28.50, 24.82, 23.86, 21.96, 21.34, 21.17, 19.28, 12.46. |
| 55 | **RMN ¹H** (250 MHz, CD₃OD) : δ (ppm) = 3.94 (m, 1H), 3.79 (m, 1H), 2.81-2.48 (m, 9H), 2.40-0.86 (m, 50H), 0.69 (s, 3H). **RMN ¹³C** (63 MHz, CD₃OD) : δ (ppm) = 175.79, 69.06, 59.48, 59.45, 57.50, 51.68, 50.69, 50.66, 49.01, 48.40, 47.42, 45.77, 43.83, 43.76, 42.56, 42.39, 41.22, 40.97, 40.79, 37.87, 37.30, 37.06, 36.84, 36.12, 34.35, 34.19, 33.59, 33.56, 31.74, 30.46, 29.46, 28.57, 28.47, 27.97, 24.79, 23.82, 22.86, 22.78, 21.95, 19.13, 12.40. |
| 56 | **RMN ¹H** (250 MHz, CD₃OD) : δ (ppm) = 4.06 (m, 1H), 3.80 (m, 1H), 3.53 (m, 1H), 2.80-2.47 (m, 13H), 2.39-0.95 (m, 56H), 0.71 (s, 3H). **RMN ¹³C** (63 MHz, CD₃OD) : δ (ppm) = 175.29, 69.07, 59.58, 57.47, 53.58, 52.86, 51.70, 50.68, 47.12, 46.26, 43.84, 43.71, 41.16, 40.98, 40.75, 37.26, 36.08, 34.21, 33.53, 33.38, 30.69, 29.56, 28.43, 27.42, 24.79, 23.76, 21.92, 21.29, 21.11, 19.22, 12.38 |

Parmi lesdits composés de formule (I), les composés **(1), (2), (5), (13), (15), (33)** et **(34)** ou un de leurs sels pharmaceutiquement acceptables, notamment leurs chlorhydrates, sont particulièrement intéressants.

Les exemples suivants illustrent en détail la préparation des composés selon l'invention. Les structures des produits obtenus ont été confirmées au moins par les spectres de RMN.

### EXEMPLES

Toutes les synthèses ont été réalisées avec des solvants purifiés selon les méthodes usuelles. Les réactifs commerciaux sont directement utilisés sans purification préalable.

« Rdt » signifie rendement.

### Exemple 1 : préparation des composés de formule (III) tels que définis ci-dessus

### Exemple 1.1 N-isopropyl-désoxycholamide 1

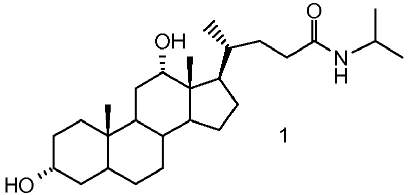

Dans un ballon bicol muni d'un barreau aimanté, on place 2 g (5.1 mmol) d' acide déoxycholique dissous dans 20 mL de THF. On additionne en suivant 1.7 équivalents de HOBT (1.36 g, 8.8 mmol) et 1 equivalent de DCC (1.04 g, 5.1mmol). On additionne également 600 µL d'isopropylamine (13 mmol) et on place sous agitation 24h à température ambiante. Après évaporation du THF et reprise dans 40 mL de CH2Cl2 on filtre le précipité qui est lavé avec de l'acétate d'éthyle. Le filtrat est concentré sous vide et purifié par chromatographie sur gel de silice (éluant : acétate d'éthyle/méthanol (9/1)). Le produit désiré **1** est obtenu sous la forme d'un solide blanc (1.9 g).
**RMN ¹H** (250 MHz, CDCl₃) : δ (ppm) = 4.17 (m, 2H), 2.39-0.63 (m, 45H). **RMN ¹³C** (63 MHz, CDCl₃) : δ (ppm) = 173.39 ; 73.17 ; 71.59 ; 50.36 ; 48.93 ; 48.06 ; 46.76 ; 42.09 ; 41.23 ; 36.26 ; 35.96 ; 35.35 ; 34.14 ; 33.68 ; 33.68 ; 33.51 ; 33.44 ; 31.68 ; 30.65 ; 28.47 ; 27.60 ; 27.17 ; 26.19 ; 25.62 ; 24.95 ; 23.75 ; 23.10 ; 22.68 ; 17.31 ; 12.69. Rdt : 79%. C₂₇H₄₇NO₃

Les composés **2-7** ont été préparés selon le mode opératoire en considérant l'acide biliaire adéquat de départ.

### Exemple 1.2 N-isopropyl-chénodésoxycholamide 2

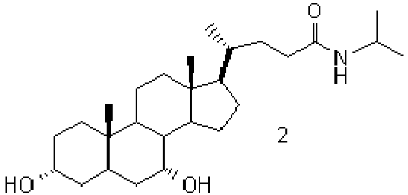

**RMN ¹H** (250 MHz, CDCl₃) : δ (ppm) = 3.96 (m, 2H), 3.69 (m, 1H), 3.31 (m, 1H), 2.30-0.75 (m, 41H), 0.50 (s, 3H). **RMN ¹³C** (63 MHz, CDCl₃) : δ (ppm) = 172.88, 72.02, 68.53, 60.46, 55.80, 50.45, 42.69, 41.49, 41.28, 39.83, 39.64, 39.41, 35.47, 35.33, 35.06, 34.60, 33.75, 32.83, 31.82, 30.64, 28.23, 23.71, 22.83, 20.59, 18.42, 14.22, 11.78. Rdt : 67%. C₂₇H₄₇NO₃

### Exemple 1.3 N-méthyl-chénodésoxycholamide 3

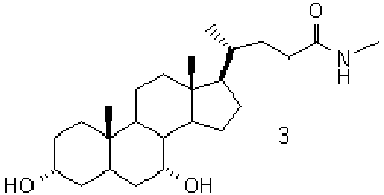

**RMN ¹H** (250 MHz, CDCl₃) : δ (ppm) = 3.78 (m, 1H), 3.60 (s, 3H), 3.39 (m, 1H), 2.35-2.06 (m, 3H), 1.98-0.73 (m, 31H), 0.59 (s, 3H). **RMN ¹³C** (63 MHz, CDCl₃) : δ (ppm) = 189.68, 174.82, 71.99, 66.51, 55.78, 51.54, 50.46, 42.69, 41.49, 39.86, 39.64, 39.41, 35.39, 35.08, 34.62, 32.84, 31.02, 30.67, 28.17, 23.71, 22.80, 20.59, 18.28, 11.78. Rdt : 57%. C₂₅H₄₂NO₃

### Exemple 1.4 N-isopropyl-lithocholamide 4

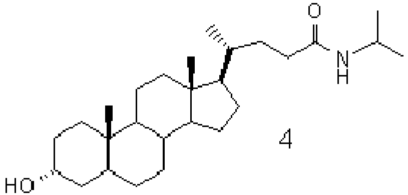

**RMN ¹H** (250 MHz, CDCl₃) : δ (ppm) = 4.11-3.40 (m, 3H), 2.11-0.55 (m, 44H). **RMN ¹³C** (63 MHz, CDCl₃) : δ (ppm) = 171.71, 71.87, 58.53, 56.01, 42.76, 42.09, 41.21, 40.43, 40.20, 36.45, 35.88, 35.50, 35.38, 34.58, 34.00, 33.84, 31.81, 30.55, 28.29, 27.20, 26.43, 24.98, 24.22, 23.40, 22.88, 20.84, 18.43, 12.05. Rdt : 92%. C₂₇H₄₇NO₂

### Exemple 1.5 N-isopropyl-cholamide 5

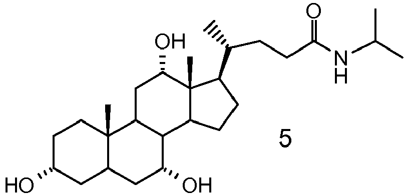

**RMN ¹H** (250 MHz, CDCl₃) : δ (ppm) = 4.19-385 (m, 3H), 3.42 (m, 1H), 2.22-0.87 (m, 40H), 0.66 (s, 3H). **RMN ¹³C** (63 MHz, CDCl₃) : δ (ppm) = 173.88, 73.24, 71.86, 68.50, 60.46, 49.07, 46.27, 41.50, 41.33, 39.33, 35.35, 34.78, 34.86, 33.81, 33.06, 31.78, 30.18, 28.01, 27.57, 26.22, 24.91, 23.26, 22.63, 22.39, 21.09, 17.41, 14.20, 12.40 Rdt : 83%. C₂₇H₄₇NO₄

### Exemple 1.6 N-isopropyl-ursodésoxycholamide 6

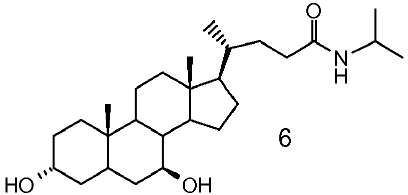

**RMN ¹H** (250 MHz, CDCl₃) : δ (ppm) = 4.01-3.92 (m, 1H), 3.59-3.33 (m, 2H), 2.20-0.79 (m, 41H), 0.52 (s, 3H). **RMN ¹³C** (63 MHz, CDCl₃) : δ (ppm) = 173.72, 71.38, 71.35, 55.69, 54.78, 43.68, 43.51, 42.14, 41.51, 40.09, 39.24, 37.14, 36.91, 35.42, 34.93, 34.01, 33.69, 31.97, 30.15, 28.66, 26.87, 25.51, 24.84, 23.38, 22.67, 21.16, 18.45, 12.07. Rdt : 70%. C₂₇H₄₇NO₃

### Exemple 1.7 N,N-diéthyl-chénodésoxycholamide 7

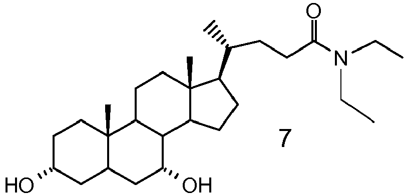

**RMN ¹H** (250 MHz, CDCl₃) : δ (ppm) = 4.03 (m, 1H), 3.76-3.57 (m, 2H), 3.37-3.22 (m, 5H), 2.31-0.83 (m, 38H), 0.59 (s, 3H). **RMN ¹³C** (63 MHz, CDCl₃) : δ (ppm) = 173.38, 72.36, 68.85, 67.57, 60.94, 56.44, 50.94, 43.16, 42.55, 42.07, 40.58, 40.21, 39.93, 36.13, 35.92, 35.56, 35.20, 33.33,32.08, 31.13, 30.47, 28.75, 24.19, 23.35, 21.57, 21.13, 19.07, 14.96, 14.71, 13.61, 12.30. Rdt : 70%. C₂₈H₄₉NO₃

### Exemple 2 : préparation des composés de formule (II) téls que définis ci-dessus

### Exemple 2.1 3-oxo-N-isopropyl-désoxycholamide 8

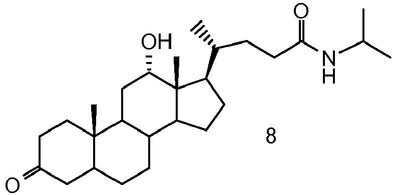

Dans un ballon bicol muni d'un barreau aimanté et surmonté d'un Dean-Stark, on place 2 g (5 mmol) de N-isopropyl-désoxycholamide **1** dissous dans 40 mL de toluène et 30 mL d'acétone. On additionne en suivant 2.2 équivalents de tri-*tert*-butylate d'aluminium (2.7 g, 11 mmol). Le mélange est porté à reflux du toluène pendant 8h. Après refroidissement, le mélange est lavé à trois reprises avec 30 mL d'acide sulfurique 2N puis avec 30 mL d'eau. Après séchage sur sulfate de sodium anhydre et filtration, le filtrat est évaporé sous vide. Le produit brut ainsi obtenu est purifié par chromatographie sur gel de silice (éluant : acétate d'éthyle). Le produit désiré **8** est obtenu sous la forme d'un solide blanc. **RMN ¹H** (250 MHz, CDCl₃) : δ (ppm) = 4.09-4.01 (m, 2H), 2.78-0.68 (m, 43H). **RMN ¹³C** (63 MHz, CDCl₃) : δ (ppm) = 213.51, 172.55, 72.85, 48.06, 47.24, 46.52, 44.23, 42.28, 41.16, 37.08, 36.82, 35.63, 35.12, 34.34, 33.74, 33.69, 31.61, 28.87, 27.44, 26.48, 25.41, 23.52, 22.78, 22.75, 22.32, 17.42, 12.72. Rdt : 49%. C₂₇H₄₅NO₃

Les composés **9-12** ont tous été produits selon le même mode opératoire que celui développé pour la synthèse du composé 8 décrit ci-dessus en considérant le diol de départ adéquat (composés **2,5-7)** préalablement préparé.

### Exemple 2.2 3-oxo-N-isopropyl-chénodésoxycholamide 9

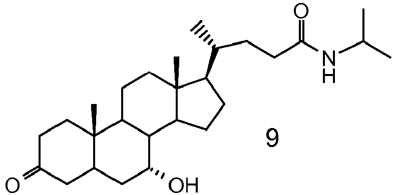

**RMN ¹H** (250 MHz, CDCl₃) : δ (ppm) = 4.07 (m, 1H), 3.91 (m, 1H), 2.44-0.64 (m, 43H). **RMN ¹³C** (63 MHz, CDCl₃) : δ (ppm) = 213.40, 172.57, 68.28, 55.79, 50.25, 45.59, 43.19, 42.65, 41.15, 39.46, 39.29, 36.93, 36.77, 35.41, 35.25, 33.82, 33.69, 33.16, 31.69, 23.60, 22.79, 22.76, 21.88, 20.91, 18.34, 11.73. Rdt : 58%. C₂₇H₄₅NO₃

### Exemple 2.3 3-oxo-N-isopropyl-cholamide 10

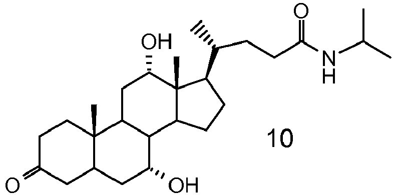

**RMN ¹H** (250 MHz, CDCl₃) : δ (ppm) = 3.99-3.86 (m, 3H), 2.41-0.62 (m, 42H). **RMN ¹³C** (63 MHz, CDCl₃) : δ (ppm) = 213.74, 173.30, 72.97, 68.97, 46.55, 46.39, 45.37, 43.01, 41.55, 41.08, 39.11, 36.68, 36.54, 35.35, 34.82, 33.93, 32.99, 31.41, 28.29, 27.51, 26.70, 23.14, 22.54, 21.38, 17.20, 12.34. Rdt : 58%. C₂₇H₄₅NO₄

### Exemple 2.4 3-oxo-N-isopropyl-ursodésoxycholamide 11

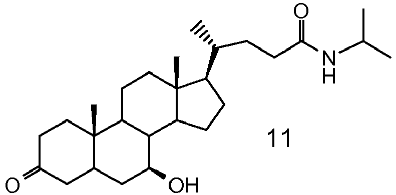

**RMN ¹H** (250 MHz, CDCl₃) : δ (ppm) = 4.07 (m, 1H), 3.58 (m, 1H), 2.56-0.67 (m, 43H). **RMN ¹³C** (63 MHz, CDCl₃) : δ (ppm) = 212.12, 172.65, 70.58, 55.61, 54.87, 44.31, 43.64, 43.20, 43.04, 41.13, 39.90, 39.27, 36.93, 36.28, 36.15, 35.27, 34.30, 33.67, 31.71, 28.53, 26.72, 22.69, 22.66, 22.57, 21.54, 18.40, 12.04. Rdt : 67%. C₂₇H₄₅NO₃

### Exemple 2.5 3-oxo-N,N-diéthyl-chénodésoxycholamide 12

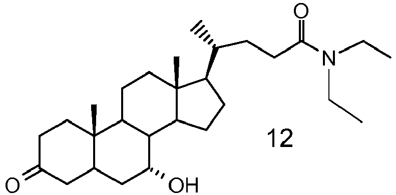

**RMN ¹H** (250 MHz, CDCl₃) : δ (ppm) = 3.89 (m, 1H), 3.29 (m, 4H), 2.43-0.67 (m, 42H). **RMN ¹³C** (63 MHz, CDCl₃) : δ (ppm) = 213.48, 172.63, 68.08, 55.85, 50.21, 45.59, 43.25, 42.57, 41.90, 39.97, 39.43, 39.29, 36.90, 36.77, 35.52, 35.22, 33.83, 33.10, 31.42, 29.82, 28.14, 23.57, 21.86, 20.90, 18.43, 14.34, 13.01, 11.71. Rdt : 65%. C₂₈H₄₇NO₃

Les composés **13** et **14** ont été produits à partir du N-méthyl-chénodésoxycholamide **3** et du N-isopropyl-lithocholamide **4** respectivement selon le même mode opératoire que celui utilisé pour la synthèse du composé **8** décrit ci-dessus, à l'exception des conditions de purification par chromatographie sur gel de silice des produits bruts qui ont été modifiées (éluant : acétate d'éthyle/éther de pétrole (1/1)).

### Exemple 2.6 3-oxo-N-méthyl-chénodésoxycholamide 13

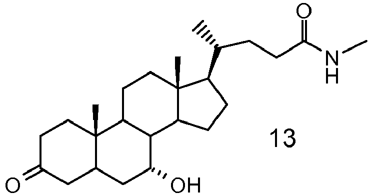

**RMN ¹H** (250 MHz, CDCl₃) : δ (ppm) = 3.79 (m, 1H), 3.54 (s, 3H), 2.34-0.58 (m, 37H). **RMN ¹³C** (63 MHz, CDCl₃) : δ (ppm) = 213.33, 174.42, 67.69, 55.44, 51.18, 49.90, 45.30, 42.99, 42.28, 39.17, 39.02, 36.60, 36.52, 35.00, 34.96, 33.63, 32.83, 30.61, 27.84, 23.26, 21.61, 20.65, 17.95, 11.45. Rdt : 42%. C₂₅H₄₁NO₃

### Exemple 2.7 3-oxo-N-isopropyl-lithocholamide 14

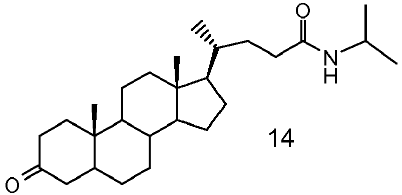

**RMN ¹H** (250 MHz, CDCl₃) : δ (ppm) = 4.06 (m, 1H), 2.67-0.65 (m, 44H). **RMN ¹³C** (63 MHz, CDCl₃) : δ (ppm) = 213.44, 172.55, 56.32, 55.93, 44.23, 42.66, 42.26, 41.06, 40.58, 39.94, 37.12, 36.90, 35.38, 34.77, 33.70, 31.69, 28.11, 26.50, 25.65, 24.05, 22.74, 22.71, 22.55, 21.08, 18.30, 11.96. Rdt : 37%. C₂₇H₄₅NO₂

### Exemple 3 : préparation des composés de formule (I)

Les composés selon l'invention ont tous été préparés par amination réductrice des précurseurs cétoniques préparés précédemment à l'exemple 2 (composés **8-14)** en présence de la chaîne polyamine appropriée.

### Exemple 3.1 préparation du composé (1)

Dans un ballon tricol muni d'une agitation magnétique, 100 mg de 3-oxo-N-isopropyl-désoxycholamide **8** (0.23 mmol) sont dissous dans 7 mL de méthanol. On additionne en suivant 3 équivalents de tétraisopropylate de titane (205 µL, 0.7 mmol) puis 2 équivalents de norspermine (95 µL, 0.46 mmol). Après agitation pendant 12h à température ambiante, on place le ballon dans un bain de glace et on ajoute sous agitation 4 équivalents de borohydrure de sodium (35 mg, 0.92 mmol). Après agitation pendant 2h et retour à température ambiante, on additionne 300 µL d'eau pour terminer la réaction. Après 1h d'agitation supplémentaire, le mélange est filtré sur Célite, rincé à l'ammoniaque puis au méthanol et évaporé sous vide. Le produit brut ainsi obtenu est purifié par chromatographie sur gel de silice (éluant : dichlorométhane/méthanol/ammoniaque (7/3/1)). Le composé **(1)** est obtenu sous la forme d'une huile jaune. Rdt : 40%. C₃₆H₆₉N₅O₂

### Exemple 3.2 préparation des composés (2), (25), (3), (20) et (30)

Les composés (2), **(25), (3), (20)** et **(30)** ont été préparés selon le même mode opératoire développé pour la synthèse du composé **(1)** en utilisant comme précurseur de départ le 3-oxo-N-isopropyl-désoxycholamide **8** et en considérant la chaîne polyamine adéquate.

Le composé **(2)** est obtenu avec un rendement de 36%. (C₃₃H₆₂N₄O₂). Les composés **(25), (3), (20)** et **(30)** sont obtenus avec des rendements respectifs de 70% (C₃₄H₆₄N₄O₂), 58% (C₃₇H₆₉N₅O₂), 43% (C₃₇H₇₃N₇O₂) et 38% (C₃₇H₇₁N₅O₂).

### Exemple 3.3 préparation des composés (31), (4), (5), (26), (6) et (21)

Les composés **(31), (4), (5), (26), (6)** et **(21)** ont été préparés selon le même mode opératoire développé pour la synthèse du composé **(1)** en utilisant comme précurseur de départ le 3-oxo-N-isopropyl-cholamide **10** et en considérant la chaîne polyamine adéquate.

Le composé **(31)** est obtenu avec un rendement de 37% (C₃₇H₇₁N₅O₃). Les composés **(31), (4), (5), (26), (6)** et **(21)** sont obtenus avec des rendements respectifs de 38% (C₃₆H₆₉N₅O₃), 47 % (C₃₃H₆₂N₄O₃), 24% (C₃₄H₆₄N₄O₃), 40% (C₃₇H₆₉N₅O₃) et 32%. C₃₇H₇₃N₇O₃.

### Exemple 3.4 préparation des composés (32), (7), (8), (27), (9) et (22)

Les composés **(32), (7), (8), (27), (9)** et **(22)** ont été préparés selon le même mode opératoire développé pour la synthèse du composé **(1)** en utilisant comme précurseur de départ le 3-oxo-N-isopropyl-chénodésoxycholamide **9** et en considérant la chaîne polyamine adéquate.

Le composé **(32)** est obtenu avec un rendement de 51% (C₃₇H₇₁N₅O₂). Les composés **(7), (8), (27), (9)** et **(22)** sont obtenus avec des rendements respectifs de 20% (C₃₆H₆₉N₅O₂), 55% (C₃₃H₆₂N₄O₂), 65% (C₃₄H₆₄N₄O₂), 15% (C₃₇H₆₉N₅O₂) et 26% (C₃₇H₇₃N₇O₂).

### Exemple 3.5 préparation des composés (33), (10), (11) et (12)

Les composés **(33), (10), (11)** et **(12)** ont été préparés selon le même mode opératoire développé pour la synthèse du composé **(1)** en utilisant comme précurseur de départ le 3-oxo-N-méthyl-chénodésoxycholamide **13** et en considérant la chaîne polyamine adéquate.

Le composé **(33)** est obtenu avec un rendement de 36% (C₃₅H₆₇N₅O₂). Les composés **(10), (11)** et **(12)** sont obtenus avec des rendements respectifs de 58% (C₃₄H₆₅N₅O₂), 51% (C₃₁H₅₈N₄O₂) et 48% (C₃₅H₆₅N₅O₂).

### Exemple 3.6 préparation des composés (34) et (13)

Les composés **(34)** et **(13)** ont été préparés selon le même mode opératoire développé pour la synthèse du composé **(1)** en utilisant comme précurseur de départ le 3-oxo-N,N-diéthyl-chénodésoxycholamide **12** et en considérant la chaîne polyamine adéquate.

Le composé **(34)** est obtenu avec un rendement de 44% (C₃₈H₇₃N₅O₂) et le composé **(13)** avec un rendement de 58% (C₃₄H₆₄N₄O).

### Exemple 3.7 préparation des composés (35), (14), (15), (28), (16) et (23)

Les composés **(35), (14), (15), (28), (16) et (23)** ont été préparés selon le même mode opératoire développé pour la synthèse du composé **(1)** en utilisant comme précurseur de départ le 3-oxo-N-isopropyl-ursodésoxycholamide **11** et en considérant la chaîne polyamine adéquate.

Le composé **(35)** est obtenu avec un rendement de 36% (C₃₇H₇₁N₅O₂). Les composés **(14), (15), (28), (16)** et **(23)** sont obtenus avec des rendements respectifs de 44% (C₃₆H₆₉N₅O₂), 34%, (C₃₃H₆₂N₄O₂), 30% (C₃₄H₆₄N₄O₂), 53% (C₃₇H₆₉N₅O₂) et38% (C₃₇H₇₃N₇O₂).

### Exemple 3.8 préparation des composés (36), (17), (18), (29), (19) et (24)

Les composés **(36), (17), (18), (29), (19)** et **(24)** ont été préparés selon le même mode opératoire développé pour la synthèse du composé **(1)** en utilisant comme précurseur de départ le 3-oxo-N-isopropyl-lithocholamide **14** et en considérant la chaîne polyamine adéquate.

Le composé **(36)** est obtenu avec un rendement de 28% (C₃₇H₇₁N₅O). Les composés **(17), (18), (29), (19) et (24)** sont obtenus avec des rendements respectifs de 22% (C₃₆H₆₉N₅O), 32% (C₃₃H₆₂N₄O), 39% (C₃₄H₆₄N₄O), 18% (C₃₇H₆₉N₅O) et 45% (C₃₇H₇₃N₇O).

### Exemple 4 : Activités antibactériennes intrinsèques des composés de formule (I)

### 1) Préparation de la préculture

Deux tubes ont été préparés :
Un témoin négatif (2 mL de milieu de culture stérile)

Un témoin positif (1940 µL de milieu de culture + 40 µL de DMSO + 20 µL de la suspension bactérienne) à partir d'une souche biologique décongelée (La conservation des souches biologiques est réalisée à - 80°C dans du glycérol).

Les tubes ont été incubés à 37° C pendant 24 heures à 100 tours/minute.

Les germes ont été manipulés sous une hotte dans le laboratoire de type L2 et avant toute manipulation un cycle d'UV a été programmé et seul du matériel stérile a été utilisé. Un test de toxicité des solvants (méthanol, éthanol, DMSO) a été réalisé et ces derniers se sont montrés non toxiques à des concentrations inférieures ou égales à 2%. Les composés chimiques à tester ont été préparées dans un mélange de DMSO/méthanol (50/50) à une concentration de 5 mg/mL.

### 2) Préparation de la microplaque pour la détermination de la concentration minimale d'inhibition (CMI)

Après 24 heures d'incubation, une mesure de la densité optique a été effectuée à l'aide d'un spectrophotomètre à 600 nm en prélevant 100µL de la suspension bactérienne diluée dans 900 µL du milieu de culture stérile. Ce test a nécessité l'utilisation d'une plaque de 96 puits et le volume nécessaire de la suspension microbienne à ensemencer a été calculé pour une DO correspondante à une valeur égale à 0.01 dans chaque puits. Dans cette plaque, la première ligne correspondait au témoin négatif (195 µL de milieu de culture stérile dans chaque puit), la deuxième ligne au témoin positif (milieu de culture ensemencé et additionné de 2 % de DMSO), la troisième ligne a été chargée deux fois en suspension bactérienne, 8 µL de produit à tester ont été placés dans chaque puit. Par la suite, une dilution en cascade au demi a été réalisée à partir de cette ligne.

La première colonne a servi comme témoin d'inhibition. Un filtre stérile a ensuite été placé sur la microplaque permettant le passage des gaz mais pas des contaminants. La microplaque a été incubée à 37° C dans une atmosphère humide durant 24h.

**NB** : Le milieu utilisé est le milieu Mueller-Hinton (MH) pour les bactéries. Tous les tests ont été réalisés en duplicate.

### 3) Cytotoxicité

Le test WST1 a été utilisé pour mesurer l'activité cytotoxique des produits. C'est un test colorimétrique qui permet de mesurer la viabilité et le taux de prolifération cellulaire. Il est basé sur le clivage de sels de tétrazolium incolores WST-1 (4-[3-(4-iodophényl)-2-(4-nitrophényl)-2H-5-tétrazolio]-1,3-benzène disulfonate) par les déshydrogénases mitochondriales en dérivé formazan de couleur jaune, quantifiables par spectrophotométrie à 420-480 nm.

Le test WST1 a été effectué sur des cellules d'ovaires de hamster chinois. Les cellules CHO-K1 (ATCC, USA) sont maintenues en culture dans du milieu Mac Coy's 5A additionné de 10% de sérum de veau fœtal, de 2 mM de L-glutamine et d'un mélange de pénicilline streptomycine (100 U/ml : 10 µg/ml). On incube à 37°C sous atmosphère enrichie en CO₂ (5%) et repiquées tous les deux jours.

Les cellules sont transférées dans des plaques de 96 puits (25000 cellules/mL) dans du milieu de Mc Coy's 5A complet, et maintenues pendant 24 h à 37°C sous atmosphère humide enrichie en CO₂ (5%). Des concentrations croissantes de produits à tester sont ajoutées dans les puits en doubles essais et 8 témoins de croissance contenant les cellules dans le milieu seul sont inclus dans chaque série de tests. Après 24 heures à 37°C (5% de CO₂), le milieu de culture est éliminé, les cellules sont rincées dans du tampon phosphate (PBS) et 50 µL de

PBS contenant 10% de réactif WST1, sont additionnés dans chaque puits. Après 20 minutes d'incubation à 37°C, la lecture des résultats s'effectue par spectrophotométrie à 450 nm.

Les résultats sont exprimés sous forme de relations dose-réponse, modélisés par une analyse de régression non-linéaire à l'aide du logiciel TableCurve. La Concentration Inhibitrice 50% (CI₅₀) représente la concentration en produit capable de réduire de 50% la viabilité cellulaire.

### 4) Lecture des résultats

Après incubation, le filtre a été remplacé par un film transparent, ensuite une lecture de DO a été réalisée dans un spectrophotomètre à plaques IEMS à 620 nm. Un calcul de la concentration minimale d'inhibition (**CMI**) a été réalisé.

Les résultats sont rassemblés dans le tableau III qui suit.

**Tableau III : Activités antibactériennes intrinsèques des composés de formule (I)**

| | **CMI** | | | | | | | | **Cl₅₀** |
|---|---|---|---|---|---|---|---|---|---|
| | **(µg/mL)** | | | | | | | | **(µg/mL)** |
| | Bactéries Gram positif | | | | Bactéries Gram négatif | | | | CHO |
| | *S. aureus* | | *S*. *intermediu s* | *E. faecalis* | *E. coli* | | *P. aeruginosa* | | |
| | Humain ATCC2592 3 | Animal (340) | Animal (1051997) | Humain ATCC292 12 | Humain ATCC2892 2 | Animal (1956) | Humain ATCC2785 3 | Animal (1051575) | |
| (1) | 4 | 2 | 0,5 | 8 | 8 | 8 | 8 | 8 | 42 |
| (2) | 4 | 2 | 1 | 14 | 14 | 14 | 28 | 14 | 35 |
| (25) | 7 | 14 | 2 | 14 | 14 | 14 | 28 | 28 | 48 |
| (3) | 15 | 15 | 4 | 124 | 124 | >124 | 124 | 62 | 120 |
| (20) | 8 | 8 | 4 | 8 | 8 | 16 | 32 | 32 | 18 |
| (30) | 8 | 8 | 1 | 15 | 15 | 30 | 4 | 4 | 30 |
| (31) | 4 | 8 | 4 | 8 | 16 | 32 | 64 | 32 | 40 |
| (4) | 8 | 16 | 4 | 32 | 16 | 32 | 64 | 32 | 65 |
| (5) | 8 | 16 | 4 | 16 | 16 | 32 | 64 | 64 | 70 |
| (26) | 28 | 28 | 14 | 56 | 28 | 56 | 112 | 56 | 80 |
| (6) | 16 | 32 | 16 | 32 | 32 | 64 | 218 | 64 | 100 |
| (21) | 17 | 17 | 8 | 30 | 130 | 30 | 130 | 30 | 60 |
| (32) | 7 | 2 | 0,5 | 7 | 15 | 31 | 30 | 15 | 34 |
| (7) | 4 | 2 | 1 | 4 | 15 | 8 | 30 | 15 | 55 |
| (8) | 6 | 3 | 1 | 8 | 14 | 14 | 54 | 27 | 50 |
| (27) | 8 | 8 | 4 | 16 | 16 | 32 | 32 | 128 | 100 |
| (9) | 16 | 8 | 8 | 62 | 15 | 15 | 124 | 62 | 90 |
| (22) | 8 | 8 | 2 | 32 | 32 | 32 | 130 | 65 | 120 |
| (33) | 3,5 | 1 | 0,8 | 14 | 14 | 7 | 7 | 2 | 35 |
| (10) | 7 | 1 | 1 | 14 | 14 | 7 | 28 | 5 | 35 |
| (11) | 6 | 3 | 1 | 13 | 13 | 13 | 12 | 6 | 20 |
| (12) | 8 | 4 | 2 | 15 | 30 | 15 | 60 | 30 | 50 |
| (34) | 4 | 4 | 2 | 2 | 8 | 8 | 16 | 16 | 50 |
| (13) | 2 | 4 | 2 | 2 | 8 | 8 | 16 | 16 | 22 |
| (35) | 16 | 16 | 8 | 64 | 64 | 64 | 32 | 64 | 55 |
| (14) | 16 | 32 | 4 | 128 | 128 | 128 | 64 | 128 | 90 |
| (15) | 28 | 56 | 7 | 112 | 56 | 112 | 56 | 112 | 140 |
| (28) | 28 | 28 | 14 | 56 | 56 | 112 | 112 | 112 | 28 |
| (16) | 60 | 60 | 15 | 120 | 60 | 120 | >120 | >120 | - |
| (23) | 32 | 32 | 16 | 64 | 128 | 128 | 128 | 128 | 115 |
| (36) | 3,75 | 3,75 | 1,87 | 7,5 | 7,5 | 15 | 15 | 30 | 36 |
| (17) | 8 | 8 | 4 | 8 | 16 | 32 | 64 | 32 | 17 |
| (18) | 6 | 3 | 0,8 | 12 | 26 | 3 | 104 | 26 | 20 |
| (29) | 14 | 14 | 7 | 28 | 28 | 28 | 112 | 112 | 6 |
| (19) | 8 | 4 | 4 | 15 | 30 | 30 | 120 | 30 | 12 |
| (24) | 8 | 8 | 4 | 16 | 32 | 64 | 128 | 128 | 35 |
| (42) | 4 | 4 | 1 | 30 | > 120 | 30 | 30 | 60 | 40 |
| (43) | 16 | 16 | 8 | 60 | 125 | 60 | 125 | 60 | 38 |
| (44) | 2 | 4 | 2 | n.d. | 15 | 30 | 60 | 60 | 75 |
| (53) | 4 | 4 | 2 | 2 | 60 | 15 | 30 | 15 | 30 |
| (45) | 7 | 4* | 2 | 14 | 110 | 14* | 110 | 28* | 60 |
| (46) | 8 | 2 | 2 | 30 | > 120 | 30 | 60 | 120 | 50 |
| (37) | 4 | 2 | 1 | 4 | 15 | 4 | 30 | 15 | 35 |
| (50) | 4 | 2 | 2 | 4 | > 130 | 17 | 17 | 17 | 25 |
| (54) | 4 | 4 | 2 | 2 | 125 | 8 | 32 | 16 | 22 |
| (38) | 4 | 4 | 2 | 4 | 30 | 15 | 30 | 30 | 12 |
| (51) | 4 | 2 | 1 | 2 | 105 | 16 | 16 | 16 | 13 |
| (55) | 2 | 4 | 2 | n.d. | 112 | 55 | 14 | 112 | 80 |
| (52) | 7 | 4 | 2 | 14 | 112 | 28 | 56 | 112 | 80 |
| (39) | 2 | 1 | 1 | n.d. | 120 | 4 | 15 | 8 | n.d. |
| (40) | 4 | 2 | 2 | 2 | 32 | 8 | 8 | 8 | n.d. |
| (56) | 4 | 4 | 4 | 2 | 65 | 16 | 130 | 65 | n.d. |
| (47) | 4 | 8 | 8 | 8 | 130 | 32 | 130 | 32 | n.d. |
| (48) | 30 | 15 | 8 | 60 | > 120 | 120 | 120 | 120 | 42 |
| (49) | 4 | 4 | 2 | 15 | 120 | 30 | 120 | 60 | 6 |

### Exemple 5 : Activités antibactériennes des composés de formule (I) en association avec de la doxycycline

### Préparation de la microplaque pour la détermination de la concentration minimale d'inhibition (CMI) de l'association d'un composé de formule (I) et de la doxycycline

Cette méthode nécessite l'utilisation d'une plaque de 96 puits, 100 µL d'un milieu de culture liquide est déposé dans chaque puits puis ensemencé avec la suspension microbienne précédemment préparée. Le volume nécessaire à ensemencer est calculé pour une DO de 0.01 qui correspond à environ 5.10⁶ bactéries dans chaque puit. Dans cette plaque, la première ligne correspond à un témoin négatif (200 µL de milieu de culture stérile dans chaque puits), la deuxième ligne à un témoin positif (100 µL de milieu de culture stérile + 100 µL de la suspension bactérienne), la troisième ligne contient 192 µL de milieu de culture, 8 µL de composé de formule (I) à tester sont placés dans chaque puits. Par la suite, une dilution en cascade est réalisée à partir de cette ligne. On additionne ensuite dans chaque puits des lignes 3 à 8 µL d'une solution de doxycycline (1 mg dissout dans 20 mL) pour obtenir une concentration finale en antibiotique de 2µg/mL. On additionne ensuite 92 µL de suspension bactérienne dans les lignes 3 à 8. La lecture des résultats (la détermination de la CMI (2 µg/mL de doxyccyline) en présence de X µg/mL de composé de formule (I)) se fait après 24 heures d'incubation à 37° dans une atmosphère humide. Après 24 heures d'incubation à 37°C, 40 µL d'iodure de nitro tétrazolium sont rajoutés dans chaque puits permettant de révéler la présence de bactéries vivantes en colorant le milieu en rose.

Sur la souche Gram-négative de *P. aeruginosa* (PAO1), la doxycycline possède une CMI de 40 µg/mL.

Les résultats sont consignés dans le tableau IV. Ils indiquent la concentration en composés de formule (I) nécessaire pour permettre de restaurer l'activité de la doxycycline (2 µg/mL).

**Tableau IV : Potentialisation de l'activité de la doxycycline à 2µg/mL en présence des composés de formule (I)**

| **Composé No** | **CMI** - *P. aeruginosa* (1051575) µg/mL |
|---|---|
| | Teneur en composé de formule (I) pour restaurer l'activité de la doxycycycline (à 2 µg/mL) |
| | (µg/mL) |
| (1) | 1 |
| (2) | 0,9 |
| (25) | 2 |
| (3) | 2 |
| (20) | 4 |
| (30) | 1 |
| (31) | 1 |
| (4) | 1 |
| (5) | 1 |
| (26) | 4 |
| (6) | 8 |
| (32) | 1 |
| (7) | 1 |
| (8) | 2 |
| (27) | 2 |
| (9) | 2 |
| (22) | 4 |
| (33) | 1 |
| (10) | 1 |
| (11) | 2 |
| (12) | 8 |
| (34) | 2 |
| (13) | 2 |
| (35) | 2 |
| (14) | 1 |
| (15) | 1 |
| (28) | 7 |
| (36) | 0,5 |
| (17) | 2 |
| (18) | 0,8 |
| (29) | 7 |
| (19) | 8 |
| (24) | 8 |
| (42) | 1 |
| (43) | 1 |
| (44) | 2 |
| (53) | 2 |
| (45) | 7 |
| (46) | 2 |
| (37) | 2 |
| (50) | 2 |
| (54) | 2 |
| (38) | 2 |
| (51) | 2 |
| (55) | 2 |
| (52) | 3,5 |
| (39) | 2 |
| (40) | 2 |
| (56) | 2 |
| (47) | 2 |
| (48) | 4 |
| (49) | 4 |

L'utilisation de faibles quantités de composés de formule B(I) permet de restaurer (de réduire) la concentration nécessaire en antibiotique pour tuer la souche considérée. On constate ainsi une très bonne synergie de certains composés avec la doxycycline restaurant ainsi l'activité de cet antibiotique à de faibles concentrations d'utilisation (2 µg/mL).

En particulier, les composés **(2), (9), (25)** et **(36)** permettent d'atteindre une synergie remarquable lorsqu'ils sont administrés avec la doxycycline à 2 µg/mL.

### Exemple 6 : Activité antibactérienne des composés de formule (I) en association avec l'ampicilline, l'érythromycine et le chloramphénicol (souche bactérienne : P. aeruginosa 1051575)

Le mode opératoire est totalement similaire à celui indiqué dans l'exemple 5 ci-dessus. 8 µL d'une solution d'antibiotique considéré (1 mg dissous dans 20 mL) sont additionnés pour obtenir une concentration finale en antibiotique de 2µg/mL à la place de 8 µL d'une solution de doxycycline.

Les résultats sont consignés dans le tableau V. Ils indiquent la concentration en composé de formule **(I)** nécessaire pour permettre de restaurer l'activité de l'antibiotique considéré.

**Tableau V : Potentialisation de l'activité de l'antibiotique considéré à 2µg/mL en présence des composés de formule (I)**

| | **CMI** *P. aeruginosa* (1051575) (µg/mL) | | | |
|---|---|---|---|---|
| | composé + ampicilline² | composé + érythromycin e³ | composé + chloramphénicol⁴ | |
| | 4 µg/mL | 2 µg/mL | 4 µg/mL | 2 \|jp/mL |
| (2) | 28 | 3* | 0.9 | 0.9 |
| (25) | 56 | 28 | 2 | n. d. |
| (42) | n. d. | 8 | n. d. | 2 |
| (5) | 64 | 32* | 2 | 4 |
| (21) | n. d. | 30 | n. d. | 8 |
| (43) | n. d. | 30 | n. d. | 4 |
| (7) | 15 | 15 | 2 | n. d. |
| (8) | 27 | 27 | 2 | n. d. |
| (27) | 64 | 28 | 1 | 4 |
| (44) | n. d. | 8 | n. d. | 4 |
| (45) | n. d. | 28 | n. d. | 7 |
| (37) | n. d. | 7 | n. d. | 2 |
| (50) | n. d. | 4 | n. d. | 2 |
| (51) | n. d. | 8 | n. d. | 2 |
| (55) | n. d. | 7 | n. d. | 2 |
| (35) | 64 | 32 | 2 | n. d. |
| (15) | 110 | 55 | 2 | 7 |
| (49) | n. d. | 30 | n. d. | 4 |

| | | | | |
|---|---|---|---|---|
| ²CMI ampicilline : 200 µg/mL ³CMI érythromycine : 200 µg/mL ⁴CMI chloramphénicol : 200 µg/mL n. d. : non déterminé | | | | |

On constate ainsi une très bonne synergie de certains composés avec divers antibiotiques classiques, à de faibles concentrations d'utilisation.

### Exemple 7 : Activité antifongique des composés de formule (I)

### 1) Préparation de la préculture

Deux tubes ont été préparés :
Un témoin négatif (2 mL de milieu de culture stérile Mueller Hinton MH)

Un témoin positif (1980 µL de milieu de culture + 20 µL de la suspension de levures ou champignons) à partir d'une souche biologique décongelée (La conservation des souches biologiques est réalisée à - 80°C dans du glycérol).

Les tubes ont été incubés à 37° C pendant 24 heures à 100 tours/minute.

Les composés chimiques à tester ont été préparés à une concentration de 5 mg/mL (sous forme de chlorhydrate dans l'eau).

### 2) Préparation de la microplaque pour la détermination de la concentration minimale d'inhibition (CMI)

La CMI est obtenue en utilisant la méthode de micro dilution en cascade dans des plaques stériles de 96 puits. On place tout d'abord 100 µL de milieu de culture MH dans chaque puit, on rajoute ensuite dans les premiers puits 8 µL de la solution (5mg/mL) de la molécule à tester. On complète en milieu pour obtenir un volume total de 200 µL dans ces puits. On réalise alors une dilution en série en prélevant 100 µL dans le premier puit puis de façon successive de puit en puit. On rajoute en suivant 100 µL d'inoculat contenant 2-6 10⁵ CFU de levures ou champignons dans chaque puit. Certains puits sont réservés pour des contrôles positifs et négatifs. Après 24 heures d'incubation on mesure la CMI comme étant la plus basse concentration capable d'inhiber la pousse fongique.

### 3) Lecture des résultats

Après incubation, une lecture de Densité Optique (DO) a été réalisée dans un spectrophotomètre à plaques IEMS à 620 nm. Un calcul de la concentration minimale d'inhibition (**CMI**) a été réalisé.

| | **Cl₅₀** (µg/mL) | **Cl₅₀** (µg/mL) |
|---|---|---|
| | *C. albicans* | CHO |
| (1) | 0.5 | 33 |
| (4) | 8 | 170 |
| (5) | 4 | 70 |
| (32) | 2 | 13 |
| (8) | 1 | 50 |
| (27) | 2 | 100 |
| (11) | 3 | 20 |
| (34) | 0,5 | 50 |
| (44) | 0,5 | 75 |
| (46) | 1 | 50 |
| (52) | 4 | 48 |
| (40) | 1 | n. d. |
| (56) | 4 | n. d. |
| (47) | 2 | n. d. |
| (35) | 1 | 55 |
| (15) | 14 | 85 |
| (19) | 4 | 12 |

| | | |
|---|---|---|
| n. d. : non déterminé | | |

Ces résultats démontrent une activité antifongique intéressante sur 2 souches pour un certain nombre de composés de formule (I).

Ainsi, les composés de l'invention, de formule (I), (I'), (Ia), (Ib) ou (Ic), et plus particulièrement les composés (1) à (56) ou l'un de leurs sels pharmaceutiquement acceptables démontrent une activité antibactérienne et antifongique. Ces composés sont utiles pour le traitement d'infections bactériennes, notamment des infections bactériennes à Gram positif telles que des infections à *Staphylococcus aureus, Staphylococcus intermedius ou Staphylococcus faecalis* et/ou à Gram négatif telles que *Escherichia Coli et Pseudonomas aeruginosa.*

Les composés de formule (I), (I'), (Ia), (Ib), (Ic) ou (Id), et plus particulièrement les composés (1) à (56) ou l'un de leurs sels pharmaceutiquement acceptables selon l'invention sont notamment utiles pour le traitement antibiotique d'infections bactériennes, notamment de souches de bactéries Gram-positives ou Gram-négatives, chez l'homme ou l'animal. A titre d'exemple, les composés selon l'invention sont utiles pour le traitement de mammites, de métrites, d'infection dentaire, d'infection urinaire, d'affection digestive, de pyodermites ou encore d'otites chez l'homme ou l'animal. Les composés selon l'invention, sont également utiles en tant que revêtement empêchant la prolifération bactérienne, pour la fabrication de produits destinés à détruire les biofilms ou à empêcher leur formation. Ces composés peuvent être mis en œuvre dans un dispositif médical, par exemple : cathéters, prothèses, implants, appareils de dialyse, instruments chirurgicaux, sutures ou pansements.

La mammite ou mastite est l'inflammation de la mamelle chez les mammifères, c'est une infection courante en élevage des femelles laitières (vaches, brebis, chèvres, bufflonnes et chamelles). Elle est caractérisée par la présence dans le lait de cellules inflammatoires (leucocytes) et éventuellement de bactéries. Cette inflammation peut avoir des conséquences cliniques avec modification de l'aspect du lait, inflammation visible de la mamelle (tuméfaction, douleur, œdème) et éventuellement atteinte de l'état général. Le plus souvent la maladie demeure subclinique avec altération de la composition du lait et diminution de la production. La mammite résulte d'une infection de la mamelle par des bactéries plus ou moins adaptées à ce biotope. En élevage laitier spécialisé, les mammites provoquent des pertes économiques importantes (lait non produit, impropre à l'usage, altération de la qualité du lait) et constituent un risque de santé publique (bactéries pathogènes et résidus antibiotiques). Les mammites sont dues à la pénétration puis au développement d'une bactérie dans la glande mammaire. L'entrée du germe se fait généralement par l'extrémité du trayon. Une mammite ne concerne donc en général pas tous les quartiers du pis de l'animal. Les principales bactéries responsables de mammite peuvent être regroupées en deux ensembles, en fonction de leur réservoir de contamination. Les germes se trouvant à la surface de la mamelle : *Staphylocoques, Streptococcus agalactiae, Streptococcus disgalactiae, Streptococcus uberis.* Ces bactéries sont principalement responsables de mammites sub-cliniques (non détectables à l'œil nu) qu'il est parfois difficile de guérir en cours de lactation, la période de tarissement est alors mise à profit pour traiter les quartiers infectés aux antibiotiques. Les germes se trouvant dans l'environnement (litière) : par exemple, *Streptococcus uberis, Escherichia coli.* Ces bactéries entrainent généralement des mammites cliniques, qui peuvent aller jusqu'à la mort rapide de l'animal en l'absence de traitement adapté. Les mammites à mycoplasmes posent encore des problèmes dans les cheptels caprins, même si elles ont actuellement quasiment disparu des troupeaux bovins.

La métrite est une inflammation de l'ensemble de la paroi utérine. Elle peut affecter différentes espèces de mammifères domestiques (ruminants, chevaux, porcins, chien, chat) et les animaux sauvages et les humains. Elle est causée par une infection bactérienne et elle est presque toujours observée après une mise bas anormale ou une infection utérine importante. Sa gravité s'échelonne d'une infection subclinique à une maladie déclarée avec fièvre et diminution de la production de lait. Dans le cas des vaches, la métrite peut prédisposer à la cétose, au déplacement de la caillette et à d'autres troubles du post-partum. Elle peut également aboutir à une baisse de la fertilité, temporaire ou permanente, et même, dans certains cas, à la mort de l'animal. La métrite est souvent liée à une contamination de l'utérus par la bactérie *Arcanobacterium pyogenes,* soit seule soit conjointement à d'autres micro-organismes pathogènes tels que : *Fusobacterium necrophorum, Bacteroides spp.* ou *Escherichia coli.* Juste après le vêlage, l'utérus constitue un environnement idéal pour la croissance bactérienne. Durant la première semaine post-partum, jusqu'à 90% des vaches sont victimes d'une infection utérine d'origine bactérienne.

La pyodermite est une maladie cutanée purulente, qui peut être aiguë ou chronique, locale ou diffuse. La pyodermite est étymologiquement une infection de la peau. Elle est d'origine externe, causée par une bactérie, généralement le *staphylocoque* ou le *Streptococcus pyogenes.* Une pyodermite peut être circonscrite ou généralisée. Cette maladie est fréquente chez le chien, mais peut affecter toutes les espèces ayant des pathogènes proches, dont l'espèce humaine. Chez le chien, on observe souvent une dermatite pyotraumatique. Il s'agit d'une lésion cutanée résultant d'une compulsion à griffer, mordiller et lécher une partie du corps. Dès que la lésion est assez importante, une infection secondaire par des bactéries opportunistes peut survenir, amenant l'animal à mordiller ou à se griffer davantage. La plupart des animaux souvent affectés ont des allergies : particulièrement les animaux allergiques aux puces. Cependant, n'importe quelle irritation cutanée peut provoquer une dermatite pyotraumatique.

La maladie parodontale ou infection dentaire est une maladie pouvant affecter toutes les espèces, dont l'homme. C'est la principale cause de maladie dentaire chez le chien et elle est fréquente chez le chat. Pourtant caractérisée par une mauvaise haleine, elle est souvent non identifiée par le propriétaire. Sa prévention passe par des soins réguliers car elle peut entraîner la perte des dents voire des infections graves. La présence de bactéries dans la bouche est normale mais lorsqu'elles se développent trop rapidement, elles peuvent entraîner la formation de plaque dentaire. Si la plaque s'accumule et n'est pas éliminée, une gingivite (inflammation des gencives) peut apparaître. À ce stade, le traitement peut être complètement curatif. Cependant, en l'absence de traitement, la maladie évolue en périodontite caractérisée par une inflammation plus importante des gencives, des dépôts de tartre sur les dents et la disparition de l'os et des structures de soutien entourant la dent. L'atteinte peut être prise en charge mais est irréversible. La parodontite peut entraîner la perte des dents et la propagation d'infections graves au niveau du foie, du cœur ou des poumons.

La cystite, ou infection urinaire, est une maladie pouvant affecter toutes les espèces dont l'homme. Cette pathologie est particulièrement fréquente chez le chat mais se rencontre également fréquemment chez le chien. Elle peut être consécutive à des traumatismes locaux tels que des calculs urinaires ou à des infections d'origine exogène.

Les affections digestives, et plus particulièrement celles conduisant à des diarrhées sont des affections très fréquentes chez l'homme et l'animal, en particulier chez le chien et le chat. Ces affections sont souvent dues à des contaminations et une prolifération bactérienne de germes aérobies ou anaérobies augmentée, ou de contamination par des protozoaires.

L'otite est une inflammation du conduit auditif. L'otite peut toucher toutes les espèces animales, ainsi que l'homme. Il s'agit d'une pathologie extrêmement fréquente chez les carnivores domestiques, en particulier le chien. Elle peut avoir de nombreuses origines dont certaines seront responsables d'otites récidivantes. Plusieurs types de bactéries (*Staphylocoques, Pseudomonas...*) et de levures (*Malassezia*) peuvent se développer dans le conduit auditif, provoquant l'apparition d'une otite. Ces otites sont alors associées à des sécrétions purulentes et à une odeur très désagréable.

Selon un aspect particulier de l'invention, les composés de formule (I) sont pour leur utilisation à titre de médicaments où ils sont administrés en combinaison avec un autre composé antibiotique, notamment de la famille des bêtalactamines (pénicillines / céphalosporines), aminosides, macrolides, polypeptides, sulfamides, quinolones, nitro-imidazolés, dérivés des nitrofuranes, dérivés du noyau benzyl-pyrimidine, des tétracyclines ou des phénicolés, tels que la doxycycline ou le chloramphénicol, la pénicilline, l'ampicilline, amoxicilline, la cloxacilline, la dicloxacilline, l'oxacilline, la nafcilline, la céfalexine, la céfapirine, la céfazoline, le ceftiofur, la céfopérazone, la céfovécine, le cefquinome, la thimaphénicol, le florfénicol, la terramycine, l'érythromycine, la spiramycine, la tylosine, la josamycine, le tilmicosine, la tulathromycine, la gamithromycine, la tildipirosine, la clyndamycine, la lyncomycine, la pirlymicine, le tiamuline, la valnémuline, l'acide oxolinique, la fluméquine, l'enrofloxacine, la danofloxacine, l'ibafloxacine, la marbofloxacine, la difloxacine, l'obifloxacine, la pradofloxacine, la rifampicine, la rifaximine, le sulfaméthizol, la sulfathiazol, la sulfadimidine, la sulfaméthoxazole, la sulfadiazine, la sulfadiméthoxine, la sulfaméthoxypyridazine, le triméthoprime, la baquiloprime, le métronidazole, le dimétridazole, la ronidazole, la nitrofurantoïne, la furazolidone ou la furaltadone. Dans une utilisation particulièrement avantageuse des composés de l'invention, on observe une synergie lors de l'utilisation conjointe des composés de l'invention avec des antibiotiques.

En effet, il a été observé que lorsque les composés de formule (I) étaient associés à un autre composé antibiotique, par exemple la doxycycline, l'ampicilline, l'érythromycine ou le chloramphénicol sur une souche Gram-négative de *Pseudonomas aeruginosa,* une synergie était observée, ainsi qu'illustré à l'exemple 5 ci-dessus. Cette propriété permet par exemple de traiter efficacement des patients avec un taux moindre d'antibiotique, ce qui peut diminuer l'apparition de résistance aux antibiotiques.

La présente invention concerne ainsi également des compositions pharmaceutiques ou vétérinaires comprenant au moins un composé choisi parmi les composés de formules (I), (la), (Ib), (Ic), (Id) et (Ie) telles que définies ci-dessus et les composés (1) à (56) tel que défini ci-dessus ou un sel pharmaceutiquement acceptable de celui-ci et au moins un antibiotique différent d'un composé précité, plus particulièrement tel que défini précédemment, et encore plus particulièrement la doxycycline.

Selon un aspect de l'invention, les compositions pharmaceutiques ou vétérinaires comprennent en outre un deuxième composé antibiotique, notamment de la famille des bêtalactamines (pénicillines / céphalosporines), aminosides, macrolides, polypeptides, sulfamides, quinolones, nitro-imidazolés, dérivés des nitrofuranes, dérivés du noyau benzyl-pyrimidine, des tétracyclines ou des phénicolés.

La présente invention concerne en outre des composés de formule (I), (I'), (Ia), (Ib), (Ic), (Id) ou (Ie) ou encore un composé de formule (1) à (56) ou l'un de ses sels pharmaceutiquement acceptables, pour leur utilisation dans une méthode de potentialiser l'activité antibiotique de composés antibiotiques pouvant être choisis parmi les composés antibiotiques précédemment cités.

Les composés conformes à l'invention sont également des composés de choix en tant que substituts aux antibiotiques. Les composés selon l'invention permettent une excellente activité contre les bactéries, tout en évitant l'apparition de résistances, qui est un atout majeur tant le problème de l'apparition de résistances aux antibiotiques classiques est devenu un problème de santé publique. De par leur mécanisme d'action, différent de celui des antibiotiques, les composés de l'invention se présentent donc comme d'excellents substituts aux antibiotiques.

Selon un autre aspect, les composés de formule (I), (I'), (Ia), (Ib), (Ic) ou (Id), et plus particulièrement les composés (1) à (56) ou l'un de leurs sels pharmaceutiquement acceptables selon l'invention sont notamment utiles pour lutter contre les maladies fongiques.

Les maladies fongiques sont généralement désignées sous le nom de mycose. Il existe de nombreux types de mycoses, qui diffèrent par l'espèce fongique en cause, la localisation de l'infection, son caractère aigu ou chronique, le mode d'infection, etc. Ces maladies sont par exemple, la teigne, la candidose ou l'onixys, la blastomycose, l'asperigllose, la coccidioïdomycose, la cryptococcose, la sporotrichose. Elles peuvent toucher les hommes ou les animaux. Les traitements sont soit topiques locaux, soit par voie orale, selon la gravité et le type d'atteinte. Parmi les champignons fréquemment impliqués dans les mycoses, en particulier humaines, on retrouve principalement :
- les « levures » (champignons ronds microscopiques), dont les Candida, les Cryptococcus, les Pityrosporum et les Pneumocystis, responsables respectivement des candidoses, des cryptococcoses, des pityrosporoses et de la pneumocystose,
- les champignons dits « filamenteux », dont les dermatophytes (en cause dans les dermatophytoses), les Aspergillus (provoquant les aspergilloses respiratoires), etc, et
- les champignons dimorphiques (histoplasmose). Les infections causées par les Candida représentent la principale cause des infections fongiques allant des infections légères de la peau ou des muqueuses aux infections graves affectant un organe. Les champignons de type Aspergillus, sont responsables de la plupart des infections après le Candida.

Selon encore un autre aspect de l'invention, les compositions pharmaceutiques ou vétérinaires comprennent en outre un deuxième composé antiparasitaire, notamment antipaludéen.

Selon un autre aspect, l'invention fournit des composés de formule (I), (I'), (Ia), (Ib), (Ic), (Id) ou (Ie) ou encore un composé de formule (1) à (56) ou l'un de ses sels pharmaceutiquement acceptables, pour leur utilisation dans le traitement d'infections parasitaires ou virales, de l'homme ou de l'animal, telles que le paludisme, le virus de l'immunodéficience féline (FIV), la péritonite infectieuse féline (PIF), la toxoplasmose, la leishmaniose, l'echinococcose, l'ehrlichiose, l'hépatite de Rubarth, la leptospirose, la maladie de Carré, la parvovirose du chien, la piroplasmose, la toux de chenil ou coqueluche, la dirofilariose, la leucose féline (FeLV), le coryza, le typhus ou encore la panleucopénie féline. Les composés selon l'invention peuvent également être utilisés en tant qu'agent antiviral.

Selon un aspect particulier, les composés de formule (I) sont administrés en combinaison avec un autre composé antipaludéen. Avantageusement, les composés de formule (I) permettent de potentialiser l'activité des composés antiparasitaires, notamment antipaludéen.

La présente invention concerne en outre les composés de l'invention pour leur utilisation dans une méthode de traitement d'un homme ou d'un animal souffrant d'infections bactériennes, fongiques, virales ou parasitaires, qui comprend au moins une étape d'administration d'une quantité efficace d'un composé selon l'une quelconque des formules (I), (I'), (Ia), (Ib), (Ic), (Id) ou (Ie) telles que définies ci-dessus et (1) à (56) ou l'un de ses sels pharmaceutiquement acceptables.

La présente invention concerne également des compositions pharmaceutiques ou vétérinaires comprenant au moins un composé choisi parmi les composés de formules (I), (la), (Ib), (Ic), (Id) et (Ie) telles que définies ci-dessus et les composés (1) à (56) tel que défini ci-dessus ou un sel pharmaceutiquement acceptable de celui-ci et un excipient pharmaceutiquement acceptable.

Les compositions pharmaceutiques ou vétérinaires selon l'invention peuvent être présentées sous des formes solides ou liquides, destinées par exemple à l'administration par voie parentérale (intraveineuse, intramusculaire, sous cutanée), orale, générale, locale, transmuqueuse, percutanée, cutanée, oculaire, pulmonaire ou topique.

Elles sont donc présentées sous forme de solutés ou de suspensions injectables ou flacons mono-doses ou multi-doses, sous forme de comprimés nus ou enrobés, de dragées, de capsules, de gélules, de pilules, de cachets, de poudres, de suppositoires ou capsules rectales, de granulés ou de solutions.

De façon avantageuse, le produit selon l'invention comprend également un ou plusieurs ingrédients additionnels bien connus de l'homme du métier tels que notamment, les agents liants, les agents de granulation, les lubrifiants, les colorants, les charges, les émulsifiants, les minéraux, les agents de pelliculage, les sels, les stabilisants, les tampons ou les vitamines. Les stabilisants comprennent les substances qui ont tendance à augmenter la durée de conservation de la composition tels que les conservateurs, les émulsifiants, les épaississants, les gaz d'emballage, les gélifiants, les humectants, les séquestrants, les synergistes ou les stabilisants.

Pour l'administration par voie orale, les excipients pouvant convenir peuvent être les dérivés de cellulose ou de cellulose microcristalline, les carbonates alcalino-terreux, le phosphate de magnésium, les amidons, les amidons modifiés, le lactose pour les formes solides.

Pour l'usage injectable, la formulation pourra comprendre un solvant aqueux, un solvant organique ou le mélange des deux ou une huile végétale, un solvant organique ou le mélange des deux. Parmi les solvants aqueux, l'eau, les solutés aqueux, le sérum physiologique, les solutés isotoniques sont les excipients les plus souvent utilisés. Parmi les huiles végétales, on peut citer par exemple l'huile de palme, l'huile de maïs, l'huile de coton, l'huile de tournesol, l'huile d'arachide, l'huile d'olive, l'huile de soja, l'huile de carthame, l'huile de coprah, l'huile de sésame, ou parmi les huiles végétales semi-synthétiques obtenues par fractionnement et/ou hydrolyse et/ou estérification totale des huiles végétales naturelles comme par exemple les triglycérides d'acide gras issus d'huiles végétales, comme les triglycérides des acides caprylique, caprique, linoléïque, succinique (vendues sous les dénominations commerciales Miglyol® 810, 812, 818, 820, 829), les esters du propylène glycol et d'acide gras issus d'huile végétale comme les esters du propylène glycol et des acides caprylique et caprique (vendues sous les dénominations commerciales Miglyol® 840), ainsi que leur mélange, ainsi que des esters parmi lesquels la Triacétine (triacétate de glycéryle), l'oléate d'éthyle, par exemple. Parmi les solvants organiques, on peut citer par exemple l'alcool benzylique, l'éthanol, la N-méthyl pyrrolidone, le glycerol-formal, le glycofurol, le Diethylene glycol monoethyl ether, le propylene glycol, le polyéthylène glycol par exemple, le PEG300, le PEG 200 et le PEG 400. La sélection du véhicule est réalisée, de manière à former des solutions liquides, en fonction de sa capacité à dissoudre la substance active à température ambiante sans en modifier la structure chimique et la stabilité. Le véhicule choisi doit être biocompatible et adaptés à la voie injectable. Le véhicule sera choisi parmi les solvants polaires, les solvants apolaires aprotiques ou leur mélange. La composition injectable liquide pourra également comprendre au moins un antioxydant choisi parmi le butylhydroxyanisol (BHA), butylhydroxytoluène (BHT), la vitamine E et ses dérivés, le propylgallate et les mélanges de ceux-ci.

Pour une administration par voie transmuqueuse, notamment rectale, le beurre de cacao ou les stéarates de polyéthylèneglycol sont les excipients préférés.

Pour l'usage percutané ou cutané, notamment sur la peau, les muqueuses ou le poil, en particulier pour les solutions à verser de type « pour-on » ou « spot-on » en médecine vétérinaire, les excipients usuels sont des solvants aqueux, alcooliques, polaire ou non, qui favorisent le passage transcutané, tel que l'eau, l'alcool benzylique, les huiles végétales et minérales, les agents de remise en suspension, les antioxydants, les tensioactifs, notamment un mélange constitué d'alcool benzylique et/ou de labrasol et/ou de laurate de propylène glycol, à titre d'agent de pénétration peut être utilisé.

Pour une utilisation oculaire, les excipients adéquats pourront également être sélectionnés par l'homme du métier en fonction des spécificités requises.

La posologie peut varier dans les limites importantes (0,05 mg à 1000 mg) en fonction de l'indication thérapeutique et de la voie d'administration, ainsi que de l'âge et du poids du sujet.

D'autres utilisations des composés de l'invention sont envisagées, par exemple en tant qu'agent de restriction des contaminations, telle que la formulation dans une pommade nasale.

La présente invention concerne également l'utilisation d'au moins un composé choisi parmi un composé de l'une quelconque des formules (I), (I'), (la), (Ib), (Ic), (Id) et (Ie) telles que définies ci-dessus, et les composés (1) à (56) tels que définis ci-dessus, ou un de ses sels pharmaceutiquement acceptables selon la présente invention pour la fabrication d'une composition pharmaceutique ou vétérinaire destinée à la prévention et/ou au traitement d'un infection bactérienne, fongique, virale ou parasitaire.

## Revendications

1. Composé de formule (I) 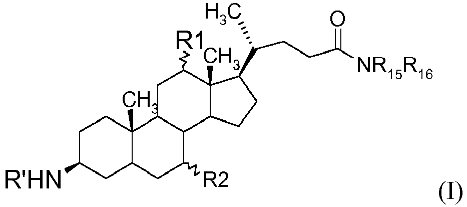 dans laquelle
R1 et R2 représentent indépendamment un atome d'hydrogène, un groupe SO₃H ou un groupe hydroxy,
R' représente un groupe -(CRₐR_{b})ₙ-X-(CR_{c}R_{d})ₘ-[Y-(CRₑR_{f})ₒ]ₜ-NR₉R₁₀, Rₐ, R_{b}, R_{c}, R_{d}, Rₑ et R_{f} représentent indépendamment un atome d'hydrogène, un groupe (C₁-C₈)alkyle ou un groupe (C₆-C₁₀)aryle,
X et Y représentent indépendamment un groupe -NR11-, un groupe -O- ou un groupe hétérocyclique divalent comprenant au moins un atome d'azote, à 5 ou 6 chaînons,
R9 et R10 représentent indépendamment un atome d'hydrogène, un groupe (C₁-C₈)alkyle ou forment ensemble, avec l'atome d'azote qui les porte, un groupe hétérocyclique à 5 ou 6 chaînons, éventuellement substitué par un ou deux groupe(s) =O ou =S,
R11 représente un atome d'hydrogène, un groupe (C₁-C₈)alkyle ou un groupe -(CH₂)ₛ-NH₂,
R15 et R16 représentent indépendamment un atome d'hydrogène, un groupe (C₁-C₈)alkyle ou un groupe (C₆-C₁₀)aryle,
n, m, o et s représentent indépendamment un nombre entier compris entre 1 et 5,
t est égal à 0, 1, 2 ou 3,
ainsi que les stéréoisomères, mélanges de stéréoisomères, et/ou sels pharmaceutiquement acceptables de celui-ci.

2. Composé selon la revendication 1, **caractérisé en ce qu'**il est défini par au moins l'un des sous-groupes suivants :
- premier sous-groupe de composés de formule (I) lesquels R1 et R2 représentent indépendamment un atome d'hydrogène ou un groupe hydroxy,
- deuxième sous-groupe de composés de formule (I) pour lesquels R15 et R16 représentent indépendamment un atome d'hydrogène ou un groupe (C₁-C₄)alkyle,
- troisième sous-groupe de composés de formule (I) pour lesquels X est un groupe -NH-, un groupe hétérocyclique à 6 chaînons comportant un ou deux atomes d'azote, de préférence un groupe 1,4-pipérazinylene ou un groupe 1,4-pipéridinylene,
- quatrième sous-groupe de composés de formule (I) pour lesquels R9 et R10 représentent un atome d'hydrogène,
- cinquième sous-groupe de composés de formule (I) pour lesquels Rₐ, R_{b}, R_{c}, R_{d}, Rₑ et R_{f} représentent un atome d'hydrogène,
- sixième sous-groupe de composés de formule (I) pour lesquels Y est un groupe -NR11-, avec R11 représentant un atome d'hydrogène, un groupe (C₁-C₄)alkyle ou un groupe -(CH₂)ₛ-NH₂ où s est égal à 1, 2 ou 3,
- septième sous-groupe de composés de formule (I) pour lesquels m est égal à 2, 3, 4, ou 5, plus préférentiellement à 2 ou 3,
- huitième sous-groupe de composés de formule (I) pour lesquels n est égal à 2, 3, 4 ou 5, plus préférentiellement à 2, 3 ou 4,
- neuvième sous-groupe de composés de formule (I) pour lesquels m est différente de 4,
- dixième sous-groupe de composés de formule (I) pour lesquels o est égal à 2 ou 3,
- onzième sous-groupe de composés de formule (I) pour lesquels le groupe - NHR' est choisi parmi : 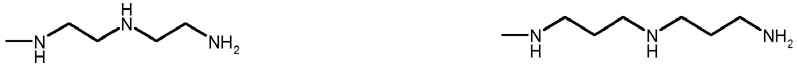 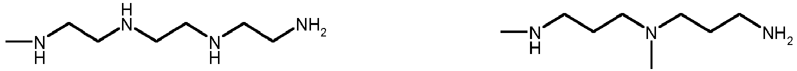 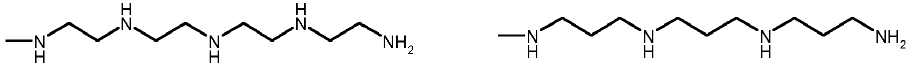 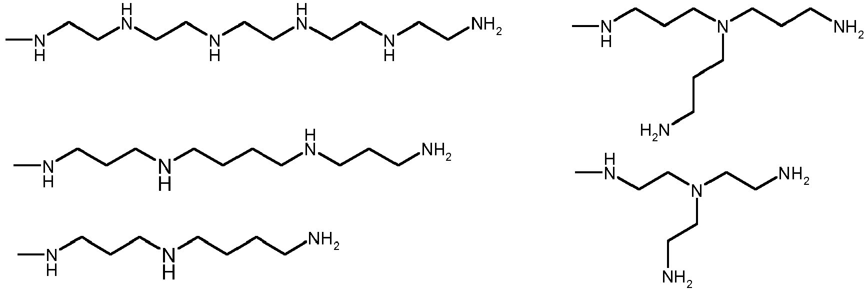 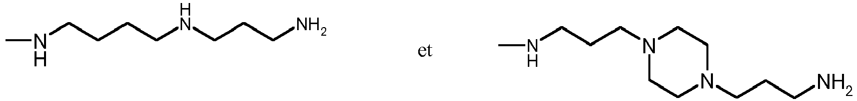 ou
- ou par la combinaison des sous-groupes tels que définis ci-dessus.

3. Composé selon la revendication 1 ou 2, **caractérisé en ce qu'**il représente la formule (I') 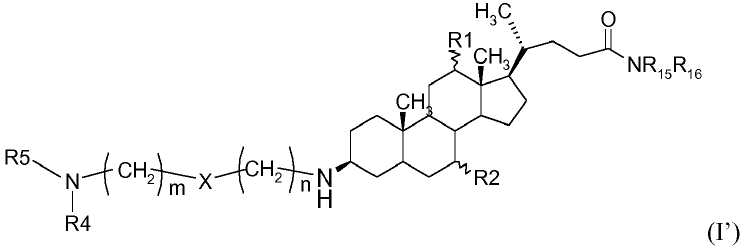 dans laquelle
R1 et R2 sont tels que définis en revendication 1 ou 2,
R15 et R16 représentent indépendamment un atome d'hydrogène ou un groupe (C₁-C₈)alkyle,
n représente le nombre entier 2, 3 ou 4,
m représente le nombre entier 2, 3 ou 4,
X représente un groupe -NR11- ou un groupe hétérocyclique divalent comprenant un ou deux atomes d'azote, à 5 ou 6 chaînons, tel qu'un groupe 1,4-pipérazinylene ou un groupe 1,4-pipéridinylene,
R4 et R11 représentent indépendamment un atome d'hydrogène, un groupe (C₁-C₈)alkyle ou un groupe -(CH₂)ₛ-NH₂,
R5 représente un atome d'hydrogène, un groupe -(CH₂)ₚ-NH₂, un groupe -(CH₂)ₚ-NH-(CH₂)_{q}-NH₂ ou un groupe -(CH₂)ₚ-NH-(CH₂)_{q}-NH-(CH₂)ᵣ-NH₂, p, q, r et s représentent indépendamment un nombre entier pouvant varier entre 1 et 5,
ainsi que les stéréoisomères, mélanges de stéréoisomères, et/ou sels pharmaceutiquement acceptables de celui-ci.

4. Composé selon l'une quelconque des revendications 1 à 3, **caractérisé en ce que** R15 et R16 représentent indépendamment un atome d'hydrogène ou un groupe (C₁-C₄)alkyle tel qu'un méthyle ou un isoproyle.

5. Composé selon l'une quelconque des revendications 1 à 4, **caractérisé en ce que** n est égal à 2 et m est égal à 3, n est égal à 2 et m est égal à 2, n est égale à 3 et m est égal à 4 ou bien n est égal à 3 et m est égal à 3.

6. Composé selon l'une quelconque des revendications 3 à 5, **caractérisé en ce que** X représente un groupe -NR11- ou un groupe 1,4-pipérazinylene et R4 et R11 représentant indépendamment un atome d'hydrogène, un groupe méthyle ou un groupe -(CH₂)ₛ-NH₂, dans lequel s est égal à 2 ou 3.

7. Composé selon l'une quelconque des revendications 3 à 5, **caractérisé en ce que** R5 représente un atome d'hydrogène, un groupe -(CH₂)ₚ-NH₂, un groupe -(CH₂)ₚ-NH-(CH₂)_{q}-NH₂ ou un groupe -(CH₂)ₚ-NH-(CH₂)_{q}-NH-(CH₂)ᵣ-NH₂, avec p est égal à 2 ou 3, q est égal à 2 et r est égal à 2.

8. Composé selon l'une quelconque des précédentes revendications, **caractérisé en ce qu'**il représente alternativement
- la formule (Ia) 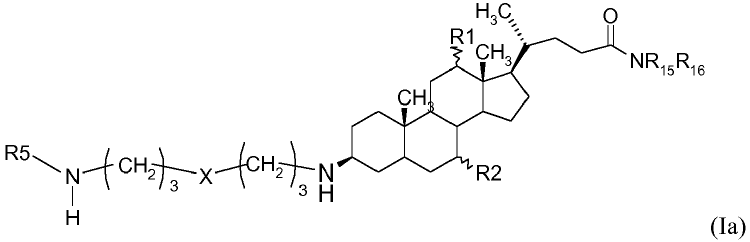 dans laquelle
R15, R16, R1 et R2 sont tels que définis dans l'une quelconque des revendications 3 à 5,
X représente un groupe -NH- ou un groupe 1,4-pipérazinylene,
R5 représente un atome d'hydrogène ou un groupe -(CH₂)ₚ-NH₂, avec p est égal à 2 ou 3,
ainsi que les stéréoisomères, mélanges de stéréoisomères, et/ou sels pharmaceutiquement acceptables de celui-ci,
- la formule (Ib) 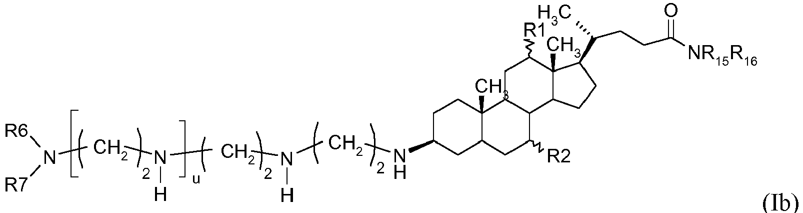 dans laquelle
R15, R16, R1 et R2 sont tels que définis dans l'une quelconque des revendications 3 à 5,
u est égal à 0, 1, 2 ou 3, préférentiellement à 1, 2 ou 3,
R6 et R7 représentent indépendamment un atome d'hydrogène ou un groupe (C₁-C₈)alkyle, de préférence un atome d'hydrogène ou un groupe (C₁-C₄)alkyle,
ainsi que les stéréoisomères, mélanges de stéréoisomères, et/ou sels pharmaceutiquement acceptables de celui-ci,
- la formule (Ic) 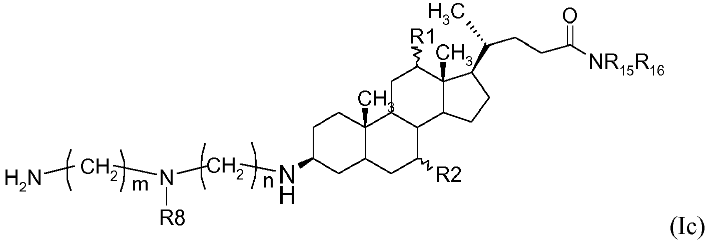 dans laquelle
R15, R16, R1, R2, n et m sont tels que définis dans l'une quelconque des revendications 3 à 5, et
R8 représente un groupe (C₁-C₈)alkyle, de préférence un groupe méthyle, ou un groupe -(CH₂)ₛ-NH₂, avec s étant un nombre entier pouvant varier entre 1 et 5, de préférence égal à 2 ou 3,
ainsi que les stéréoisomères, mélanges de stéréoisomères, et/ou sels pharmaceutiquement acceptables de celui-ci,
- la formule (Id) 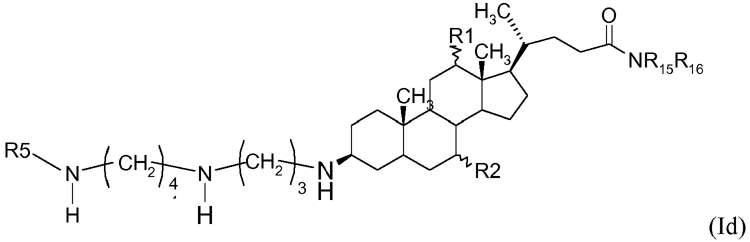 dans laquelle
R15, R16, R1 et R2 sont tels que définis dans l'une quelconque des revendications 3 à 5,
R5 représente un groupe -(CH₂)ₚ-NH₂, avec p est égal à 2 ou 3,
ainsi que les stéréoisomères, mélanges de stéréoisomères, et/ou sels pharmaceutiquement acceptables de celui-ci, ou
- la formule (Ie) 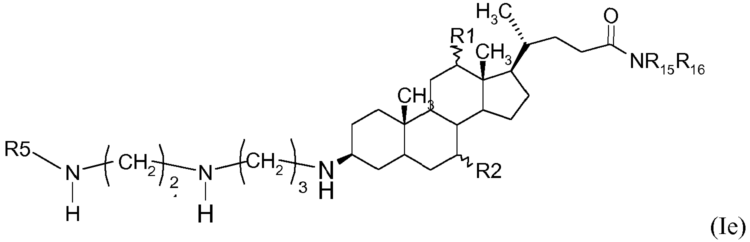 dans laquelle
R15, R16, R1 et R2 sont tels que définis selon l'une quelconque des revendications 3 à 5,
R5 représente un groupe -(CH₂)ₚ-NH₂, avec p est égal à 2 ou 3,ainsi que les stéréoisomères, mélanges de stéréoisomères, et/ou sels pharmaceutiquement acceptables de celui-ci.

9. Composé de formule (I) tel que défini à la revendication 1 choisi parmi les composés suivants :
- **(1)** 3β-norspermino-N-isopropyl-désoxycholamide,
- **(2)** 3β-norspermidino-N-isopropyl-désoxycholamide,
- **(3)** 3β-(1,4-Bis(3-aminopropyl)pipérazine)-N-isopropyl-désoxycholamide,
- **(4)** 3β-norspermino-N-isopropyl-cholamide,
- **(5)** 3β-norspermidino-N-isopropyl-cholamide,
- **(6)** 3β-(1,4-Bis(3-aminopropyl)pipérazine)-N-isopropyl-désoxycholamide,
- **(7)** 3β-norspermino-N-isopropyl-chénodésoxycholamide,
- **(8)** 3β-norspermidino-N-isopropyl-chénodésoxycholamide,
- **(9)** 3β-(1,4-Bis(3-aminopropyl)pipérazine)-N-isopropyl-chénodésoxy cholamide,
- **(10)** 3β-norspermino-N-méthyl-chénodésoxycholamide,
- **(11)** 3β-norspermidino-N-méthyl-chénodésoxycholamide,
- **(12)** 3β-(1,4-Bis(3-aminopropyl)pipérazine)-N-méthyl-chénodésoxy cholamide,
- **(13)** 3β-norspermidino-N,N-diéthyl-chénodésoxycholamide,
- **(14)** 3β-norspermino-N-isopropyl-ursodésoxycholamide,
- **(15)** 3β-norspermidino-N-isopropyl-ursodésoxycholamide,
- **(16)** 3β-(1,4-Bis(3-aminopropyl)pipérazine)-N-isopropyl-ursodésoxy cholamide,
- **(17)** 3β-norspermino-N-isopropyl-lithocholamide,
- **(18)** 3β-norspermidino-N-isopropyl-lithocholamide,
- **(19)** 3β-(1,4-Bis(3-aminopropyl)pipérazine)-N-isopropyl-lithocholamide,
- **(20)** 3β-(pentaéthylènehexamine)-N-isopropyl-désoxycholamide,
- **(21)** 3β-(pentaéthylènehexamine)-N-isopropyl-cholamide,
- **(22)** 3β-(pentaéthylènehexamine)-N-isopropyl-chénodésoxycholamide,
- **(23)** 3β-(pentaéthylènehexamine)-N-isopropyl-ursodésoxycholamide,
- **(24)** N-isopropyl-3β-pentaéthylènehexamine-désoxycholamide,
- **(25)** 3β-(1,4-Bis(3-aminopropyl)pipérazine)-N-isopropyl-désoxycholamide,
- **(26)** 3β-(Bis(3-aminopropyl)méthylamine)-N-isopropyl-cholamide,
- **(27)** 3β-(Bis(3-aminopropyl)méthylamine)-N-isopropyl-chénodésoxy cholamide,
- **(28)** 3β-(Bis(3-aminopropyl)méthylamine)-N-isopropyl-ursodésoxy cholamide,
- **(29)** 3β-(Bis(3-aminopropyl)méthylamine)-N-isopropyl-lithocholamide,
- **(30)** 3β-spermino-N-isopropyl-désoxycholamide,
- **(31)** 3β-spermino-N-isopropyl-cholamide,
- **(32)** 3β-spermino-N-isopropyl-chénodésoxycholamide,
- **(33)** 3β-spermino-N-méthyl-désoxycholamide,
- **(34)** 3β-spermino-N,N-diéthyl-chénodésoxycholamide,
- **(35)** 3β-spermino-N-isopropyl-ursodésoxycholamide,
- **(36)** 3β-spermino-N-isopropyl-lithocholamide,
- **(37)** 3β-norspermidino-N-diisopropyl-chénodésoxycholamide,
- **(38)** 3β-norspermidino-N-cyclohexyl-chénodésoxycholamide,
- **(39)** 3β-norspermino-N,N-diéthyl-chénodésoxycholamide,
- **(40)** 3β-norspermino-N,N-diisopropyl-chénodésoxycholamide,
- **(42)** 3β-(Tris(3-aminopropyl)amine)-N-isopropyl-désoxycholamide,
- **(43)** 3β-(Tris(3-aminopropyl)amine)-N-isopropyl-cholamide,
- **(44)** 3β-(Tris(3-aminopropyl)amine)-N,N-diéthyl-chénodésoxycholamide,
- **(45)** 3β-(Tris(2-aminoéthyl)amine)-N-isopropyl-chénodésoxycholamide,
- **(46)** 3β-(Tris(3-aminopropyl)amine)-N-isopropyl-chénodésoxycholamide,
- **(47)** 3β-(Tris(3-aminopropyl)amine)-N-cyclohexyl-chénodésoxycholamide,
- **(48)** 3β-(Tris(3-aminopropyl)amine)-N-isopropyl-ursodésoxycholamide,
- **(49)** 3β-(Tris(3-aminopropyl)amine)-N-isopropyl-lithocholamide,
- **(50)** 3β-spermino-N,N-diisopropyl-chénodésoxycholamide,
- **(51)** 3β-spermino-N-cyclohexyl-chénodésoxycholamide,
- **(52)** 3β-spermidino-N-isopropyl-chénodésoxycholamide,
- **(53)** 3β-(Bis(3-aminopropyl)éthylènediamine)-N,N-diéthyl-chénodésoxy cholamide,
- **(54)** 3β-(Bis(3-aminopropyl)éthylènediamine)-N,N-diisopropyl-chénodésoxy cholamide,
- **(55)** Mélange 50/50 de 3β-spermidino-N-isopropyl-chénodésoxycholamide et de 3β-N-[4'N-(3'-aminopropyl)aminobutyl]amino-N-isopropyl-chénodésoxycholamide,
- **(56)** 3β-(Tris(3-aminopropyl)amine)-N,N-diisopropyl-chénodésoxy cholamide,
ou l'un de ses sels pharmaceutiquement acceptables, et plus particulièrement choisis parmi les composés **(1)**, **(2)**, **(5)**, **(13)**, **(15)**, **(33)** et **(34)**.

10. Composé de formule (I) selon l'une quelconque des revendications précédentes, pour son utilisation à titre de médicament.

11. Composition pharmaceutique ou vétérinaire comprenant un composé de formule (I) tel que défini dans l'une quelconque des revendications 1 à 9 et un excipient pharmaceutiquement acceptable.

12. Compositions pharmaceutiques ou vétérinaires comprenant au moins un composé de formule (I) tel que défini dans l'une quelconque des revendications 1 à 9 et au moins un antibiotique, différent d'un tel composé de formule (I), plus particulièrement de la famille des bêtalactamines, aminosides, macrolides, polypeptides, sulfamides, quinolones, nitro-imidazolés, dérivés des nitrofuranes, dérivés du noyau benzyl-pyrimidine, des tétracyclines ou des phénicolés, tels que la doxycycline ou le chloramphénicol, la pénicilline, l'ampicilline, amoxicilline, la cloxacilline, la dicloxacilline, l'oxacilline, la nafcilline, la céfalexine, la céfapirine, la céfazoline, le ceftiofur, la céfopérazone, la céfovécine, le cefquinome, la thimaphénicol, le florfénicol, la terramycine, l'érythromycine, la spiramycine, la tylosine, la josamycine, le tilmicosine, la tulathromycine, la gamithromycine, la tildipirosine, la clyndamycine, la lyncomycine, la pirlymicine, le tiamuline, la valnémuline, l'acide oxolinique, la fluméquine, l'enrofloxacine, la danofloxacine, l'ibafloxacine, la marbofloxacine, la difloxacine, l'obifloxacine, la pradofloxacine, la rifampicine, la rifaximine, le sulfaméthizol, la sulfathiazol, la sulfadimidine, la sulfaméthoxazole, la sulfadiazine, la sulfadiméthoxine, la sulfaméthoxypyridazine, le triméthoprime, la baquiloprime, le métronidazole, le dimétridazole, la ronidazole, la nitrofurantoïne, la furazolidone ou la furaltadone, et encore plus particulièrement la doxycycline, l'ampicilline, l'érythromycine ou le chloramphénicol.

13. Composé selon l'une quelconque des revendications 1 à 9 pour son utilisation pour prévenir et/ou inhiber et ou traiter les infections bactériennes, fongiques, virales ou parasitaires chez l'homme ou l'animal.

14. Un composé selon l'une quelconque des revendications 1 à 9, pour son utilisation dans une méthode de potentialiser l'activité antibiotique de composés antibiotiques pouvant être choisis parmi les composés antibiotiques tels que décrits en revendication 12.

15. Procédé de préparation d'un composé de formule (I) tels que définis à l'une quelconque des revendications 1 à 9, comprenant une étape d'amination réductrice du composé de formule (II) 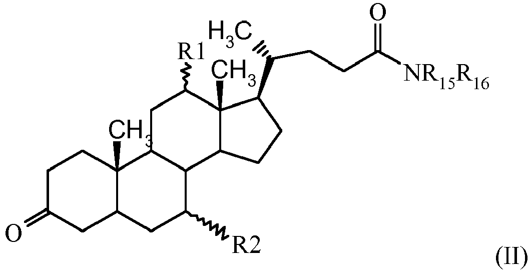
dans laquelle R15, R16, R1 et R2 sont tels que définis dans l'une des revendications 1, 2 ou 3,
avec une amine de formule R'NH2, dans laquelle R' est telle que définie dans la revendication 1, en présence d'un agent réducteur pouvant être choisi parmi le tétraisopropylate de titane, le tétraisopropylate de zirconium, le NaBH₃CN, le NaBH₄, ou d'un mélange d'entre eux, préférentiellement le couple tétraisopropylate de titane/NaBH₄, pour obtenir ledit composé de formule (I).

## Patentansprüche

1. Verbindung der Formel (I) 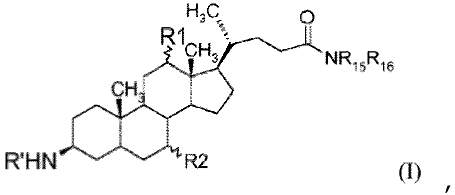 worin
R1 und R2 unabhängig ein Wasserstoffatom, eine Gruppe SO₃H oder eine Hydroxygruppe darstellen,
R' eine Gruppe - (CRₐR_{b})ₙ-X-(CR_{c}R_{d})ₘ-[Y-(CRₑR_{f})ₒ]ₜ-NR₉R₁₀ darstellt,
Rₐ, R_{b}, R_{c}, R_{d}, Rₑ und R_{f} unabhängig ein Wasserstoffatom, eine (C₁-C₈) Alkylgruppe oder eine (C₆-C₁₀) Arylgruppe darstellen,
X und Y unabhängig eine Gruppe -NR11-, eine Gruppe -O- oder eine mindestens ein Stickstoffatom enthaltende zweiwertige 5- oder 6-gliedrige heterozyklische Gruppe darstellen,
R9 und R10 unabhängig ein Wasserstoffatom, eine (C₁-C₈)Alkylgruppe darstellen oder zusammen mit dem Stickstoffatom, das sie trägt, eine 5- oder 6-gliedrige heterozyklische Gruppe bilden, die gegebenenfalls durch eine oder zwei Gruppe(n) =O oder =S substituiert ist,
R11 ein Wasserstoffatom, eine (C₁-C₈)Alkylgruppe oder eine Gruppe -(CH₂)ₛ-NH₂- darstellt,
R15 und R16 unabhängig ein Wasserstoffatom, eine (C₁-C₈)Alkylgruppe oder eine (C₆-C₁₀)Arylgruppe darstellen, n, m, o und s unabhängig eine ganze Zahl zwischen 1 und 5 darstellen,
t gleich 0, 1, 2 oder 3 ist,
sowie deren Stereoisomere, Stereoisomerengemische und/oder pharmazeutisch akzeptablen Salze.

2. Verbindung nach Anspruch 1, **dadurch gekennzeichnet, dass** sie definiert ist durch mindestens eine der folgenden Untergruppen:
- erste Untergruppe von Verbindungen der Formel (I), worin R1 und R2 unabhängig ein Wasserstoffatom oder eine Hydroxygruppe darstellen,
- zweite Untergruppe von Verbindungen der Formel (I), worin R15 und R16 unabhängig ein Wasserstoffatom oder eine (C₁-C₄)Alkylgruppe darstellen,
- dritte Untergruppe von Verbindungen der Formel (I), worin X eine Gruppe -NH-, eine 6-gliedrige heterozyklische Gruppe mit einem oder zwei Stickstoffatomen, vorzugsweise eine 1,4-Piperazinylengruppe oder eine 1,4-Piperidinylengruppe, ist,
- vierte Untergruppe von Verbindungen der Formel (I), worin R9 und R10 ein Wasserstoffatom darstellen,
- fünfte Untergruppe von Verbindungen der Formel (I), worin Rₐ, R_{b}, R_{c}, R_{d}, Rₑ und R_{f} ein Wasserstoffatom darstellen,
- sechste Untergruppe von Verbindungen der Formel (I), worin Y eine Gruppe -NR11- ist, wobei R11 ein Wasserstoffatom, eine (C₁-C₄)Alkylgruppe oder eine Gruppe -(CH₂)ₛ-NH₂-, worin s gleich 1, 2 oder 3 ist, darstellt,
- siebte Untergruppe von Verbindungen der Formel (I), worin m gleich 2, 3, 4 oder 5, vorzugsweise 2 oder 3, ist,
- achte Untergruppe von Verbindungen der Formel (I), worin n gleich 2, 3, 4 oder 5, vorzugsweise 2, 3 oder 4, ist,
- neunte Untergruppe von Verbindungen der Formel (I), worin m verschieden von 4 ist,
- zehnte Untergruppe von Verbindungen der Formel (I), worin o gleich 2 oder 3 ist,
- elfte Untergruppe von Verbindungen der Formel (I), worin die Gruppe -NHR' ausgewählt ist aus: 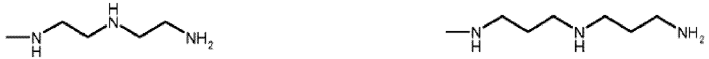 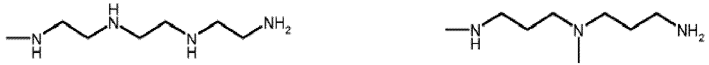 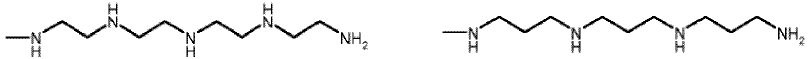 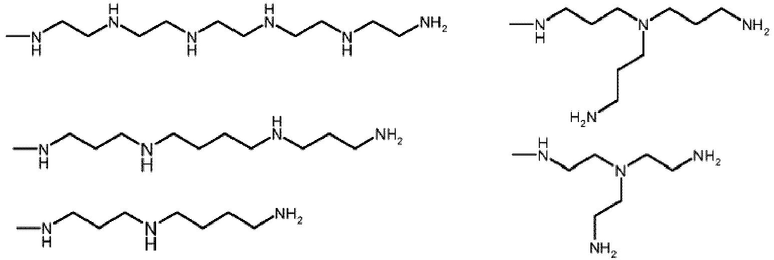 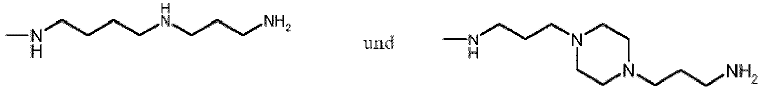 oder
- durch Kombination der vorstehend definierten Untergruppen.

3. Verbindung nach Anspruch 1 oder 2, **dadurch gekennzeichnet, dass** sie Formel (I') darstellt 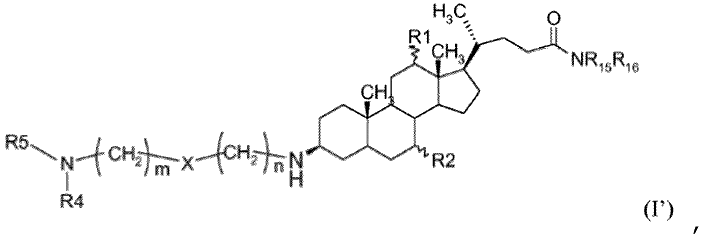 worin
R1 und R2 wie in Anspruch 1 oder 2 definiert sind,
R15 und R16 unabhängig ein Wasserstoffatom oder eine (C₁-C₈)Alkylgruppe darstellen,
n die ganze Zahl 2, 3 oder 4 darstellt,
m die ganze Zahl 2, 3 oder 4 darstellt,
X eine Gruppe -NR11- oder eine zweiwertige 5- oder 6-gliedrige heterozyklische Gruppe mit einem oder zwei Stickstoffatomen, wie eine 1,4-Piperazinylengruppe oder eine 1,4-Piperidinylengruppe, darstellt,
R4 und R11 unabhängig ein Wasserstoffatom, eine (C₁-C₈)Alkylgruppe oder eine Gruppe -(CH₂)ₛ-NH₂-darstellen,
R5 ein Wasserstoffatom, eine Gruppe - (CH₂) ₚ-NH₂-, eine Gruppe - (CH₂) ₚ-NH- (CH₂) _{q}-NH₂- oder eine Gruppe -(CH₂)ₚ-NH-(CH₂)_{q}-NH- (CH₂)ᵣ-NH₂- darstellt,
p, q, r und s unabhängig eine ganze Zahl darstellen, die zwischen 1 und 5 variieren kann,
sowie deren Stereoisomere, Stereoisomerengemische und/oder pharmazeutisch akzeptablen Salze.

4. Verbindung nach einem der Ansprüche 1 bis 3, **dadurch gekennzeichnet, dass** R15 und R16 unabhängig ein Wasserstoffatom oder eine (C₁-C₄)Alkylgruppe, wie ein Methyl oder ein Isopropyl, darstellen.

5. Verbindung nach einem der Ansprüche 1 bis 4, **dadurch gekennzeichnet, dass** n gleich 2 ist und m gleich 3 ist, n gleich 2 ist und m gleich 2 ist, n gleich 3 ist und m gleich 4 ist oder n gleich 3 ist und m gleich 3 ist.

6. Verbindung nach einem der Ansprüche 3 bis 5, **dadurch gekennzeichnet, dass** X eine Gruppe -NR11- oder eine 1,4-Piperazinylengruppe darstellt und R4 und R11 unabhängig ein Wasserstoffatom, eine Methylgruppe oder eine Gruppe -(CH₂)ₛ-NH₂-, worin s gleich 2 oder 3 ist, darstellen.

7. Verbindung nach einem der Ansprüche 3 bis 5, **dadurch gekennzeichnet, dass** R5 ein Wasserstoffatom, eine Gruppe -(CH₂)ₚ-NH₂-, eine Gruppe - (CH₂)ₚ-NH- (CH₂) _{q}-NH₂- oder eine Gruppe - (CH₂) ₚ-NH- (CH₂) _{q}-NH- (CH₂) ᵣ-NH₂-, worin p gleich 2 oder 3 ist, q gleich 2 ist und r gleich 2 ist, darstellt.

8. Verbindung nach einem der vorhergehenden Ansprüche, **dadurch gekennzeichnet, dass** sie alternativ darstellt
- Formel (Ia) 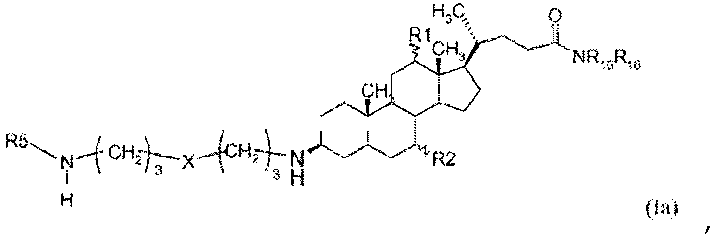 worin
R15, R16, R1 und R2 wie in einem der Ansprüche 3 bis 5 definiert sind,
X eine Gruppe -NH- oder eine 1,4-Piperazinylengruppe darstellt,
R5 ein Wasserstoffatom oder eine Gruppe - (CH₂)ₚ-NH₂-, worin p gleich 2 oder 3 ist, darstellt,
sowie deren Stereoisomere, Stereoisomerengemische und/oder pharmazeutisch akzeptablen Salze,
- Formel (Ib) 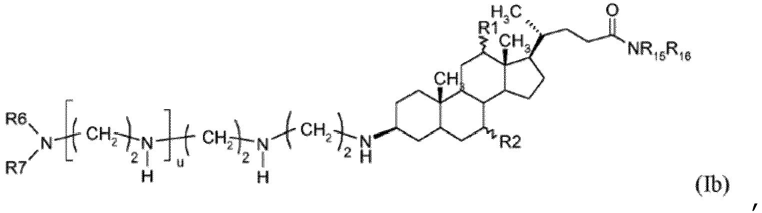 worin
R15, R16, R1 und R2 wie in einem der Ansprüche 3 bis 5 definiert sind,
u gleich 0, 1, 2 oder 3, vorzugsweise 1, 2 oder 3, ist,
R6 und R7 unabhängig ein Wasserstoffatom oder eine (C₁-C₈)Alkylgruppe, vorzugsweise ein Wasserstoffatom oder eine (C₁-C₄)Alkylgruppe, darstellen,
sowie deren Stereoisomere, Stereoisomerengemische und/oder pharmazeutisch akzeptablen Salze,
- Formel (Ic) 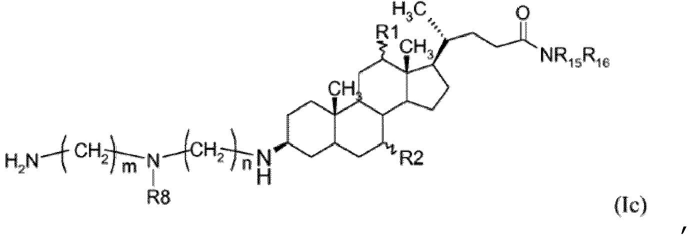 worin
R15, R16, R1, R2, n und m wie in einem der Ansprüche 3 bis 5 definiert sind und
R8 eine (C₁-C₈)Alkylgruppe, vorzugsweise eine Methylgruppe, oder eine Gruppe -(CH₂)ₛ-NH₂-, worin s eine ganze Zahl, die zwischen 1 und 5 variieren kann, vorzugsweise gleich 2 oder 3, ist, darstellt,
sowie deren Stereoisomere, Stereoisomerengemische und/oder pharmazeutisch akzeptablen Salzen,
- Formel (Id) 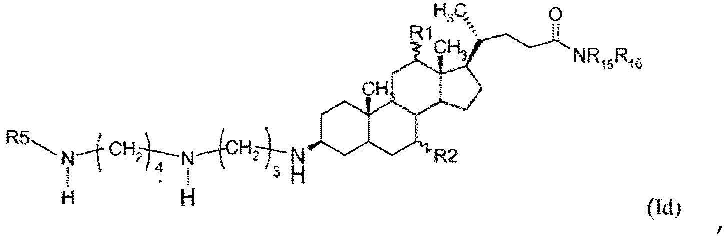 worin
R15, R16, R1 und R2 wie in einem der Ansprüche 3 bis 5 definiert sind,
R5 eine Gruppe -(CH₂)ₚ-NH₂-, worin p gleich 2 oder 3 ist, darstellt,
sowie deren Stereoisomere, Stereoisomerengemische und/oder pharmazeutisch akzeptablen Salze, oder
- Formel (Ie) 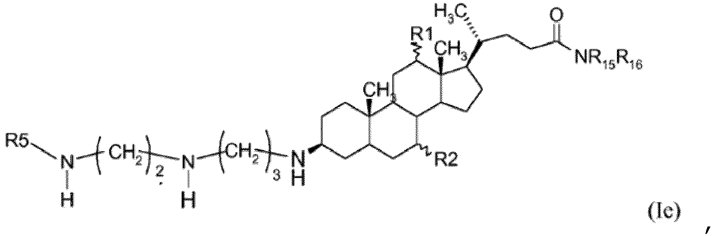 worin
R15, R16, R1 und R2 wie gemäß einem der Ansprüche 3 bis 5 definiert sind,
R5 eine Gruppe -(CH₂)p-NH₂-, worin p gleich 2 oder 3 ist, darstellt,
sowie deren Stereoisomere, Stereoisomerengemische und/oder pharmazeutisch akzeptablen Salze.

9. Verbindung der Formel (I) nach Anspruch 1, ausgewählt aus den folgenden Verbindungen:
- (1) 3β-Norspermino-N-isopropyldesoxycholamid,
- (2) 3β-Norspermidino-N-isopropyldesoxycholamid,
- (3) 3β-(1,4-Bis(3-aminopropyl)piperazin)-N-isopropyldesoxycholamid,
- (4) 3β-Norspermino-N-isopropylcholamid,
- (5) 3β-Norspermidino-N-isopropylcholamid,
- (6) 3β-(1,4-Bis(3-aminopropyl)piperazin)-N-isopropyldesoxycholamid),
- (7) 3β-Norspermino-N-isopropylchenodesoxycholamid,
- (8) 3β-Norspermidino-N-isopropylchenodesoxycholamid,
- (9) 3β-(1,4-Bis(3-aminopropyl)piperazin)-N-isopropylchenodesoxycholamid,
- (10) 3ß-Norspermino-N-methylchenodesoxycholamid,
- (11) 3ß-Norspermidino-N-methylchenodesoxycholamid,
- (12) 3ß-(1,4-Bis(3-aminopropyl)piperazin)-N-methylchenodesoxycholamid,
- (13) 3β-Norspermidino-N,N-diethylchenodesoxycholamid,
- (14) 3β-Norspermino-N-isopropylursodesoxycholamid,
- (15) 3β-Norspermidino-N-isopropylursodesoxycholamid,
- (16) 3β-(1,4-Bis(3-aminopropyl)piperazin)-N-isopropylursodesoxycholamid,
- (17) 3β-Norspermino-N-isopropyllithocholamid,
- (18) 3β-Norspermidino-N-isopropyllithocholamid,
- (19) 3β-(1,4-Bis(3-aminopropyl)piperazin)-N-isopropyllithocholamid,
- (20) 3β-(Pentaethylenhexamin)-N-isopropyldesoxycholamid,
- (21) 3β-(Pentaethylenhexamin)-N-isopropylcholamid,
- (22) 3β-(Pentaethylenhexamin)-N-isopropylchenodesoxycholamid,
- (23) 3β-(Pentaethylenhexamin)-N-isopropylursodesoxycholamid,
- (24) N-Isopropyl-3β-pentaethylenhexamindesoxycholamid,
- (25) 3β-1,4-Bis(3-aminopropyl)piperazin)-N-isopropyldesoxycholamid,
- (26) 3β-(Bis(3-aminopropyl)methylamin)-N-isopropylcholamid,
- (27) 3β-(Bis(3-aminopropyl)methylamin)-N-isopropylchenodesoxycholamid,
- (28) 3β-(Bis(3-aminopropyl)methylamin)-N-isopropylursodesoxycholamid,
- (29) 3β-(Bis(3-aminopropyl)methylamin)-N-isopropyllithocholamid,
- (30) 3ß-Spermino-N-isopropyldesoxycholamid,
- (31) 3ß-Spermino-N-isopropylcholamid,
- (32) 3ß-Spermino-N-isopropylchenodesoxycholamid,
- (33) 3ß-Spermino-N-methyldesoxycholamid,
- (34) 3ß-Spermino-N,N-diethylchenodesoxycholamid,
- (35) 3ß-Spermino-N-isopropylursodesoxycholamid,
- (36) 3ß-Spermino-N-isopropyllithocholamid,
- (37) 3ß-Norspermidino-N-diisopropylchenodesoxycholamid,
- (38) 3β-Norspermidino-N-cyclohexylchenodesoxycholamid,
- (39) 3β-Norspermino-N,N-diethylchenodesoxycholamid,
- (40) 3β-Norspermino-N,N-diisopropylchenodesoxycholamid,
- (42) 3β-(Tris(3-aminopropyl)amin)-N-isopropyldesoxycholamid,
- (43) 3β-(Tris(3-aminopropyl)amin)-N-isopropylcholamid,
- (44) 3β-(Tris(3-aminopropyl)amin)-N,N-diethylchenodesoxycholamid,
- (45) 3β-(Tris(2-aminoethyl)amin)-N-isopropylchenodesoxycholamid,
- (46) 3ß-(Tris(3-aminopropyl)amin)-N-isopropylchenodesoxycholamid,
- (47) 3β-(Tris(3-aminopropyl)amin)-N-cyclohexylchenodesoxycholamid,
- (48) 3β-(Tris(3-aminopropyl)amin)-N-isopropylursodesoxycholamid,
- (49) 3β-(Tris(3-aminopropyl)amin)-N-isopropyllithocholamid,
- (50) 3β-Spermino-N,N-diisopropylchenodesoxycholamid,
- (51) 3β-Spermino-N-cyclohexylchenodesoxycholamid,
- (52) 3β-Spermidino-N-isopropylchenodesoxycholamid,
- (53) 3β-(Bis(3-aminopropyl)ethylendiamin)-N,N-diethylchenodesoxycholamid,
- (54) 3β-(Bis(3-aminopropyl)ethylendiamin)-N,N-diisopropylchenodesoxycholamid,
- (55) 50/50-Gemisch von 3β-Spermidino-N-isopropylchenodesoxycholamid und 3β-N-([4'N-(3'-Aminopropyl)aminobutyl]amino-N-isopropylchenodesoxycholamid,
- (56) 3β-(Tris(3-aminopropyl)amin)-N,N-diisopropylchenodesoxycholamid
oder einem ihrer pharmazeutisch akzeptablen Salze und insbesondere ausgewählt aus den Verbindungen (1), (2), (5), (13), (15), (33) und (34).

10. Verbindung der Formel (I) nach einem der vorhergehenden Ansprüche zur Verwendung als Arzneimittel.

11. Pharmazeutische oder veterinärmedizinische Zusammensetzung, umfassend eine Verbindung der Formel (I) nach einem der Ansprüche 1 bis 9 und einen pharmazeutisch akzeptablen Exzipienten.

12. Pharmazeutische oder veterinärmedizinische Zusammensetzungen, umfassend mindestens eine Verbindung der Formel (I) nach einem der Ansprüche 1 bis 9 und mindestens ein Antibiotikum, das verschieden von einer solchen Verbindung der Formel (I) ist, insbesondere aus der Familie der Beta-Lactame, Aminoglykoside, Makrolide, Polypeptide, Sulfonamide, Chinolone, Nitroimidazole, Derivate von Nitrofuranen, Derivate des Benzylpyrimidin-Rings, Tetrazykline oder Phenicole, wie Doxycyclin oder Chloramphenicol, Penicillin, Ampicillin, Amoxicillin, Cloxacillin, Dicloxacillin, Oxacillin, Nafcillin, Cefalexin, Cefapirin, Cefazolin, Ceftiofur, Cefoperazon, Cefovecin, Cefquinom, Thimaphenicol, Florfenicol, Terramycin, Erythromycin, Spiramycin, Tylosin, Josamycin, Tilmicosin, Tulathromycin, Gamithromycin, Tildipirosin, Clyndamycin, Lyncomycin, Pirlymicin, Tiamulin, Valnemulin, Oxolinsäure, Flumequin, Enrofloxacin, Danofloxacin, Ibafloxacin, Marbofloxacin, Difloxacin, Obifloxacin, Pradofloxacin, Rifampicin, Rifaximin, Sulfamethizol, Sulfathiazol, Sulfadimidin, Sulfamethoxazol, Sulfadiazin, Sulfadimethoxin, Sulfamethoxypyridazin, Trimethoprim, Baquiloprim, Metronidazol, Dimetridazol, Ronidazol, Nitrofurantoin, Furazolidon oder Furaltadon und noch stärker bevorzugt Doxycyclin, Ampicillin, Erythromycin oder Chloramphenicol.

13. Verbindung nach einem der Ansprüche 1 bis 9 zur Verwendung zum Verbeugen und/oder Hemmen und/oder Behandeln von Bakterien-, Pilz-, Viren- oder Parasiteninfektionen bei einem Menschen oder Tier.

14. Verbindung nach einem der Ansprüche 1 bis 9 zur Verwendung bei einem Verfahren zur Potenzierung der antibiotischen Aktivität von Antibiotikaverbindungen, die aus den Antibiotikaverbindungen nach Anspruch 12 ausgewählt werden können.

15. Verfahren zur Herstellung einer Verbindung der Formel (I) nach einem der Ansprüche 1 bis 9, umfassend einen Schritt der reduktiven Aminierung der Verbindung der Formel (II) 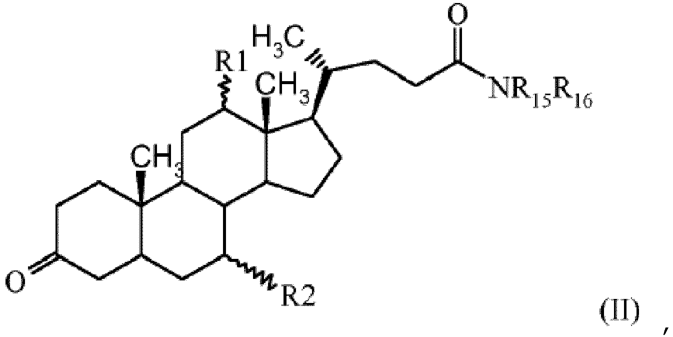
worin R15, R16, R1 und R2 wie in einem der Ansprüche 1, 2 oder 3 definiert sind,
mit einem Amin der Formel R'NH2, worin R' wie in Anspruch 1 definiert ist, in Gegenwart eines Reduktionsmittels, das aus Titantetraisopropylat, Zirkontetraisopropylat, NaBH₃CN, NaBH₄ oder einem Gemisch davon, insbesondere dem Paar Titantetraisopropylat/NaBH₄, ausgewählt werden kann, zum Erhalten der Verbindung der Formel (I).

## Claims

1. Compound of formula (I) 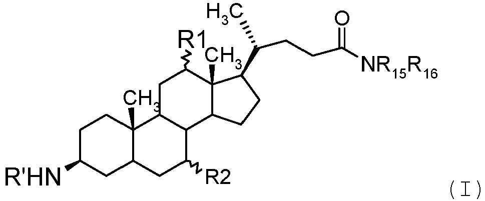 in which
R1 and R2 independently represent a hydrogen atom, an SO₃H group or a hydroxyl group,
R' represents a group - (CRₐR_{b})ₙ-X-(CR_{c}R_{d})ₘ-[Y-(CRₑR_{f})ₒ]ₜ-NR₉R₁₀,
Rₐ, R_{b}, R_{c}, R_{d}, Rₑ and R_{f} independently represent a hydrogen atom, a (C₁-C₈) alkyl group or a (C₆-C₁₀) aryl group,
X and Y independently represent a group -NR11-, a group -O- or a divalent 5-membered or 6-membered heterocyclic group comprising at least one nitrogen atom,
R9 and R10 independently represent a hydrogen atom, a (C₁-C₈) alkyl group or form, together with the nitrogen atom that bears them, a 5-membered or 6-membered heterocyclic group, optionally substituted with one or two groups =O or =S,
R11 represents a hydrogen atom, a (C₁-C₈)alkyl group or a -(CH₂)ₛ-NH₂ group,
R15 and R16 independently represent a hydrogen atom, a (C₁-C₈)alkyl group or a (C₆-C₁₀) aryl group,
n, m, o and s independently represent an integer between 1 and 5,
t is equal to 0, 1, 2 or 3,
and also the stereoisomers, mixtures of stereoisomers, and/or pharmaceutically acceptable salts thereof.

2. Compound according to Claim 1, **characterized in that** it is defined by at least one of the following subgroups:
- first subgroup of compounds of formula (I) for which R1 and R2 independently represent a hydrogen atom or a hydroxyl group,
- second subgroup of compounds of formula (I) for which R15 and R16 independently represent a hydrogen atom or a (C₁-C₄) alkyl group,
- third subgroup of compounds of formula (I) for which X is an -NH- group, a 6-membered heterocyclic group including one or two nitrogen atoms, preferably a 1,4-piperazinylene group or a 1,4-piperidinylene group,
- fourth subgroup of compounds of formula (I) for which R9 and R10 represent a hydrogen atom,
- fifth subgroup of compounds of formula (I) for which Rₐ, R_{b}, R_{c}, R_{d}, Rₑ and R_{f} represent a hydrogen atom,
- sixth subgroup of compounds of formula (I) for which Y is a group -NR11-, with R11 representing a hydrogen atom, a (C₁-C₄) alkyl group or a -(CH₂)ₛ-NH₂ group in which s is equal to 1, 2 or 3,
- seventh subgroup of compounds of formula (I) for which m is equal to 2, 3, 4 or 5, more preferentially 2 or 3,
- eighth subgroup of compounds of formula (I) for which n is equal to 2, 3, 4 or 5, more preferentially 2, 3 or 4,
- ninth subgroup of compounds of formula (I) for which m is other than 4,
- tenth subgroup of compounds of formula (I) for which o is equal to 2 or 3,
- eleventh subgroup of compounds of formula (I) for which the group -NHR' is chosen from: 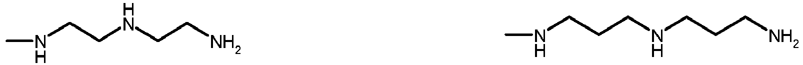 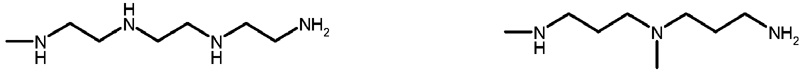 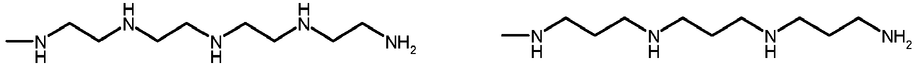 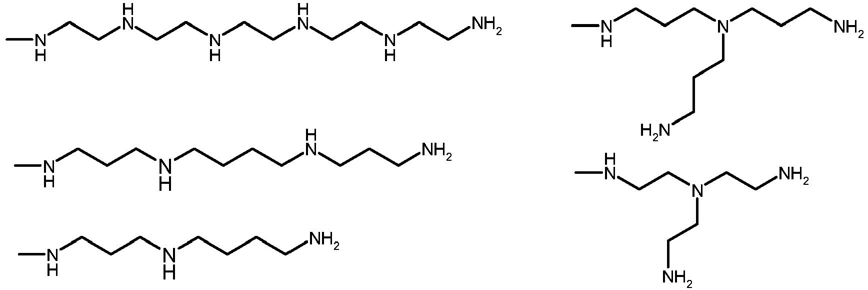 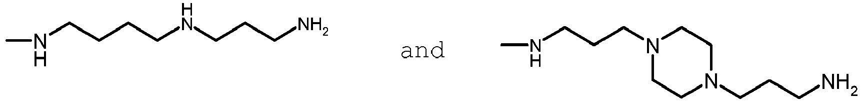
- or by the combination of the subgroups as defined above.

3. Compound according to Claim 1 or 2, **characterized in that** it represents formula (I') 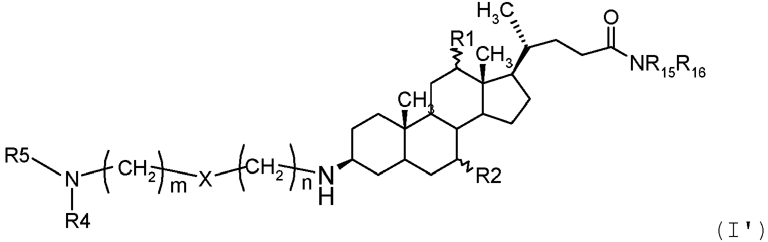 in which
R1 and R2 are as defined in Claim 1 or 2,
R15 and R16 independently represent a hydrogen atom or a (C₁-C₈)alkyl group,
n represents the integer 2, 3 or 4,
m represents the integer 2, 3 or 4,
X represents a group -NR11- or a divalent 5-membered or 6-membered heterocyclic group comprising one or two nitrogen atoms, such as a 1,4-piperazinylene group or a 1,4-piperidinylene group,
R4 and R11 independently represent a hydrogen atom, a (C₁-C₈)alkyl group or a -(CH₂)ₛ-NH₂ group,
R5 represents a hydrogen atom, a -(CH₂)ₚ-NH₂ group, a - (CH₂)ₚ-NH-(CH₂)_{q}-NH₂ group or a - (CH₂)ₚ-NH-(CH₂)_{q}-NH-(CH₂)ᵣ-NH₂ group,
p, q, r and s independently represent an integer which may range between 1 and 5,
and also the stereoisomers, mixtures of stereoisomers, and/or pharmaceutically acceptable salts thereof.

4. Compound according to any one of Claims 1 to 3, **characterized in that** R15 and R16 independently represent a hydrogen atom or a (C₁-C₄)alkyl group such as a methyl or an isopropyl.

5. Compound according to any one of Claims 1 to 4, **characterized in that** n is equal to 2 and m is equal to 3, n is equal to 2 and m is equal to 2, n is equal to 3 and m is equal to 4 or n is equal to 3 and m is equal to 3.

6. Compound according to any one of Claims 3 to 5, **characterized in that** X represents a group -NR11- or a 1,4-piperazinylene group and R4 and R11 independently represent a hydrogen atom, a methyl group or a -(CH₂)ₛ-NH₂ group, in which s is equal to 2 or 3.

7. Compound according to any one of Claims 3 to 5, **characterized in that** R5 represents a hydrogen atom, a - (CH₂)ₚ-NH₂ group, a - (CH₂)ₚ-NH-(CH₂)_{q}-NH₂ group or a - (CH₂)ₚ-NH-(CH₂)_{q}-NH-(CH₂)ᵣ-NH₂ group, in which p is equal to 2 or 3, q is equal to 2 and r is equal to 2.

8. Compound according to any one of the preceding claims, **characterized in that** it alternatively represents
- formula (Ia) 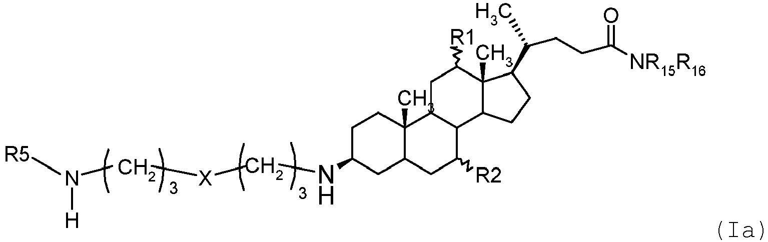 in which
R15, R16, R1 and R2 are as defined in any one of Claims 3 to 5,
X represents an -NH- group or a 1,4-piperazinylene group, R5 represents a hydrogen atom or a -(CH₂)ₚ-NH₂ group, in which p is equal to 2 or 3,
and also the stereoisomers, mixtures of stereoisomers, and/or pharmaceutically acceptable salts thereof,
- formula (Ib) 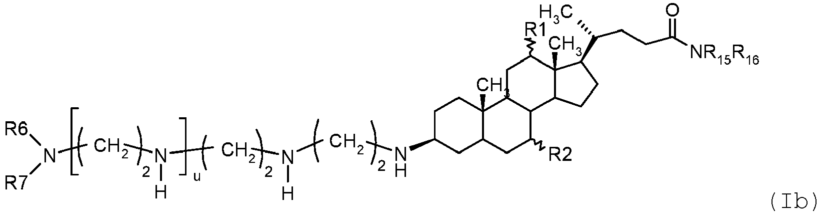 in which
R15, R16, R1 and R2 are as defined in any one of Claims 3 to 5,
u is equal to 0, 1, 2 or 3, preferentially equal to 1, 2 or 3,
R6 and R7 independently represent a hydrogen atom or a (C₁-C₈)alkyl group, preferably a hydrogen atom or a (C₁-C₄)alkyl group,
and also the stereoisomers, mixtures of stereoisomers, and/or pharmaceutically acceptable salts thereof,
- formula (Ic) 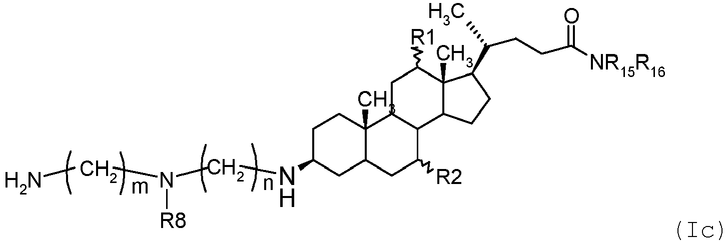 in which
R15, R16, R1, R2, n and m are as defined in any one of Claims 3 to 5, and
R8 represents a (C₁-C₈) alkyl group, preferably a methyl group, or a -(CH₂)ₛ-NH₂ group, with s being an integer which may range between 1 and 5, preferably equal to 2 or 3,
and also the stereoisomers, mixtures of stereoisomers, and/or pharmaceutically acceptable salts thereof,
- formula (Id) 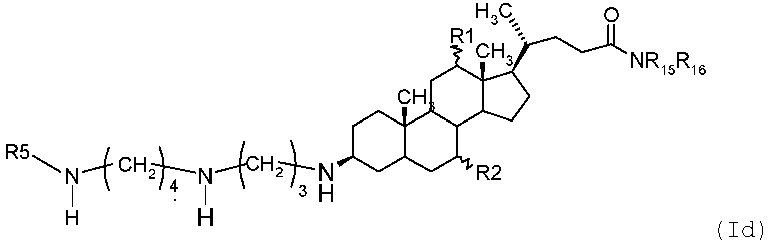 in which
R15, R16, R1 and R2 are as defined in any one of Claims 3 to 5,
R5 represents a -(CH₂)ₚ-NH₂ group, in which p is equal to 2 or 3,
and also the stereoisomers, mixtures of stereoisomers, and/or pharmaceutically acceptable salts thereof, or
- formula (Ie) 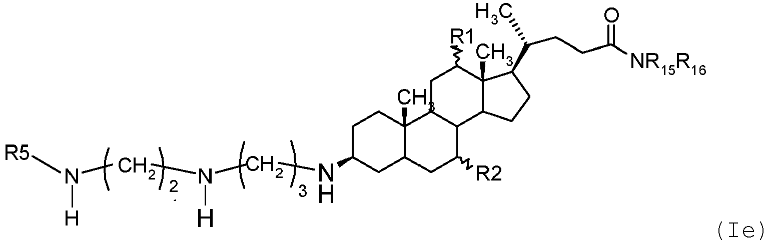 in which
R15, R16, R1 and R2 are as defined according to any one of Claims 3 to 5,
R5 represents a -(CH₂)p-NH₂ group, in which p is equal to 2 or 3, and also the stereoisomers, mixtures of stereoisomers, and/or pharmaceutically acceptable salts thereof.

9. Compound of formula (I) as defined in Claim 1, chosen from the following compounds:
- **(1)** 3β-norspermino-N-isopropyldeoxycholamide,
- **(2)** 3β-norspermidino-N-isopropyldeoxycholamide,
- **(3)** 3β-(1,4-bis(3-aminopropyl)piperazine)-N-isopropyldeoxycholamide,
- **(4)** 3β-norspermino-N-isopropylcholamide,
- **(5)** 3β-norspermidino-N-isopropylcholamide,
- **(6)** 3β-(1,4-bis(3-aminopropyl)piperazine)-N-isopropyldeoxycholamide,
- **(7)** 3β-norspermino-N-isopropylchenodeoxycholamide,
- **(8)** 3β-norspermidino-N-isopropylchenodeoxycholamide,
- **(9)** 3β-(1,4-bis(3-aminopropyl)piperazine)-N-isopropylchenodeoxycholamide,
- **(10)** 3β-norspermino-N-methylchenodeoxycholamide,
- **(11)** 3β-norspermidino-N-methylchenodeoxycholamide,
- **(12)** 3β-(1,4-bis(3-aminopropyl)piperazine)-N-methylchenodeoxycholamide,
- **(13)** 3β-norspermidino-N,N-diethylchenodeoxycholamide,
- **(14)** 3β-norspermino-N-isopropylursodeoxycholamide,
- **(15)** 3β-norspermidino-N-isopropylursodeoxycholamide,
- **(16)** 3β-(1,4-bis(3-aminopropyl)piperazine)-N-isopropylursodeoxycholamide,
- **(17)** 3β-norspermino-N-isopropyllithocholamide,
- **(18)** 3β-norspermidino-N-isopropyllithocholamide,
- **(19)** 3β-(1,4-bis(3-aminopropyl)piperazine)-N-isopropyllithocholamide,
- **(20)** 3β-(pentaethylenehexamine)-N-isopropyldeoxycholamide,
- **(21)** 3β-(pentaethylenehexamine)-N-isopropylcholamide,
- **(22)** 3β-(pentaethylenehexamine)-N-isopropylchenodeoxycholamide,
- **(23)** 3β-(pentaethylenehexamine)-N-isopropylursodeoxycholamide,
- **(24)** N-isopropyl-3β-pentaethylenehexaminedeoxycholamide,
- **(25)** 3β-(1,4-bis(3-aminopropyl)piperazine)-N-isopropyldeoxycholamide,
- **(26)** 3β-(bis(3-aminopropyl)methylamine)-N-isopropylcholamide,
- **(27)** 3β-(bis(3-aminopropyl)methylamine)-N-issopropylchenodeoxycholamide,
- **(28)** 3β-(bis(3-aminopropyl)methylamine)-N-isopropylursodeoxycholamide,
- **(29)** 3β-(bis(3-aminopropyl)methylamine)-N-isopropyllithocholamide,
- **(30)** 3β-spermino-N-isopropyldeoxycholamide,
- **(31)** 3β-spermino-N-isopropylcholamide,
- **(32)** 3β-spermino-N-isopropylchenodeoxycholamide,
- **(33)** 3β-spermino-N-methyldeoxycholamide,
- **(34)** 3β-spermino-N,N-diethylchenodeoxycholamide,
- **(35)** 3β-spermino-N-isopropylursodeoxycholamide,
- **(36)** 3β-spermino-N-isopropyllithocholamide,
- **(37)** 3β-norspermidino-N-diisopropylchenodeoxy-cholamide,
- **(38)** 3β-norspermidino-N-cyclohexylchenodeoxycholamide,
- **(39)** 3β-norspermino-N,N-diethylchenodeoxycholamide,
- **(40)** 3β-norspermino-N,N-diisopropylchenodeoxycholamide,
- **(42)** 3β-(tris(3-aminopropyl)amine)-N-isopropyldeoxycholamide,
- **(43)** 3β-(tris(3-aminopropyl)amine)-N-isopropylcholamide,
- **(44)** 3β-(tris(3-aminopropyl)amine)-N,N-diethylchenodeoxycholamide,
- **(45)** 3β-(tris(2-aminoethyl)amine)-N-isopropylchenodeoxycholamide,
- **(46)** 3β-(tris(3-aminopropyl)amine)-N-isopropylchenodeoxychol-amide,
- **(47)** 3β-(tris(3-aminopropyl)amine)-N-cyclohexylchenodeoxycholamide,
- **(48)** 3β-(tris(3-aminopropyl)amine)-N-isopropylursodeoxycholamide,
- **(49)** 3β-(tris(3-aminopropyl)amine)-N-isopropyllithocholamide,
- **(50)** 3β-spermino-N,N-diisopropylchenodeoxycholamide,
- **(51)** 3β-spermino-N-cyclohexylchenodeoxycholamide,
- **(52)** 3β-spermidino-N-isopropylchenodeoxycholamide,
- **(53)** 3β-(bis(3-aminopropyl)ethylenediamine)-N,N-diethylchenodeoxycholamide,
- **(54)** 3β-(bis(3-aminopropyl)ethylenediamine)-N,N-diisopropylchenodeoxycholamide,
- **(55)** 50/50 mixture of 3β-spermidino-N-isopropylchenodeoxycholamide and of 3β-N-[4'N-(3'-aminopropyl)aminobutyl]amino-N-isopropylchenodeoxycholamide,
- **(56)** 3β-(tris(3-aminopropyl)amine)-N,N-diisopropylchenodeoxy
cholamide,
or a pharmaceutically acceptable salt thereof, and more particularly chosen from compounds **(1), (2), (5), (13), (15), (33)** and **(34).**

10. Compound of formula (I) according to any one of the preceding claims, for its use as a medicament.

11. Pharmaceutical or veterinary composition comprising a compound of formula (I) as defined in any one of Claims 1 to 9 and a pharmaceutically acceptable excipient.

12. Pharmaceutical or veterinary compositions comprising at least one compound of formula (I) as defined in any one of Claims 1 to 9 and at least one antibiotic, other than such a compound of formula (I), more particularly from the family of beta-lactamines, aminosides, macrolides, polypeptides, sulfamides, quinolones, nitro-imidazoles, nitrofuran derivatives, benzyl-pyrimidine nucleus derivatives, tetracyclines or phenicols, such as doxycycline or chloramphenicol, penicillin, ampicillin, amoxicillin, cloxacillin, dicloxacillin, oxacillin, nafcillin, cephalexin, cephapirin, cefazolin, ceftiofur, cefoperazone, cefovecin, cefquinome, thimaphenicol, florfenicol, terramycin, erythromycin, spiramycin, tylosin, josamycin, tilmicosin, tulathromycin, gamithromycin, tildipirosin, clindamycin, lincomycin, pirlimycin, tiamulin, valnemulin, oxolinic acid, flumequine, enrofloxacin, danofloxacin, ibafloxacin, marbofloxacin, difloxacin, obifloxacin, pradofloxacin, rifampicin, rifaximin, sulfamethizole, sulfathiazole, sulfadimidine, sulfamethoxazole, sulfadiazine, sulfadimethoxine, sulfamethoxypyridazine, trimethoprim, baquiloprim, metronidazole, dimetridazole, ronidazole, nitrofurantoin, furazolidone or furaltadone, and even more particularly doxycycline, ampicillin, erythromycin or chloramphenicol.

13. Compound according to any one of Claims 1 to 9, for its use for preventing and/or inhibiting and/or treating bacterial, fungal, viral or parasitic infections in man or animals.

14. Compound according to any one of Claims 1 to 9, for use thereof in a method for potentiating the antibiotic activity of antibiotic compounds which may be chosen from the antibiotic compounds as described in Claim 12.

15. Process for preparing a compound of formula (I) as defined in any one of Claims 1 to 9, comprising a step of reductive amination of the compound of formula (II) 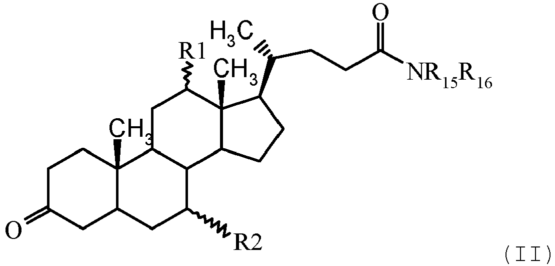
in which R15, R16, R1 and R2 are as defined in one of Claims 1, 2 or 3,
with an amine of formula R'NH2 in which R' is as defined in Claim 1, in the presence of a reducing agent which may be chosen from titanium tetraisopropoxide, zirconium tetraisopropoxide, NaBH₃CN, NaBH₄ or a mixture thereof, preferentially the titanium tetraisopropoxide/NaBH₄ couple, to obtain said compound of formula (I)
